# EUROPEAN PATENT SPECIFICATION

(11) **EP 1 531 832 B1**
(45) Date of publication and mention of the grant of the patent: **15.04.2009**
(21) Application number: 03761605.9
(22) Date of filing: 30.06.2003
(51) Int. Cl.: A61K 31/702, A61P 1/04, A61P 1/12

(54) **THERAPEUTIC COMPOSITIONS FOR USE IN PROPHYLAXIS OR TREATMENT OF DIARRHEAS**
THERAPEUTISCHE ZUSAMMENSETZUNGEN ZUR VERWENDUNG BEI DER PROPHYLAXE ODER BEHANDLUNG VON DURCHFALL
COMPOSITIONS THERAPEUTIQUES SERVANT A LA PROPHYLAXIE OU AU TRAITEMENT DE DIARRHEES

(30) Priority: 28.06.2002 FI 20021275; 14.04.2003 FI 20030564
(43) Date of publication of application: 25.05.2005
(73) Proprietor: Glykos Finland Oy, 00790 Helsinki (FI)
(72) Inventor: ANGSTRÖM, Jonas, S-416 57 Göteborg (SE); TENEBERG, Susann, S-430 63 Hind s (SE); SAARINEN, Juhani, FIN-00370 Helsinki (FI); SATOMAA, Tero, FIN-00700 Helsinki (FI); ROCHE, Niamh, S-146 49 Stockholm a (SE); NATUNEN, Jari, FIN-01520 Vantaa (FI); MILLER-PODRAZA, Halina, S-421 37 Västra Frölunda (SE); KARLSSON, Karl-Anders, S-411 43 Göteborg (SE); ABUL-MILH, Maan, S-424 53 Angered (SE)
(74) Representative: Karvinen, Leena Maria
(86) International application number: PCT/FI2003/000528
(87) International publication number: WO 2004/002495

(56) References cited:
- WO-A1-00/33854
- WO-A1-00/51644
- WO-A1-01/43751
- WO-A1-96/39190
- WO-A1-02/056893
- US-A- 5 217 715

## Description

### FIELD OF THE INVENTION

The present invention relates to the fields of carbohydrate biochemistry and clinical microbiology. The invention provides a therapeutic composition comprising purified fractions of compounds being or containing a pathogen-inhibiting oligosaccharide sequence for use as a medicament. The present invention especially describes an oligosaccharide-containing substance or receptor binding to human diarrhea causing pathogens. The invention is based on wide studies about multiple different pathogenic bacteria having diverse pathologic adhesive mechanisms. The inventors found out multiple common receptors which can be specifically regulated, thus reducing the possibilities for effective therapy by single receptor sequence(s). Special therapeutic compositions comprising at least two different oligosaccharide sequences were found out to be especially useful. The present invention is especially directed to diarrheagenic *Escherichia coli* and/or zoonotic *Helicobacter* species, and use thereof in, e.g., pharmaceutical and nutritional compositions for prophylaxis and treatment of conditions due to the presence of human diarrhea causing pathogens, especially diarrheagenic *Escherichia coli* and/or zoonotic *Helicobacter* species. The invention is also directed to the use of the receptors for diagnostics of human diarrhea causing pathogens, especially diarrheagenic *Escherichia coli* and zoonotic *Helicobacter* species.

### BACKGROUND OF THE INVENTION

Of prime interest with respect to bacterial colonization and infection is the mechanism(s) by which bacteria adheres to the epithelial cell surfaces. The prior art describing bindings of various bacteria does not describe the use of the receptor combinations according to the present invention against infections, especially against intestinal infections. The present invention is also useful for gastrointestinal infections, especially oral infections, and can be used against lung infections.

### Carbohydrate binding of EPEC

CHO-cell mutants have been used to study the effect of glycosylation on EPEC binding. Since a sialic-acid-and-Gal-lacking mutant had lower binding activity than a sialic acid lacking mutant, the authors suggested that the binding sequence could be Galp3GlcNAc or Galβ4GlcNAc and sialic acid. The idea of Galβ3GlcNAc or Galβ4GlcNAc usage was also patented. It was suggested that sialic acid may be necessary for EPEC mediated cell detachment (Vanmale, R.P. et al., 1995). However, the cell surface glycosylation is involving several classes of glycoproteins and glycolipids, and the biosynthetic pathways for glycosylations are so complicated that mutations have multiple biosynthetic effects on glycosylations which are not properly characterized yet. The present invention shows that not all sialic acid oligosaccharide sequences were effectively active, and similarly, the disaccharides alone in all structures are not effectively active. The present invention is directed to the use of more specific effective structures which could not be determined based on the previous data. In another study the same scientist inhibited attachment of an EPEC-strain to Hep-2 cells by N-acetyl lactosamine-BSA and Lex -BSA neoglycoproteins in the concentration range 0.4- 0.8 mg/ml (Vanmaele, R.P. et al., 1995). According to the present invention the disaccharide sequences or Lex are not enough for strong binding to EPEC, but larger or more specific oligosaccharide sequences according to the invention are preferred. The present invention also describes simultaneous use of compositions of two or several oligosaccharide sequences for theraphy of all types of diarrhea causing infections for several pathogens, even when pathogen is undetermined. The present invention is specifically directed to therapeutically useful polyvalent conjugates which are effective in lower concentrations and does not comprise unnatural protein structures, which are potent antigens or allergens.

WO96/39190 indicates binding specificities of heat labile toxin of ETEC E. coli. They list lactose, Galβ3GlcNAc GalNacβ4Gal, and NeuNAcα3Gal linked to solid, inert support. The results with solid support and weak partial receptor epitopes are not relevant to the present invention. The two first disaccharide epitopes were revealed here to be inactive as monovalent inhibitors. The patent application does not describe the longer more effective epitopes nor soluble polyvalent conjugates according to the invention.

A small variety of commercial glycolipids has been used to screen the specifity of an EPEC strain. In decreasing order of activity asialo-GM1, asialo-GM2, globoside and lacto-N-tetraose were observed to bind, while sialylated gangliosides, lactosylceramide, globotriaosylceramide (Galα4Galβ4GlcβCer), and Forssmann glycolipid were negative. Asialo-GM 1 binding was studied with several strains. The binding active epitope was considered to be GalNAcβ4Galor GalNAcβ3Gal with weaker activity. The authors also describe binding to asialo-GM1 neoglycoprotein and GalNAc neoglycoprotein but not inhibition of the binding to the asialo-GM1 by neoglycoproteins at 25 micromolar concentration or undefined oligosaccharides at 1 mM concentration (Jagannatha, H.M. et al., 1991). The authors did not observe the several binding specificities obvious from the present invention, wherein several strains were tested. These specificities include lactosylceramide binding, Galα4Gal-binding (globotriose and Forssman antigen negative) or sialic acid dependent bindings of the bacteria. Their results indicated specifically that the contradictory bindings described were not inhibitable by monovalent or polyvalent oligosaccharide sequences and therefore this study did not show therapeutically useful types of binding as the present invention does. The failure to show all the bindings and inhibition may be related in technical failure in the process.

Several oligosaccharide fractions from human milk were analysed for inhibition of EPEC strains at a concentration 3 mg/ml. Inhibiting activity was observed in pentasaccharide fraction, possible difucosylactose fraction, possible lacto- and neolactotetraose fraction, heptasaccharide fraction and hexasaccharide fraction. The fractions were named after expected major components. Compositions of the fractions were determined by monosaccharide analysis which does not reveal the exact structures of the components. The real compositions of the fractions and the presence of potential minor or other saccharides were not assessed (Cravioto, A, et al 1991). As the active compound or compounds were not characterized, the data would not have lead to the present invention.

Human milk lactoferrin, secretory IgA and free secretory component has been shown to inhibit EPEC-binding to glycoproteins of HELA-cells, with no indications to carbohydrate structures (Nascimento de Araújo, and Giugliano 2001).

Inhibition of the EHEC toxin binding to Galα4Gaβ4Glc and binding data about other toxins of *E.coli* binding to Galβ4GlcNAcβ3Galβ4GlcβCer has not been shown to cure the diseases. There are suggestions with regard to the use of solid phase conjugates containing Galα4Galβ4Glc for inhibition of toxins in therapeutics against diarrhea. The clinical trials using the single epitopes failed. The polyvalent conjugates according to the present invention are specifically directed to soluble polyvalent conjugates for effective inhibitions of pathogens, especially adhesion of diarrhea causing *E*. *coli* bacteria. As a specific embodiment the invention is directed to the prevention of non-toxin secreting *E*. *coli.* The toxin blocking oligosaccharides are not indicated for the types of *E. coli.*

Purified colonialization factors of certain ETEC strains were shown to bind to asialo-GM1 (Galβ3GalNAcβ4Lac-Cer) but not to sialylated control gangliosides (Oroe, et al., 1990). A colonialization factor was shown to bind to several galactoglycoproteins in the rabbit intestine. This binding could be inhibited by asialo-GM1, GM1, GM2, but not so effectively by GM3 and the adhesin bound to GalNAcβ4Gal-neoglycoprotein. Human meconium glycoprotein and its asialo- and afucoform inhibited the binding more weakly and bovine glycophorin most weakly. As the binding of the *Maackia amuriensis* lectin, the meconium glycoprotein binding was also probably polylactosamine dependent. Sialic acid residues were considered not to be important for the bindings (Neeser, J.R. et al, 1989; Wennerås, C. et al. 1995). However, this study shows no useful defined multiepitope solution for treatment of diarrheas or other infections. The polylactosamine specificity was not defined, if present. The present invention shows that not all of polylactosamine type sequences, such as the branched structure, are active. Use of combinations of specificities are not defined.

Human milk gangliosides GM and GM3 and more weakly GD3 were inhibiting the binding of an ETEC and an EPEC strain to human cancer Caco-2 cells, while lactosylceramide, GD3-lactone, and N-acetylneuraminic acid was negative. The present invention shows a lactosylceramide binding and sialic acid dependent bindings. This prior art shows a potential single not well characterized specificity which, if existant, is probably not even among the binding specificities disclosed in the present invention.

EPEC binding to HeLa cells was inhibited by 100 mM N-acetylgalactosamine and a bacterial membrane protein was purified by affinity chromatography using GalNAc (Scatelsky, et al. 1988). However, disclosed weak bindings to a monosaccharide do not allow any conclusions on the biological significance of said binding.

EPECs may bind to Man, alpha-methyl-Man and Man-containing N-glycan sequences. The most active compounds contained Manal-3Man- structure (Neeser et al. 1986). An earlier study characterized Manα1-3Manβ1-4GlcNAc, Manα1-paranitrophenyl and Manα1-3(Manα1-6)Manα1-6(Manα1-3)Manα1-OMe as good binders to type 1 villi of *E. coli* 346 (Firon et al., 1982). However, the publications do not determine the use of the epitope together with other specific binding molecules.

Hemagglutination of erythrocytes by an ETEC strain was inhibited by mucin type II (Sigma), a red cell glycoprotein preparation, gangliosides type II, and sialic acid (1 mg/ml). The hemagglutination could be prevented by protease, sialidase, periodate, urea and guanidium chloride. This study does not describe the nature of the sialic acid potentially involved in the binding under the experimental conditions (Barthus et al, 1985).

CFA/I was purified and shown to be a polymeric protein with Mw about 23,800. The purified protein had hemagglutination activity when aggregated by acid. Only N-acetylneuraminic acid could inhibit the hemagglutination. The effect ofNeuNAc was suggested to be non-specific (Evans et al, 1979). Potentially sialylated, sialidase sensitive, glygoprotein receptors have been reported for ETEC (Pieroni, P., and Worobec, E.A. 1988, Wennerås et al., 1990)
Enteroaggregative *E*. *coli* (EAEC) binding to HeLa-cells have been reported to be inhibitable by human milk protein fractions, which were not characterized (Nascimento de Araújo, and Giugliano 2000). The specificity of the binding towards the carbohydrates involved, if any, has not been described.

Monovalent oligosaccharide Galα4Galβ4Glc has been suggested for inhibition of shiga toxina and shigalike toxin in US20030405503 but the inhibitor seems not be useful in monovalent form. Minor effects were obtained in inhibition of toxin with monovalent inhibitor at 5 % concentration corresponding to about 100 mM oligosaccharide, such high concentration may have dehydrating or other unexpected effects. The present invention is further directed to inhibition of non-toxigenic diarrhea causing *E*. *coli* with monovalent Galα4Gaβ4Glc where effect is seen with useful concentration of 0.3 mM and inhibition at low concentrations of the Globo-receptor oligosaccharides.

Two applications describe the use of Lacto-N-neotetraose for stimulating Bifidobacterium [US5906982] and inhibition of *E*. *coli* and some other bacteria [US6083934]. The inhibition is not against binding of the *E. coli* but occurs during cultivation of the bacteria. The invention does not demonstrate the usefulness of the substance in combination of other saccharides. The applications do not show inhibition of diarrheagenic *E*. *coli* species or types according to the invention. Lacto-N-tetraose has been also claimed for improving the stool quality of infants in an application of Lundblad and Biocarb, but the invention was not directed to treatment of diarrhea, especially the diarrheas caused by *E*. *coli* according to the invention,

### Uropathogenic E. coli

Many studies describing the bingding of uropathogenic *E*. *coli* have been performed. This bacterium binds for example to Galα1-4Gal-sequenccs. PCT/SE81/00065 describes binding activity of E. coli causing urinary tract diseases, not intestinal disease. The data does not show Galα4Gal binding of a diarrheagenic *E.coli*. The uropathogenic bacteria are different from the intestinal diarrhea causing apthogens such as EHEC, ETEC, EPEC, EAEC, or EIEC. Bindings and infection mechanisms of bacteria vary between strains and types of bacteria, and results from one indication cannot be generalized to other indications. The binding specificities of the bacteria infecting different organs are adapted to the tissue specific receptors present on certain tissues. The glycosylations in human and animal is in general tissue- and species-specific. A potential situation where cross-reactivity between species may arise, needs to be addressed by characterizing the exact receptor structures in target tissues and the specificities of the cross-reacting bacteria.

In general the prior art does not describe useful combinations of specified receptor activities for effective treatment of infections, especially intestinal infections. The prior art concentrates on single specificities, which are in general not shown to be present simultaneously on a single strain of bacteria. Due to variations in single bacterial strain the binding specificities may vary between experiments. Further, the prior art does not show useful therapies using monovalent oligosaccharide sequences or polyvalent sequences as described in the present invention

The prior art does not suggest a simultaneous therapeutical use of several inhibitors of carbohydrate mediated pathogen binding. The therapeutically useful combinations of carbohydrate mediated pathogen binding could be considered,
1) if a certain strain of a pathogenic bacterium (or a pathogen cell) has several binding specificities; and
2) if these binding specificities are simultaneously present on the pathogen; and
3) if corresponding receptor oligosaccharide sequences are present on a relevant target tissue; and
4) if relevant receptor oligosaccharide sequences are available for the binding specificities of the pathogen.

When considering usefulness of therapeutic receptor combinations, the effects of possible inhibitor oligosaccharides alone and in combinations must be established. The present invention shows useful substances and compositions for inhibition of pathogens. The prior art is about potential bindings and does not allow any determination of the effective inhibitors of pathogen binding as shown in the present invention.

### Identification of relevant receptor oligosaccharide on human gastrointestinal tract

The present invention discloses the presence of glycoprotein bound oligosaccharide sequences which can serve as primary or first contact receptors on human gastrointestinal epithelium. Several novel receptor sequences are demonstrated. A combination of pathogen binding data with novel information of the most relevant first contact receptors allowed us to determine useful inhibitors for pathogen binding. The analysis of receptors according to the invention revealed that several novel receptor types are present on gastrointestinal epithelia and these are, as first contact receptors, more available for a primary contact with pathogens.

### Zoonotic Helicobacter species

There are more than 20 characterised *Helicobacter* species to date (On, 2001). The species have been isolated from several hosts including primates, pigs, felines, canines, poultry and rodents (On, 2001). In their hosts, *Helicobacter* spp. have been identified from both the gastric and enterohepatic niches of the gastrointestinal tract, where they have been associated with a wide spectrum of clinical outcomes (Fox *et al.,* 2000; Nilsson *et al.,* 2001).

The present invention is also directed to *non-H.pylori Helicobacter* species, especially to enterohepatically infecting ones causing diarrheas and liver diseases. Typically these bacteria, referred as *zHelicobacter* (*zHe*/*icobacteria* in plural), are zoonotically active infecting both human and animals, such as cattle and pets, preferred pet animals are cats and dogs. In a separate embodiment the present invention is directed to gastric infections caused by *zHelicobacteria.*

### Carbohydrates binding to the human gastric pathogen H. pylori

*Helicobacter pylori* is the most widely studied species of the genus and is associated with gastric pathology (Hunt 1996). In particular the bacterium has the noted ability to attach to both Lewis (Le^{b}) (Borén *et al.,* 1993), and Sialyl-dimeric-Le^{x} antigens which may be extremely relevant in the maintenance of a chronic infection (Gerhard *et al.,* 2001; Madhavi *et al.,* 2002). Glycoconjugates, both lipid- and protein-based, have been reported to serve as receptors for the binding of this microorganism as, *e.g*., sialylated glycoconjugates (Evans *et al.,* 1988), sulfatide and GM3 (Saitoh *et al.,* 1991), polyglycosylceramides (Miller-Podraza *et al.,* 1996; 1997a), lactosylceramide (Ångström *et al.,* 1998) and gangliotetraosylceramide (Lingwood *et al.,* 1992; Ångström *et al.,* 1998). Other potential receptors for *Helicobacter pylori* include the polysaccharide heparan sulphate (Ascensio *et al.,* 1993) as well as the phospholipid phosphatidylethanolamine (Lingwood *et al.,* 1992). Binding to lactotetraosylceramide (Teneberg, *et al.,* 2002) and to type 2 lactosamines (PCT/FI02/00043) has been recently described.

US patents of Zopf et al.: 5,883,079 (March 1999), 5,753,630 (May 1998) and 5,514,660 (May, 1996) describe Neu5Acα3Gal- containing compounds as inhibitors of the *H. pylori* adhesion. The sialyl-lactose molecule inhibits *Helicobacter pylori* binding to human gastrointestinal cell lines (Simon *et al.,* 1997) and is also effective in a rhesus monkey animal model of the infection (Mysore *et al.,* 1999). The compound is in clinical trials. US patent Krivan et al. 5,446,681 (November 1995) describes bacterium receptor antibiotic conjugates comprising an asialo ganglioside coupled to a penicillin antibiotic. Especially is claimed the treatment of *Helicobacter pylori* with the amoxicillin-asialo-GM1 conjugate. The oligosaccharide sequences/glycolipids described in the invention do not belong to the ganglioseries of glycolipids. US patents of Krivan et al.: 5,386,027 (January 1995) and 5,217,715 (June 1993) describe the use of oligosaccharide sequences or glycolipids to inhibit several pathogenic bacteria but *Helicobacter* species according to the invention were not shown.

The references above list carbohydrate receptors *of H. pylori,* which is not the target of the present invention. The invention is further directed to the treatment of enteric diseases especially diarrhea, and hepatobiliary diseases including gall bladder stones and liver cancers.

It has been established previously that both *H. pylori* and *H. mustelae* bind gangliotetraosylceramide which was confirmed in this study (Milh et al). The species are not among the *zHelicobacter* species according to invention but were tested as control species.

### SUMMARY OF THE INVENTION

The present invention relates to a therapeutical composition comprising a purified fraction(s) of at least two compounds being or containing a pathogen inhibiting oligosaccharide sequence selected from the pathogen receptors as defined in the formula

[Sacch1]ₘ₁Galβx(Fucα4)ₘ₂Glc[NAc]ₘ₃[β3Gal{β4Glc(NAc)ₙ₁}ₙ₂]ₙ₂[βR₂]ₙ₄ (I)

wherein x is linkage position 3 or 4, Sacch1 is GlcNAcβ3, Galα3, GalNAcβ4, Galα4, or Neu5Xα3/6, wherein X is independently either Ac or Gc;
n1, n2, n3, n4, m1, m2, and m3 are independently integers 0 or 1 with the provisions that m2 may be 1 only when x is 3 and m1 is 0 and m3 is 1;
m3 may be 0 only when Sacch1 is Neu5Xα3, Galα3, GalNAcβ4 or Galα4;
when n4 is 1, then m3 is 0 and n3 is 0, and
when n4 is 0, then m1 is 1 or m2 is 1 or n3 is 1;
R₂ is a ceramide comprising a hydroxyl fatty acid or an analog of a ceramide comprising a hydroxyl fatty acid and
Sacch1 is Galα or GalNAcβ with the provision that when at least two receptors are used these have at least one different variable selected from the group consisting of Sacch1, x, m2, and n4 with the provision that two sialic acid receptors or two neolacto receptors cannot be selected;
and with the provision that when the composition contains only one receptor according to formula (I) then it is together with at least one alpha-hexose receptor as defined in the formula

Hexαp[(Hexαr)]ₙHex (II)

wherein Hex is Gal or Man, n is independently 0 or 1, p and r are linkage position 3 or 6 between Man residues, with the provision that when Hex is Man, then p is 3 and then r is 6, and when p is 6, then r is 3, and when Hex is Gal, then p is 4 and n is 0, with the provision that when Hex is Gal it is not with Galα4Gal-receptor according to the formula I;
for use as a medicament.

The invention especially describes a simultaneous use of at least two carbohydrate receptors of the above groups binding to pathogens, especially diarrhea-causing *Escherichia coli* and/or zoonotic *Helicobacter* species, and analogs or derivatives of the oligosaccharide sequence having binding activity to *Escherichia coli* and/or zoonotic *Helicobacter* species for the treatment and prophylaxis of diarrheas due to the presence of *Escherichia coli* and/or zoonotic *Helicobacter* species.

Among the objects of the invention are the use of the diarrheagenic *Escherichia coli* and/or zoonotic *Helicobacter* species binding oligosaccharide sequences described in the invention as a medicament, and the use of the same for the manufacture of a pharmaceutical composition, particularly for the treatment of any condition due to the presence of *Escherichia coli* and/or zoonotic *Helicobacter* species.

The present invention also relates to the methods of treatment for conditions due to the presence of diarrheagenic *Escherichia coli* and/or zoonotic *Helicobacter* species. The invention is also directed to the use of the receptor(s) described in the invention as an *Escherichia coli* and/or zoonotic *Helicobacter* species -binding or -inhibiting substance for diagnostics of diarrheagenic *Escherichia coli* and/or zoonotic *Helicobacter* species.

Another object of the invention is to provide substances, pharmaceutical compositions and nutritional additives or compositions containing *Escherichia coli* and/or zoonotic *Helicobacter* species -binding oligosaccharide sequence(s).

Other objects of the invention are the use of the above-mentioned *Escherichia coli* and/or zoonotic *Helicobacter* species binding substances for the typing of *Escherichia coli* and/or zoonotic *Helicobacter* species, and the *Escherichia coli* and/or zoonotic *Helicobacter* species binding assays.

The invention is also directed to the use of the oligosaccharide sequences according to the invention in food safety products for inhibition of pathogens, especially diarrhea causing bacteria such as diarrheagenic *E*. *coli* and/or zoonotic *Helicobacter* species. The present invention is also directed to food safety analytics to determine presence of diarrhea causing *E. coli* and/or zoonotic *Helicobacter* species by the use of the receptor carbohydrates according to the invention.

The present invention is also directed to novel oligosaccharide receptors present on glycoproteins of human gastrointestinal tract. The invention is directed to the use of the recepors for analysis of pathogen binding and pathogenic conditions.

### A BRIEF DESCRIPTION OF THE DRAWINGS

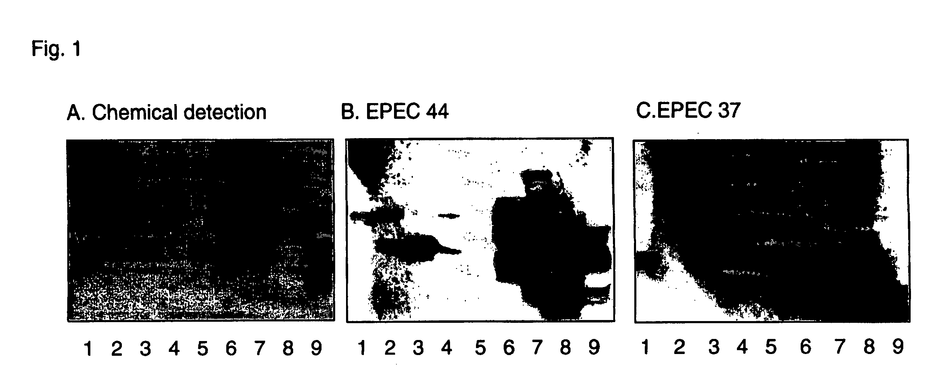

FIG. 1. **Binding of wild type diarrheagenic *Escherichia coli* strains to glycosphingolipid mixtures**. (A) Glycosphingolipids detected with anisaldehyde. (B and C) Autoradiograms obtained after binding of radiolabeled wild type diarrheagenic *E. coli* isolates. The glycosphingolipids were separated on aluminum-backed silica gel plates, using chloroform/methanol/water (60:35:8, by volume) as solvent system, and the binding assay was performed as described in "Experimental procedures". The lanes were: Lane 1, non-acid glycosphingolipids of mouse feces, 40 µg; lane 2, non-acid glycosphingolipids of guinea pig erythrocytes, 40 µg; lane 3, Forssman glycosphingolipid, (GalNAcα3GalNAcβα4Galp4GlcβlCer), 4 µg; lane 4, gangliotriaosylceramide (GalNAcβ4Galβ4Glcβ1Cer), 4 µg; lane 5, gangliotetraosylceramide (Galβ3GalNAcβ4Galβ4GlcβlCer), 4 µg; lane 6, non-acid glycosphingolipids of human meconium, 40 µg; lane 7, non-acid glycosphingolipids of human stomach, 40 µg: lane 8, globoside (GalNAcβGalα4Galβ4GlclCer), 4 µg; lane 9, acid glycosphingolipids of human erythrocytes, 40 µg. Autoradiography was for 12 h.

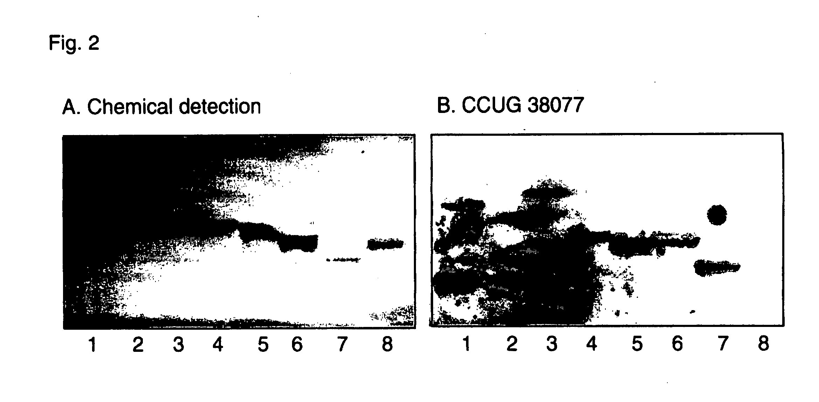

FIG. 2. **Binding of diarrheagenic *Escherichia coli* strain CCUG 38077 to pure glycosphingolipids on thin-layer chromatogram**. (A) Chemical detection by anisaldehyde. (B) Autoradiogram obtained by binding of ³⁵S-labeled *E*. *coli* strain CCUG 38077. The glycosphingolipids were separated on aluminum-backed silica gel plates, using chloroform/methanol/water (60:35:8, by volume) as solvent system, and the binding assay was performed as described under " Experimental procedures". The lanes were: Lane 1, lactotetraosylceramide (Galβ3GlcNAcβ3Galβ4GlcβlCer), 4 µg; lane 2, NeuAc-GM3 (NeuAcα3Galβ4GlcβlCer), 4 µg; lane 3, NeuGc-GM3 (NeuGcα3Galβ4GlcβlCer), 4 µg; lane 4, NeuAcα3-sialylparagloboside (NeuAcα3Galβ4GlcNAcβ3Galβ4GlcβlCer), 4 µg; lane 5, NeuGc-sialylparagloboside (NeuGcα3Galβ4GlcNAcβ3Galβ4Glcβ1Cer), 4 µg; lane 6, sialyl-Le^{a} hexaglycosylceramide (NeuAcα3Galβ3(Fucα4)GlcNAcβ3Galβ4Glcβ1Cer), 4 µg; lane 7, NeuGc-neolactohexaosylceramide (NeuGcα3Galβ4GlcNAcβ3Galβ4GlcNAcβ3Galβ4Glcβ1Cer), 4 µg; lane 8, GD1a ganglioside (NeuAcα3Galβ3GalNAcβ4(NeuAcα3)Galβ4Glcβ1Cer), 4 µg. Autoradiography was for 12 h.

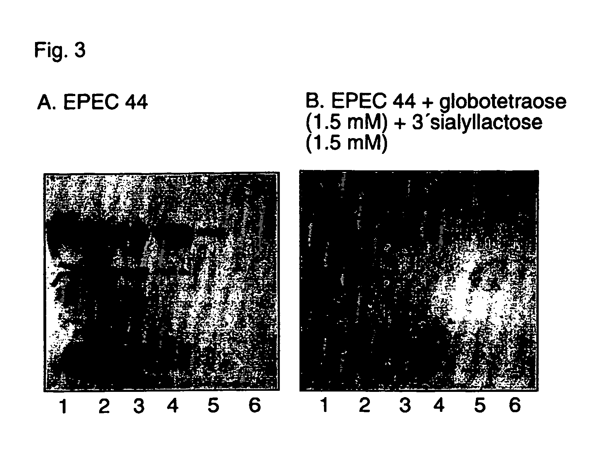

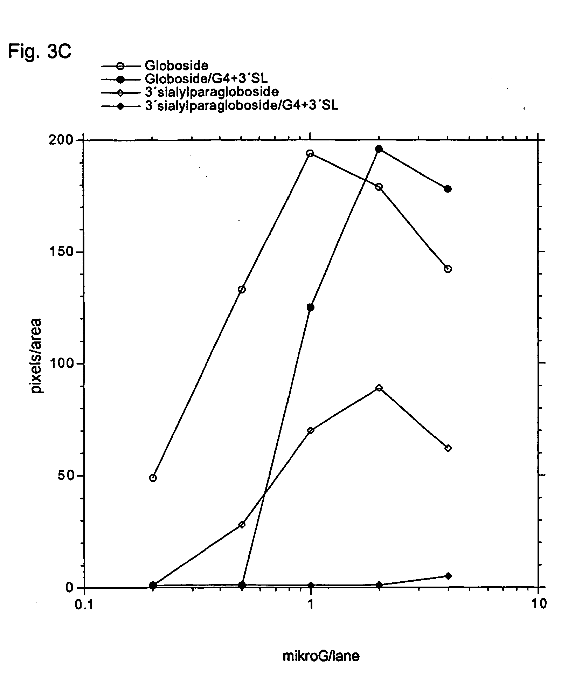

FIG. 3. **Effect of preincubation with oligosaccharides. Radiolabeled** wild type *E. coli* strain 44 was incubated with a mixture of globotetraose (1.5 mM) and 3'sialyllactose (1.5 mM) in PBS for 1 h at room temperature. Thereafter the suspensions were utilized in the chromatogram binding assay. (A) Binding of radiolabeled *E. coli* strain 44. (B) Binding of *E. coli* strain 44 incubated with globotetraose and 3'sialyllactose. Lanes 1-5 were serial dilutions of globoside (GalNAcβGalα4Galβ4Glcpβ1Cer) and 3'sialylparagloboside (NeuAcα3Galβ4GlcNAcβ3Galβ4Glcβ1Cer) Lane 1, 4 µg of each compound, lane 2, 2 µg of each compound, lane 3,1 µg of each compound, lane 4, 0.5 µg of each compound, lane 5, 0.2 µg of each compound, lane 6, B7 type 1 heptaglycosylceramide (Galα3(Fucα2)Galβ3(Fucα4)GlcNAcβ3Galβ4Glcβ1Cer; negative control), 4 µg. The glycosphingolipids were separated on aluminum-backed silica gel plates, using chloroform/methanol/water (60:35:8, by volume) as solvent system, and the binding assay was performed as described under "Experimental procedures". Autoradiography was for 12 h. (C) Binding curves obtained after quantification of binding by densitometry. The autoradiograms in (A) and (B) were analyzed using the NIH Image program.

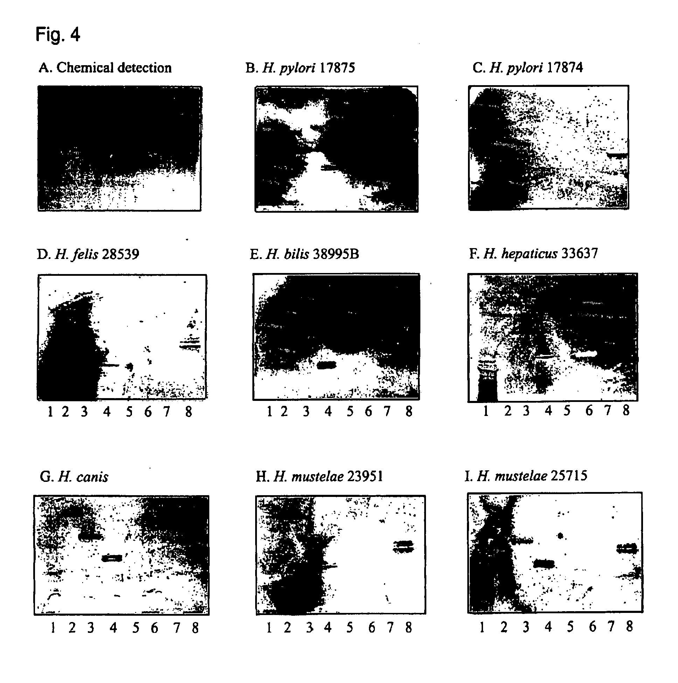

FIG. 4. Glycosphingolipids separated on thin-layer chromatogram after chemical detection (A) and autoradiograms obtained after binding of different *Helicobacter* spp (B-I). Lanes: 1, acid glycosphingolipid fraction of human granulocytes, 40 µg; 2, Galβ4Glcβ1Cer (lactosylceramide) of dog intestine, 2 µg; 3, Galα3Galβ4Glcβ1Cer (isoglobotriaosylceramide) of dog intestine, 2 µg; 4, GalβGalNAcβ4Galβ4Glcβ1Cer (gangliotetraosylceramide) of mouse feces, 2 µg; 5, Galβ3(Fucα4)GlcNAcβ3Galβ4Glcβ1Cer (Leₐ-5 glycosphingolipid) of human meconium, 2 µg; 6, Fucα2Galβ3(Fucα4)GlcNAcβ3Galβ4Glcβ1Cer (Le_{b}-6 glycosphingolipid) of human meconium, 2 µg; 7, GalNAcβ3Galα4Galβ4Glcβ1Cer (globotetraosylceramide) of human erythrocytes, 2 µg; 8, Galβ3GlcNAcβ3Galβ4Glcβ1Cer (lactotetraosylceramide) of human meconium, 2 µg.

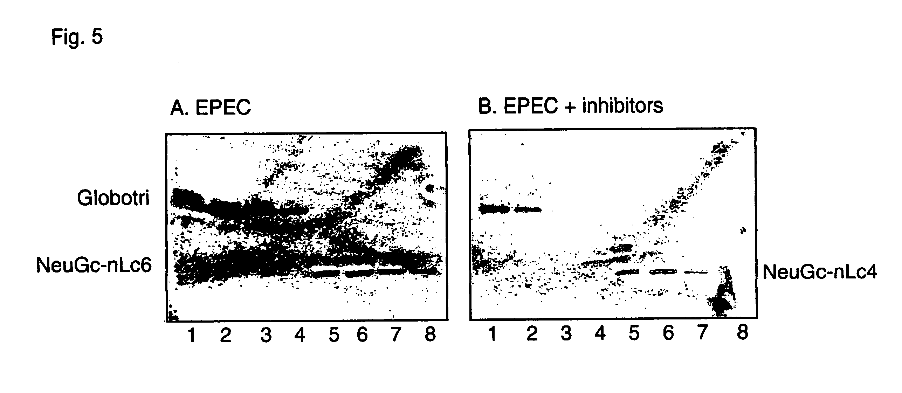

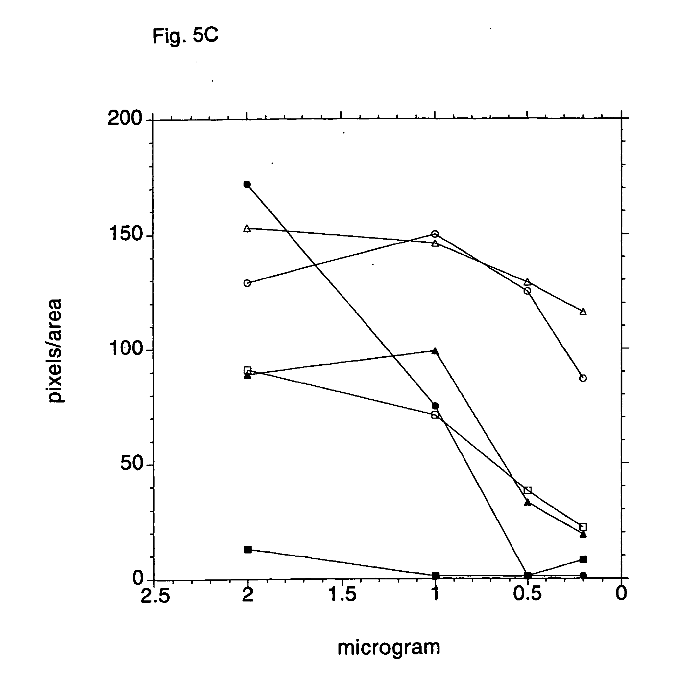

FIG. 5. **Inhibition of binding of enteropathogenic *E. coli* to glycosphingolipids by soluble oligosaccharides**. Radiolabeled wild type *E*. *coli* strain 37 was incubated with a mixture of globotriaose (1.5 mM), neolactotetraose (1.5 mM) and 6'sialyllactose (1.5 mM) in PBS for 1 h at room temperature. Thereafter the suspensions were utilized in the chromatogram binding assay. (A) Binding of radiolabeled *E. coli* strain 37. (B) Binding of *E. coli* strain 37 incubated with a mixture of globotriaose, neolactotetraose and 6'sialyllactose. Lanes 1-4 were serial dilutions of globotriaosylceramide (Galα4Galβ4Glc β1Cer) and NeuGc-neolactohexaosylceramide (NeuGcα3Galβ4GlcNAcβ3Galβ4GlcNacβ3Galβ4Glcβ1Cer).Lane 1, 2 µg of each compound, lane 2,1 µg of each compound, lane 3, 0.5 µg of each compound, lane 4, 0.2 µg of each compound. Lanes 5-8 were serial dilutions of NeuGc-neolactotetraosylceramide (NeuGcα3Galβ4GlcNAcβ3Galβ4Glcβ1Cer).Lane 5, 2 µg of each compound, lane 6, 1 µg of each compound, lane 7, 0.5 µg of each compound, lane 8, 0.2 µg of each compound. The glycosphingolipids were separated on aluminium-packed silica gel plates, using chloroform/methanol/water (60:35:8, by volume) as solvent system, and the binding assay was performed as described under "Experimental procedures". Autoradiography was for 12 h.

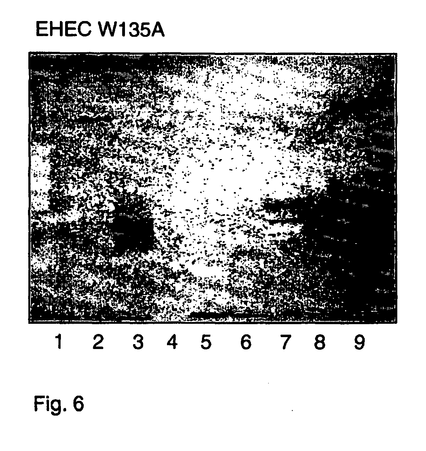

(C) Binding curves obtained after quantification of binding by densitometry. The autoradiograms in (A) and (B) were analyzed using the NIH Image program. Open circles, binding to globotriaosylceramide (Fig. 6A/filled circles, binding to globotriaosylceramide after oligosaccharide incubation (Fig. 6B). Open squares, binding to NeuGc-neolactohexaosylceramide (Fig. 6A)/filled squares, binding to NeuGc-neolactohexaosylceramide after oligosaccharide incubation (Fig. 6B). Open triangles, binding to NeuGc-neolactotetraosylceramide (Fig. 6A)/filled triangles, binding to NeuGc-neolactotetraosylceramide after oligosaccharide incubation (Fig. 6B).

FIG. 6. **Enterohemorrhagic *E*. *coli* does not bind to globoseries glycosphingolipids or sialic acid carrying glycosphingolipids**. Binding of wild type enterohemorrhagic *E. coli* strain W135A to glycosphingolipid mixtures. The glycosphingolipids were separated on aluminium-packed silica gel plates, using chloroform/methanol/water (60:35:8, by volume) as solvent system, and the binding assay was performed as described in "Experimental procedures". The lanes were: Lane 1, non-acid glycosphingolipids of blood group O erythrocytes, 40 µg; lane 2, non-acid glycosphingolipids of bovine intestine, 40 µg; lane 3, acid glycosphingolipids of bovine intestine, 40 µg; lane 4, non-acid glycophingolipids of sheep intestine, 40 µg; lane 5, acid glycosphingolipids of sheep intestine (Folch upper phase), 40 µg; lane 6, acid glycosphingolipids of sheep intestine (Folch lower phase9, 40 µg; lane 7, non-acid glycosphingolipids of cat intestine, 40 µg; lane 8, acid glycosphingolipids of horse intestine 40 µg; lane 9, acid glycosphingolipids of human eosinophil granulocytes, 40 µg. Autoradiography was for 12 h.

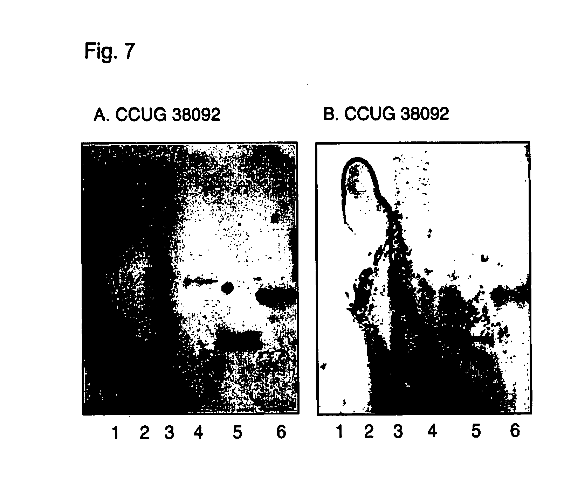

FIG. 7. **Selective loss of binding to isoglobotriaosylceramide upon sub-culture.** Binding of enteroinvasive *E. coli* CCUG 38092 to pure glycosphingolipids on thin-layer chromatogram. (A) Autoradiogram obtained by binding of ³⁵S-labeled *E*. *coli* strain CCUG 38092. (B) Autoradiogram obtained by binding of ³⁵S-labeled *E*. *coli* strain CCUG 38092 after sub-culture. The glycosphingolipids were separated on aluminium-packed silica gel plates, using chloroform/methanol/water (60:35:8, by volume) as solvent system, and the binding assay was performed as described under "Experimental procedures". The lanes were: Lane 1, Galactosylceramide (Galβ1Cer), 4 µg; lane 2, lactosylceramide with non-hydroxy ceramide (Galβ4Glcβ1Cer), 4 µg; lane 3, NeuGc-GM3 (NeuGcα3Galβ4GlcβlCer), 4 µg; lane 4, isoglobotriaosylceramide (Galα3Galβ4Glcβ1Cer), 4 µg; lane 5, gangliotetraosylceramide (Galβ3GalNAcβ4Galβ4Glcβ1Cer), 4 µg; lane 6, globoside (GalNAcβ3Galα4Galβ4Glcβ1Cer), 4 µg. Autoradiography was for 12 h.

### DETAILED DESCRIPTION OF THE INVENTION

Binding of pathogenic viruses and bacteria to human tissues depends on carbohydrate receptors. Several carbohydrates have been in clinical or preclinical trials for the possible effect on the inhibition of infections by pathogenic bacteria or viruses. For example, a sialylated oligosaccharide has been a candidate for the inhibition of human gastric pathogen *H. pylori* and another oligosaccharide has been suggested to be effective against otitis media causing bacteria, but no results have come from the first trial after several years of studying and the other trial has also been announced to have been unsuccessful. There have also been failures with two phase 3 trials concerning oligosaccharide conjugates inhibiting bacterial toxins. Such failures are partially related to a poor understanding on exact pathogenic mechanisms behind the diseases. The present invention includes wide studies on the receptors and molecular mechanisms of pathogenesis which allow treatment and diagnostics of multiple pathogens. Especially, the present invention is directed to the treatment of diseases such as various types of diarrheas caused by binding of *E*. *coli* and/or zoonotic *Helicobacter* species to human intestine.

In a specific embodiment the invention can also be used for treatment of infections of cattle or pet animals. The binding specificities of animal infecting bacteria are different from those of the human pathogens. However, the general mechanisms using several specificities at the same time, and use of polyvalent conjugates, especially soluble polyvalent conjugates according to the invention, are also preferred for use with animals. The binding specificities are also partially cross-reactive and some of the receptor combinations described by the present invention are also useful for animal theraphies, and against some bacterial strains spread from animals such as cows. As the present invention show receptor sequences which are also described from animals living with man and probably play a role in the transfer of the infection, e.g. from cattle to human.

The present invention describes carbohydrate compositions and substances which inhibit pathogens and can be used for theraphy against pathogens. Binding of pathogens such as pathogenic bacteria, toxins, viruses, fungi, or parasites to human or animal tissues depends mainly on receptor carbohydrates. (The term "pathogen cells" means herein pathogens comprising eukaryotic or prokaryotic cells such as pathogenic bacteria, fungi and parasites.) The present invention is specifically directed to the treatment of infection by a pathogen or a pathogen cell having several binding specificities. The present invention describes carbohydrate compositions and substances which inhibit pathogens. The present carbohydrate compositions and substances can be used to inhibit the carbohydrate receptor mediated pathogen binding and prevent or inhibit the interaction. The present invention is specifically directed to the inhibiton of a pathogen cell, which bind to human/animal cell or tissue surfaces using several simultaneous binding specificities.

Often the receptor carbohydrate is located on the surface of the cells of a human or animal that is infected by a pathogen. Alternatively the receptor carbohydrate is located on the surface of the pathogen and recognized by the host animal or human. The receptor carbohydrate may be recognized by carbohydrate binding proteins, such as lectins or carbohydrate binding enzymes such as glycosidases, glycosyltransferases or transglycosylating enzymes or antibodies. Alternatively two oligosaccharide sequences can recognize each other by carbohydrate-carbohydrate interactions.

### General prevention of pathogens by group of defined general receptors and especially using combinations of pathogen inhibiting oligosaccharide sequences

The present invention solves the problems of the inefficacy in therapeutical use of oligosaccharides. The invention demonstrates a simultaneous use of several binding specificities presented by common pathogens. The invention is preferably targeted to use at least two different pathogen inhibiting oligosaccharide sequences, more preferably at least three different pathogen binding oligosaccharide sequences for treatment of conditions due to the presence of a pathogen. In a preferred embodiment four or more different oligosaccharide sequences are used. The present invention is specifically directed to the treatment of infection by a pathogen or a pathogen cell having several binding carbohydrate specificities. The carbohydrate binding specificities according to the present invention can be inhibited by monovalent or polyvalent carbohydrates. Preferentially, the pathogen causing the infection has at least three different inhibitable carbohydrate binding specificities and more preferably at least four inhibitable carbohydrate binding specificities which are inhibited according to the invention. The present invention is especially directed to the treatment of relevant infections when receptor oligosaccharides are present on the target tissue of pathogenesis. The preferred use of two or more oligosaccharide sequences is based on the relevance of the compositions used, feasibility of the compositions for inhibition and special synergistic effects of the compositions against one or several pathogens.

The present invention is especially directed to the treatment of diarrheas caused by *E. coli.* The invention shows useful combinations of receptor-active oligosaccharide sequences for treatment of infections caused by diarrheagenic *E.coli* bacteria, especially *Escherichia coli* -species including EPEC (enteropathogenic *Escherichia coli*)*,* ETEC (enterotoxigenic *Escherichia coli*)*,* EHEC (enterohemorrhagic *Escherichia coli*)*,* EAEC (enteroaggregative *Escherichia coli*) and EIEC (enteroinvasive *Escherichia coli.* The present invention shows a large variety of *E. coli* bacterial strains and demonstrates a group of eight receptor activities which are common to all diarrhea causing *E. coli* bacteria. The prior art is directed to a limited number of receptor sequences and limited number of strains of specific pathogens such as EPEC or ETEC and contains conflicting data about the specificities. The differences between the *E. coli* strains do not allow any generalization concerning the binding specificities of different types or strains of the bacteria. The relevance of the binding specificities to larger groups of strains or the major types of *E*. *coli* can only be assessed by studying numerous strains as shown by the present invention. Precise knowledge of the binding specificities common to the major pathogens and pathogen types causing diarrheas allows rational design of effective theraphies.

The present invention provides a new general treatment for diarrhea. According to the invention, the treated diarrhea is caused by *E. coli,* i.e. the infection is caused by the major diarrheagenic (or diarrhea causing) *Escherichia coli* bacteria, especially the subgroups including EPEC (enteropathogenic *Escherichia coli*), ETEC (enterotoxigenic *Escherichia coli*), EHEC (enterohemorrhagic *Escherichia coli*), EAEC (enteroaggregative *Escherichia coli*) and EIEC (enteroinvasive *Escherichia coli*)*.* The five subgroups cover the majority of all clinically relevant diarrheas caused by diarrheagenic *E.coli.* The prior art does not describe carbohydrate based theraphies for the five major types of the diarrheas caused by *E. coli.* The general treatment for these is especially useful because of the resistance problems developing, when traditional antibiotics are used. The carbohydrate based antiadhesion theraphies are not likely to have the same problems due to limited amounts of possible receptors in gastrointestinal system. The general broad-spectrum diarrhea therapy of the invention is also useful when the pathogen causing patient's diarrhea is not diagnosed.

According to the present invention several receptor oligosaccharide sequences are common to diarrhea causing *E. coli-* bacteria. These receptors are useful for diagnostics of diarrheas or for treatment of diarrheas due to a diarrheagenic *E*. *coli.* The invention describes for the first time general effective theraphies against all major types of diarrhea causing *E. coli* bacteria. The present invention is especially directed to the use of at least two or several of the receptor oligosaccharides sequences to be used against diarrheagenic *E. coli.*
Moreover, the present invention is directed to the use of specific combinations of the receptor active oligosaccharide sequences for diagnosis of diarrheagenic *E. coli* or for prevention or treatment of infections caused by the diarrheagenic *E. coli.*

The present invention is also directed to the treatment of intestinal infections when a patient is infected by a bacterium resistant to traditional antibiotics. The present invention is further directed to the use of the receptor oligosaccharide sequences according to the present invention in connection with traditional antibiotics to improve the therapeutic effects thereof.

This design can be used together with analysis of specific pathogen strains with regard to the eight receptor binding specificities or preferred specific subgroups thereof as described by the present invention.

The present invention is also directed to general theraphies against diarrhea causing types *of E. coli.* Previuos inventions or studies are directed only to single types of diarrhea causing *E. coli* bacteria. When many strains of the different types of pathogens were studied, the eight binding specificities were for the first time shown to be common to all the major types of diarrhea causing *E.coli* such as EPEC (enteropathogenic *Escherichia coli*)*,* ETEC (enterotoxigenic *Escherichia coli*)*,* EHEC (enterohemorrhagic *Escherichia coli*)*,* EAEC (enteroaggregative *Escherichia coli*) and EIEC (enteroinvasive *Escherichia coli*)*.* The present invention is directed to the use of a single component of the eight receptor binding specificities against at least three of the types of the *E. coli* bacteria, more preferentially against at least against four of the *E. coli* types and most preferentially against all five of the *E. coli* types. Similarly, the present invention is directed to the use of combinations of the eight receptor binding specificities against at least three and more preferentially against all the major types of *E. coli* causing diarrheas.

The present invention is also directed to specific combinations of the binding specificities which are especially useful for the prevention of an infection. The combinations are based on
- the knowledge of the properties of oligosaccharide sequences as bacterial inhibitors
- the knowledge of the presence of relevant receptor structures in intestinal epithelium
- the knowledge of the different receptor levels in the infection cascade
- the knowledge of the receptors specifically useful against pathogens to avoid normal flora interactions
- the design of special low cost inhibitors for the binding specificities
- the design of specific receptor combinations for local infections when specific strains have binding activity to a subgroup of the binding specificities

The present invention is also targeted to the therapy of important but less studied *E. coli* types or species causing diarrheas. The invention is specifically directed to the treatment of infections caused by EAEC (enteroaggregative *Escherichia coli*)*.* The invention is also directed to the treatment of diarrheas caused by EIEC (enteroinvasive *Escherichia coli*). These infections cause diarrheas, especially in children in developing countries and novel therapies to treat these are of importance. The need of therapeutic blocking substances for EAEC and EIEC is emphasized because the lack of the knowledge of the specificities of the bacteria.

As a specific embodiment the present invention is directed to the treatment of diarrhea causing not toxin secreting pathogen, preferably non-toxin secreting *E. coli.* The toxin blocker oligosaccharides should not have any effect towards such pathogens. Preferably the non-toxin secreting *E. coli* does not secrete Gala4Gal-based carbohydrate recognizing toxins such as verotoxin. In another embodiment the non-toxin secreting *E. coli* does not express heat labile toxin. The non-toxin secreting *E. coli* is preferably EPEC, EAEC, or EIEC, more preferably EAEC or EIEC.

The present invention surprisingly finds out that Galα4Gal carbohydrates can be used for inhibition of non-toxin secreting *E. coli.* The present invention is directed to use the globo-receptors alone for inhibition of tissue binding of any type of *E. coli* and more preferably for the treatment of non-toxin secreting *E. coli.* In a preferred embodiment the present invention is directed to inhibition of binding of a toxin secreting pathogen preferably pathogenic *E*. *coli* or *zHelicobacter* by an oligosaccharide according to the invention specifically inhibiting the binding of the pathogen in absence of the toxin binding to the oligosaccharide and in another embodiment the invention is direacted to the treatment of the infection and removal of the pathogen in the presence of the toxin binding to the inhibitor oligosaccharide.

The group of eight binding specificities described contain novel receptors for the less studied *E*. *coli* types and species. The present invention is directed to the use of these receptors alone and as a part of compositions against the specific types of *E*. *coli.* In a preferred embodiment at least two or at least three oligosaccharide receptor types are used against the EAEC and/or EIEC. The present invention is also directed to the use of specific combinations of the receptor oligosaccharide species according to the present invention against EAEC and/or EIEC.

The present invention is also targeted to novel therapeutic oligosaccharides and oligosaccharide combinations against ETEC.

The present invention is also targeted to novel therapeutic oligosaccharides and oligosaccharide combinations against EPEC.

The present invention is also targeted to novel therapeutic oligosaccharides and oligosaccharide combinations against EHEC.

Preferred diarrhea-diseases to be treated according to the present invention include for example watery diarrheas, bloody diarrheas and severe diarrheas. The specific indications further include traveller's diarrhea, children's diarrheas especially in developing countries and severe diarrhea related diseases including hemorrhagic diarrhea, haemolytic uremic syndrome (HUS), especially when caused by EHEC. The present invention is also directed to the treatment of persistent diarrheas, especially when caused by EAEC. The persistent diarrheas specifically mean diarrheas lasting 14 days or longer. The present invention is also directed to shigellosis like diarrheas, especially when caused by EIEC. The shigellosis type diarrheas resemble very closely diseases caused by *Shigella* spp. (and pathogens causing them resemble very closely *Shigella* spp.) including watery and bloody diarrheas. It may be difficult or impossible to differentiate shigellosis and EIEC infections. The traveller's diarrhea is a common infection especially for persons travelling in developing countries and it is caused by several types of *E. coli,* especially ETECs.

In developing countries hundreds of millions of children get infected by diarrhea causing *E. coli.* The children diarrheas of developing countries are specifically caused by multiple types of *E. coli* including EPEC (enteropathogenic *Escherichia coli*)*,* ETEC (enterotoxigenic *Escherichia coli*)*,* EHEC (enterohemorrhagic *Escherichia coli),* EAEC (enteroaggregative *Escherichia coli*) and EIEC (enteroinvasive *Escherichia coli*). There is currently no general specific treatment for the diarrheas. Increasing resistance to traditional antibiotics is an increasing problem.The present invention is especially directed for the treatments and analysis of children's diarrheas in developing countries. For treatment of children's diarrheas in developing countries, the therapeutical compositions and substances may be included in hydration solutions (for example comprising salt and sucrose) used for treatments of diarrheas. The therapeutical compositions may also be used together with charcoal tablets or mixed in the charcoal tablets. The compositions and substances according to the invention can be used in combination of traditional theraphies of infections, especially treatments of gastrointestinal infections such as diarrheas caused by *E. coli.*

The present invention further directed to generally milder infection not involving the EHEC bacteria. The generally milder infections are preferably not treated with traditional antibiotics. Milder infections may be produced by ETEC, EAEC, and EIEC strains. Previously suggested but clinically unsuccessful therapy of EHEC-diarrheas by toxin blocking Globotriose-silica was not inhibiting the binding of the bacteria to natural glycolipid receptors. The present invention showed that the Globo-binding is extremely rare or non-existetn among EHECs. This forms an exeption of the general binding specificitie of the diarrhea causing *E*. *coli.*

### Preferred general oligosaccharide receptor groups useful for treatment of infections caused by E. coli and other diarrhea causing bacteria

The present invention discloses common structural motifs of the receptor subtypes of diarrhea causing *E. coli.* One receptor group is based on common structure Galβ3/4Glc(NAc)₀₋₁, this backbone epitope includes lactose (Galβ4Glc) and similar lactose-amines Galβ3GlcNAc (type 1 lactosamine, Lacto receptors), Galβ4GlcNAc (type 2 lactosamine, Neolacto receptors). The backbone epitopes as such are not very effective or are practically inactive as disasaccharides. The present invention disloses the presence of several combinations of activated structures based on the backbone structures. However, the present invention is preferably targeted to the natural types of the activated receptor epitopes present on the human tissues. These structures are especially preferred as therapeutics produced from these are not likely to toxic and natural enzymes exist for production of the receptors.

The lactose epitope and the N-acetylactosamine structures form a common scaffold which is recognized similarily by numerous lectins and enzymes in glycobiology. The disaccharide epitopes share common conformations in interactions with proteins recognizing galactosylated structures. For example human fucosyltransferase III can recognize all three sequences as acceptors. The present invention notices that the disaccharide sequences can be activated by various natural modifications of glycans. The activator parts may be linked to the galactose residue or to the glucopyranose structure which may carry N-acetylmodification. The modifications direct the carbohydrate inhibitors to various receptors on the bacterium. The inventors found out that certain combinations of the active receptor blocking carbohydrates are needed for effective blocking of the pathogens.

The receptor backbone structure is activated by various derivatizations such as elongation from the reducing end by (β3-linkages to galactose or to lactose or to another lactosamine, especially with type 1 and type 2 lactosamine giving naturally occurring structures such as Galβ4GlcNAcβ3Galβ4Glc (Lacto-N-neotetraose), Galβ3GlcNAcp3Galp4Glc (Lacto-N-tetraose), Galβ4GlcNAcβ3Gal, and Galβ3GlcNAcβ3Gal. Furthermore, the receptor backbone structure may be activated by a ceramide structure, comprising a specific hydroxyfatty acid (as described for *H. pylori* in Ångström et al. 1998), this was demostrated by the special Lactosylceramides described here as Lactosylceramide binding.

Furthermore the Lactosamine, especially Galβ4GlcNAc, or lactose structures can be activated by adding specific natural monosaccharides to the non-reducing end. A specially activating nonreducing end terminal structures includes sialic acids preferably NeuNAc or NeuGc linked with α3- or α6-linkage, in a preferred embodiment with α6-linkage. The most preferred structures of the sialic acid binding includes sialyl-lactoses NeuNAcα6Galβ4Glc, NeuNAcα3Galβ4Glc, NeuGcα6Galβ4Glc, NeuGca3Galβ4Glc and corresponding sialylalactosamines NeuNAcα6Galβ4GlcNAc, NeuNAcα3Galβ4GlcNAc, NeuGcα6Galβ34GlcNAc, NeuGcα3Galβ4GlcNAc, and even the elongated LNT and LNnT based forms of these. Even truncated sialic acid epitopes have binding activity towards *E*. *coli.* The N-glycolylneuraminic acid was especially strongly activating structure.

The other activating structures at the non-reducing end side are GalNAcβ4 or Galβ3GalNAcβ4 giving natural type receptor structures described here as specific group Ganglio-receptors. Furthermore the activating structure at the non-reducing end side also includes Galα4 and GalNAcβ3Galα4 giving natural type receptors called Globo-receptors. Ganglio and globoreceptors have activity even as terminal diasaccharides. A preferred form of the Globo-receptor is therefore classified as α-linked Hexose-receptors, including the minimal structure Galα4Gal.

The inventors further notice that the lactosamine receptors, especially Neolacto-receptors may represent terminal GlcNAclβ3-structures.

The present invention is further directed to activation of type 1 lactosamine by Fucα4-structure linked to the GlcNAc forming so called Lewis a structure Galβ3(Fucα4)GlcNAc. The Fucosyl-receptors are especially preferred with reducing activating structures such as lactosylceramide.

The activation by the terminal Galα4-structure was shown to be so effective that the Galα4Gal-structure is active even without the reducing end Glc/GlcNAc. The minimal activating epitope is thus Galα4Gal, more preferably Galα4Galβ. This structure shares the common Gal-structure and is also active as trisaccharide epitopes such as Galα4Galβ4Glc and Galα4Galβ4GlcNAc. The present invention is further directed to partial epitopes Neu5Gcα3Gal, Neu5Acα6Gal, Neu5Gcα6Gal and Neu5Acα3Gal and in a separate embodiment to GalNAcβ4Gal.

Beside the lactosamine receptors the present invention describes specifically Galα4Gal-receptors and Manα3/6Man receptors. The two alpha-linked-hexose receptor types are especially preferred because these are low cost natural receptors.

Combined formulas of the invention for use as a medicament, especially for the treatment of diarrhea, are:
A therapeutical composition containing purified fraction(s) of at least two compounds being or containing a pathogen inhibiting oligosaccharide sequence selected from the pathogen receptors as defined in the formula

   [Sacch1]ₘ₁Galβ3x(Fucα4)ₘ₂Glc[NAc]ₘ₃[β3Gal{β4Glc(NAc)ₙ₁}ₙ₂]ₙ₃[βR₂]ₙ₄ (I)
wherein x is linkage position 3 or 4, Sacch1 is GlcNAcβ3, Galα3, GalNAcβ4, Galα4, or Neu5Xα3/6, wherein X is independently either Ac or Gc;
n1 n2, n3, n4, m1 m2, and m3 are independently integers 0 or 1, with the provisions that m2 may be 1 only when x is 3 and m1 is 0 and m3 is 1, m3 may be 0 only when Sacch1 is Neu5Xα3, Neu5Xα6, Galα3, GalNAcβ4 or Galα4,
when n4 is 1, then m3 is 0 and n3 is 0, and
when n4 is 0, then m1 is 1 or m2 is 1 or n3 is 1;
R₂ is a ceramide comprising a hydroxyl fatty acid or an analog of a ceramide comprising a hydroxyl fatty acid, and
Sacch1 is Galα or GalNAcβ with the provision that when at least two receptors are used these have at least one different variable selected from the group Sacch1 x, m2, n4, with the provisio that not two sialic acid receptors or two neolacto receptors are selected;
with the provision that when only one receptor according to formula (I) is used then it is used together with at least one alpha-hexose receptor as defined in the formula

Hexαp[(Hexαr)]ₙHex (II)

wherein Hex is Gal or Man, n is independently 0 or 1, p and r are linkage position 3 or 6 between the Man residues, with the provision that when Hex is Man, then p is 3 and then r is 6, and when p is 6, then r is 3, and when Hex is Gal p is 4 and n is 0, with the provision that when Hex is Gal it is not used with Galα4Gal-receptor according to the formula I.

In a preferred embodiment the present invention is directed to a therapeutical composition containing purified fraction(s) of at least two compounds being or containing a pathogen inhibiting oligosaccharide sequence selected from the pathogen receptors according to the Formula I, with the provision that when the non-reducing terminal activating sequence is Galα4, GalNAcβ4, Neu5Xα3, or Neu5Xα6 the compositions may comprise shorter oligosaccharide sequences Galα4Gal, GalNAcβ4Gal, Neu5Xα3Gal, or Neu5Xα6Gal, respectively without the reducing end terminal Glc or GlcNAc. More preferably when the terminal activating sequence is Galα4 the composition may comprise the partial epitope Galα4Gal. The composition optionally further contains a Mannose receptor comprising the oligosaccharide sequence Manα3[(Manα6)]ₙMan, wherein n is 0 or 1.

### Lactose based activated structures

In a specific embodiment the present invention is directed to compositions and use of natural type lactose comprising oligosaccharide sequences. This group includes the milk type oligosaccharide sequences such as LNT, LNnT and sialyllactoses and the natural type glycolipid core sequences Galα4Galp4Glc, GalNAcβ4Galβ4Glc and the lactosylceramide receptor with the hydroxyl fatty acid. The lactosamine groups Galβ4GlcNAc and Galp3GlcNAc can be considered as activating groups for the lactose residue of LNT and LNnT and more elongated structures based thereof. The lactose residue was not observed to have adhesion blocking activity against diarrhea causing *E.coli.* The lactose residue can be however effectively activated to preferred high activity substances by adding at the nonreducing side highly activating monosaccharide units GalNAcβ4, Galα4, or Neu5Xα3, Neu5Xα6 or more elongated disaccharide units GalNAcβ3Galα4, Galβ3GalNAcβ4 or Galβ4GlcNAcβ3 or Galβ3GlcNAcβ3 or trisaccharide units Neu5Xα3Galβ4GlcNAcβ3, Neu5Xα6Galβ4GlcNAcβ3, or GlcNAcβ3. In separate embodiments the non-reducing end activating group is Lewis a structure Galβ3(Fucα3)GlcNAcβ3, or Galα4Galβ4GlcNAcβ3 or GalNAcβ4Galβ4GlcNAcβ3. More preferably the non-reducing end activator structure is selected from the frequently occurring sequences Galα4, Neu5Xα3, Neu5Xα6 or in more elongated disaccharide units Galβ4GlcNAcβ3 or Galβ3GlcNAcβ3. A ceramide structure comprising a hydroxyl fatty acid according to the invention can be used as activating structure at the reducing end. In a preferred embodiment these activating structures are combined.

In a preferred embodiment the present invention is directed to therapeutical composition containing purified fraction(s) of at least two compounds being or containing a pathogen inhibiting oligosaccharide sequence selected from the pathogen receptors as defined by the simplified formula

[A1]ₘ₃Galβ4Glc[βA2]ₙ₄ (Ib)

wherein m3 and n4 are independently integers 0 or 1
wherein the natural type non-reducing end activator sequence A1 is selected from the group GalNAcβ4, Galα4, Neu5Xα3, Neu5Xα6, GalNAcβ3Galα4, Galβ3GalNAcβ4 Galβ4GlcNAcβ3, GlcNAcβ3Galβ4GlcNAc, Galβ3GlcNAcβ3, Neu5Xα3Galβ4GlcNAcβ3, Neu5Xα6Galβ4GlcNAcβ3, and Galβ3(Fucα3)GlcNAcβ3, more preferably from the group Galα4, Neu5Xα3, Neu5Xα6, Galβ4GlcNAcβ3 and Galβ3GlcNAcβ3,
and A2 is a ceramide cotaining a hydroxyl fatty acid according to the invention,
with the provision that the oligosaccharide sequences comprise at least A1 or A2 or both.

When only two only A1 containing oligosaccharide sequences are used, both of the A1 sequences are preferably not sialylated.
The composition optionally further contains a Mannose receptor oligosaccharide sequence comprising the oligosaccharide sequence Manα3[(Manα6)]ₙMan, wherein n is 0 or 1.

In a preferred embodiment the present invention is directed to a therapeutical composition containing purified fraction(s) of at least two compounds being or containing a pathogen inhibiting oligosaccharide sequence selected from the pathogen receptors as defined by the formula

[Sacch1]ₘ₁[Galβ3x(Fucα4)ₘ₂GlcNAcβ3]ₘ₃Galβ4Glc[βA2]ₙ₄ (1c)

wherein x is linkage position 3 or 4, Sacch1 is GlcNAcβ3, Galα3, GalNAcβ4, Galα4, or Neu5Xα3/6, wherein X is independently either Ac or Gc;
n4, m1 m2, and m3 are independently integers 0 or 1,
with the provisions that m2 may be 1 only when x is 3,
when Sacch1 is GlcNAcβ3 then m3 is 1 and x is 4, and
m3 may be 0 only when m1 is 1 or when n4 is 1,
when n4 is 0, then m1 is 1 or m3 is 1;
A2 is a ceramide comprising a hydroxyl fatty acid or an analog of a ceramide comprising a hydroxyl fatty acid, and
with the provision that at least two receptors are selected so that these have at least one different variable selected from the group Sacch1, x, m2, n4, preferably with the provisio that not two sialic acid receptors are selected.
The composition optionally further contains a Mannose receptor comprising the oligosaccharide sequence Manα3[(Manα6)]ₙMan, wherein n is 0 or 1.

The present invention is in a preferred embodiment directed to a composition comprising at least two compounds described above by Formula 1c when m2 is 0. The present invention is in a preferred embodiment directed to a composition comprising at least two compounds described above by Formula 1c when n4 is 0.

In a preferred embodiment the present invention is directed to therapeutical composition containing purified fraction(s) of at least two compounds being or containing a pathogen inhibiting oligosaccharide sequence selected from the pathogen receptors as defined by the formula

[Sacch1]ₘ₁[GaβxGlcNAcβ3]ₘ₃Galβ4Glc (Id)

wherein x is linkage position 3 or 4, Sacch1 is Galα4, Neu5Xα3 or Neu5Xα6, wherein X is independently either Ac or Gc;
m1, and m3 are independently integers 0 or 1,
with the provision that either m1 is 1 or m3 is 1,
with the provision that at least two receptors are selected so that these have at least one different variable Sacch1 or x, preferably with the provisio that not two sialic acid receptors are selected. In a preferred embodiment Neu5X is Neu5Ac.

The more preferred lactose based oligosaccharide sequences in compositions and for uses according to the invention include preferred substances according to the formula 1c: Galα4Galβ4Glc, NeuNAcα3Galβ4Glc, NeuNAcα6Galβ4Glc, Galβ4GlcNAcβ3Galβ4Glc and Galβ3GlcNAcβ3Galβ4Glc. In a preferred embodiment the preferred sialylated structures according to formula 1 c include sialyllactosamines NeuNAcα3Galβ4GlcNAc, NeuNAcα6Galβ4GlcNAc. The seven highly preferred oligosaccharide sequences according to the present invention thus include Galα4Galβ4Glc, NeuNAcα3Galβ4Glc, NeuNAcα6Galβ4Glc, NeuNAcα3Galβ4GlcNAc, NeuNAcα6Galβ4GlcNAc, Galβ4GlcNAcβ3Galβ4Glc and Galβ3GlcNAcβ3Galβ4Glc.

In a preferred embodiment the preferred sialylated structures include one or all of the common sialyl-oligosaccharides of bovine milk NeuNAcα3Galβ4Glc, NeuNAcα6Galβ4Glc and NeuNAcα6Galβ4GlcNAc. The bovine milk oligosaccharides may be provided as a fraction of bovine milk as described by Nakamura et al., 2003. In a separate embodiment N-glycolylneuraminic acid containing oligosaccharide sequences are preferred. The preferred N-glycolyl oligosaccharide sequences include NeuNGcα3Galβ4Glc, NeuNGcα6Galβ4Glc, NeuNGcα3Galβ4GlcNAc and NeuNGcα6Galβ4GlcNAc.

In a preferred embodiment lactose based oligosaccharide sequences in compositions and for uses according to the invention include neutral oligosaccharide sequences Galα4Galβ4Glc, Galβ4GlcNAcβ3Galβ4Glc (LNnT) and Galβ3GlcNAcβ3Galβ4Glc (LNT).

In a preferred embodiment at least one preferred sialylated oligosaccharide, preferably a bovine milk fraction comprising sialylated oligosaccharides is used together with at least one preferred neutral oligosaccharide.

In a preferred embodiment the composition is not human milk nor a fraction of human milk oligosaccharides. Due to shortage of material and risks of infection human milk is not a preferred source of oligosaccharides. In a preferred embodiment the composition of two oligosaccharide sequences is not a human milk oligosaccharide fraction potentially comprising a mixture of LNT and LNnT. Preferred binary combinations of most preferred neutral oligosaccharides include compositions comprising LNnT and Galα4Galβ4Glc, and compositions comprising LNT and Galα4Galβ4Glc. In another preferred embodiment compositions comprising LNnT are preferred over compositions comprising LNT, when a regional infecting strain of bacterium is using LNnT specificity. LNnT is also preferred to be used in monovalent inhibitor compositions, especially when low concentrations of the inhibitors are used.

### Use of the preferred structure as single substances

The present invention is specifically directed to use a single substance according to Formula 1d optionally with other structures according to the invention for inhibition of diarrhea causing *E*. *coli,* preferably human diarrheagenic *E*. *coli.* More preferably the invention is directed to inhibition of any non-toxin secreting diarrheagenic *E*. *coli* type according to the invention. In a preferred embodiment monovalent oligosaccharides are used, more preferably monovalent oligosaccharides are used under 2 mM final concentration, more preferably under 1 mM concentration. In a specific embodiment globotriose oligosaccharide is used as concentration under 0.3 mM or under 0.1 mM concentration but preferably above 0.01 mM concentration.
In another embodiment the said oligosaccharides are used as soluble polyvalent conjugates containing a single oligosaccharide and optionally other carbohydrates according to the invention. More preferably the oligosaccharide is used as polyvalent conjugate to a soluble oligosaccharide or polysaccharide according to the invention. The present invention is further directed to use of the single oligosaccharide epitopes according to the invention for treatment of diarrheas caused by any single type of *E. coli* according to the invention, preferably EAEC and EIEC types of *E*. *coli.*

The preferred structures are multiply preferred according to the present invention for example as frequent binding epitopes according to the invention. The availability of the saccharides for effective commercial production and natural presence and acceptability as natural type sequences makes the saccharide further preferable. The most preferred oligosaccharide mixtures according to the invention are further preferred as monovalent inhibitors of bacterial adhesion. The results showed that the Globo-receptors, Lacto-receptors, sialic acid receptors and Neolacto-receptors are inhibitable at low concentrations specifically by corresponding monovalent oligosaccharides. The use of the free monovalent specific oligosaccharides as inhibitors of adhesion of diarrhea causing *E*. *coli* has not previously been demonstrated. Furthermore active combinations of the free preferred oligosaccharides were shown, for example the mixtures of globo-oligosaccharides, LNnT, and sialyl-oligosaccharides were shown to be active low concentration inhibitors in simultaneously on diarrhea causing *E*. *coli.* The present invention also shows first time the simultaneous presence of multiple binding activities on a specific diarrheagenic *E.coli*. Furthermore these form a structurally defined group of activated lactose structures effective against different receptor structures of human diarrheagenic bacteria.
The present invention is further directed to the use of receptor types from different of contacts in infection as described by the invention. The first contact receptors are present on the level of of glycoproteins and possibly largest glycolipids, while the second contact receptors are present closer to the membrane on glycolipids as described by the invention.

A therapeutical composition according to the claim 1 comprising a purified fraction(s) of at least two compounds being or containing a pathogen inhibiting oligosaccharide sequence according to the formula I are selected from to the groups a) and b):
a) At least one first contact receptor of lacto, neolacto, fucose or sialic acid receptor types
   as defined in the formula

   [Sacch1]ₘ₁Galβx(Fucα4)ₘ₂Glc[NAc]ₘ₃[β3Gal{β4Glc(NAc)ₙ₁}ₙ₂]ₙ₃ (III)

   x is linkage position 3 or 4,
   Sacch1 is either Neu5Xα3/6, wherein independently X is either Ac or Gc meaning that the sialic acic is either Neu5Ac or Neu5Gc, or GlcNAcβ3
   wherein n1, n2, n3, m1, m2, and m3 are independently integers 0 or 1;
   with the provisions that m2 may be 1 only when x is 3,
   that m3 may be 0 only when Sacch1 Neu5Xα3/6.
   and
b) At least one second contact receptor of
   Lactosylceramide, ganglio-, or Galα4Gal-type receptors as defined in the formula

   [Sacch4]ₘ₁Galβ4Glc(NAc)ₙ₁[βR₂]ₙ₃ (IV)
wherein n1 and n3 are independently integers 0 or 1, with the proviso that when n3 is 1, then n1 is 0;
m1 is either 1 or 0, with the provisio that when n3 is 0 then m1 is 1
R₂ is a ceramide comprising a hydroxyl fatty acid or an analog of a ceramide comprising a hydroxyl fatty acid, and
Sacch is Galα or GalNAcβ.

And optionally at least one alpha-hexose receptor as defined in the formula

Hexαp[(Hexαr)]ₙHex (II)

wherein
Hex is Gal or Man,
n is independently 0 or 1, p and r are linkage position 3 or 6 between the Man residues, with the proviso that when Hex is Man then p is 3 and then r is 6, and
when p is 6 then r is 3 and when Hex is Gal p is 4 and n is 0, with the provisio that when Hex is Gal it is not used with Galα4Gal-receptor according to the formula IV;
for use as a medicament.

More preferred first contact recptors includes Sialyl-receptors, Lacto-receptors and Neolacto-receptors. Among the second contact receptors the Galα4Gal-receptors are especially preferred. In a specific embodiment the Mannose receptor is included in the group of first contact receptors as the high-Mannose structures are presented by glycoproteins.

### The frequent binding specificities among gastric pathogens, especially diarrhea causing E. coli.

The present invention is preferably directed to the use of most frequently occurring binding specificities of diarrhea causing *E*. *coli.* The most frequent binding specifities include Globo-receptors, Sialyl-receptors, Lacto-receptor, and Neolacto-receptors. The frequent structures includes oligosaccharide sequences according to the formula:

[Sacch1]ₚGalβyGlc(NAc)ᵣβ3 {Galβ4[Glc(NAc)ᵤ]ᵥ}ₛ (V)

wherein p, r, s, u and v are each independently 0 or 1, and y is either linkage position 3 or 4, x is either linkage position 3 or 4,
wherein Sacch1 is NeuNXα3 or NeuNXα6 or Galα4 or GlcNAcβ3 with the proviso that
r may be 0 only when s is 0 and Sacch is NeuNXα3 or NeuNXα6 or Galα4.

Alternatively the Globo-receptors may be other terminal Galα4Gal-sequences.

Of the frequent binding specifities the Globo-receptors represent especially strong binding and stabile binding. The Sialyl-receptors, Lacto-receptors, and Neolacto-receptors are also preferred because of the stabile and strong bindings indicating increased importance during infections. The inventors were also able to show that the binding specificities toward the corresponding glycolipid sequences are inhibitable even by low concentrations of monovalent oligosaccharides. The preferred frequent binding specificities include activated strongly binding forms of the sequences, more preferably human and animal milk oligosaccharides lacto-N-tetraose (LNT), lacto-N-neotetraose (LNnT), sialyl-lactoses NeuNAcα3Lac, NeuNAcα6Lac, sialyl-lactosamines NeuNAcα3LacNAc, NeuNAcα6LacNAc and the elongated forms NeuNAcαGalβ4GlcNAcβ3Galβ4Glc, NeuNAcα6Galβ4GlcNAcβ3Galβ4Glc. Additionally human natural type oligosaccharide sequences Galα4Galβ4Glc and carboxylic acid reduced pectin type sequences Gal[α4Gal]ₙ are preferred.

The present invention is preferably directed to the use of at least two oligosaccharide sequences form different of the frequent binding specificities are used for inhibition of diarrheagenic pathogen, preferably *E*. *coli,* more preferably the non-toxin secreting diarrhegenic *E*. *coli* or less severe diarrhea causing *E*. *coli.* In a preferred embodiment at least three oligosaccharide receptor types are used, and most preferably all four frequent oligosaccharide receptor types are used.

In another preferred embodiment at least one of the receptor types used is a globoreceptor. In another embodiment at least one of the receptor types used is a sialic acid receptor. Preferably sialic acid receptor and Globo-receptor are used together, more preferably with a Lacto-receptor or Neolactoreceptor.

In another preferred embodiment a Lacto-receptor is used together with a Neolacto-receptors and additionally with a Globo-receptor or a sialic acid receptor.

In another preferred embodiment a sialic acid receptor is used together with a Neolacto-receptor or a Lacto-receptor and optionally with a Globo-receptor. In a preferred embodiment the milk type oligosaccharide receptors Lacto-, Neolacto and sialic acid receptor are used together.

The present invention is further directed to the use of any of the preferred combinations of frequent binding epitopes with at least one of the other binding specificities including the Lactosylceramide receptors, Mannose-receptors, Fucosyl-receptors and Ganglio-receptors. In a preferred embodiment any of the preferred combinations of frequent receptors and the Lactosylceramide-receptors are used together.
In a preferred embodiment any of the preferred combinations of frequent receptors and the Mannose-receptors are used together.
In a preferred embodiment any of the preferred combinations of frequent receptors and the Ganglio-receptors are used together.

The data of the invention was mainly obtained by using numerous different strains of diarrhea causing *E. coli.* The generality of the intestinal receptors was further studied with several types of zoonotic Helicobacter species. The inventors were able to find at least five overlapping binding specificities with the *E*. *coli* specificities. These include Lactosylceramide-receptors, Lacto-receptors, Neolacto-receptors, Ganglio-receptors and Sialic acid receptors. These receptors are likely to be common with human and many pet and cattle animals.

### General receptors with activity against potentially zoonotic pathogens

The group of receptors is preferred as "general receptors" in diarrheas with zoonotic potential. The most stable expression of the general receptors was shown by the neutral galactose based receptors. Among this family lactose/lactosamine receptors form a special structurally similar class of receptors.

A preferred embodiment of the invention is a therapeutical composition wherein at least one of said compounds comprises a pathogen inhibiting oligosaccharide sequence selected from a further group of pathogen receptors:

i) [Galβy]ₚ[Hex(NAc)ᵣαz/βz]ₛGalβx[Glc(NAc)ᵤ]ᵥ (VI)

wherein p, r, s, u and v are each independently 0 or 1, and y is either linkage position 3 or 4, x is either linkage position 3 or 4, and z is either linkage position 3 or 4, and Hex is either Gal or Glc,
so that
when v is 1 and u is 0 then x is 4,
when v is 0 then s is 1 and preferably also p is 1,
when s is 0 the also p is 0 and v is 1,
when p is 1, and y=3, Hex is Galβ or Glcβ and r=1, or p is 1 and y=4 and Hex is Glcβ and
r=1 so that the terminal Gal is β3- or β4- linked to GlcNAcβ3 or the terminal Gal is β3-linked to GalNAcβ),
when p is 0 and z is 4, then Hex is Galβ and r is 1 so that the terminal monosaccharide structure is GalNAcβ4, or p =0 and z=3 so that the terminal is HexNAc/Hexo/β3),
when there is nonreducing terminal Galβ3/4, this can be further substituted by SAα3/6,
wherein SA is a sialic acid, preferably NeuNAc, N-acetylneuraminic acid, or NeuNGc, N-glycolylneuraminic acid,
preferably together with pharmaceutically acceptable carriers and adjuvants.

### Preferred neutral galactose based general receptors according to the invention

According to the present invention the Galactoseβ3/4 -based general receptors include structures according to the formula:

[Galβy]ₚ[Hex(NAc)ᵣαz/βz]ₛGalβx[Glc(NAc)ᵤ]ᵥ (VII)

wherein p, r, s, u and v are each independently 0 or 1, and y is either linkage position 3 or 4, x is either linkage position 3 or 4, and z is either linkage position 3 or 4, and Hex is either Gal, or Glc ,
so that
when v is 1 and u is 0 then x is 4,
when v is 0 then s is 1 and preferably also p is 1
when s is 0 the also p is 0 and v is 1
when p is 1, and y=3, Hex is Galβ or Glcβ and r=1, or p is 1 and y=4 and Hex is Glcβ and r=1 so that the terminal Gal is β3- or β4- linked to GlcNAcβ or the terminal Gal is β3-linked to GalNAcβ),
when p is 0 and z is 4, then Hex is Galβ and r is 1 so that the terminal monosaccharide structure is GalNAcβ4, or p =0 and z=3 so that the terminal is HexNAc/Hexα/β3).

### Major general, receptor types according to the invention

The formula above is further divided to major structure groups including
1. Lactose/lactosamine type carbohydrate receptor
   This group further includes Lactose- receptors, and lactosamine receptors including Lacto-receptors, and Neolacto receptors
2. Ganglio-receptors
3. Sialic acid receptor

### Preferred lactose/lactosamine type general receptors

[Galpy]ₚ[Hex(NAc)ᵣa3]ₛGalβx[Glc(NAc)ᵤ]ᵥ (VIII)

wherein p, r, s, u and v are each independently 0 or 1, and y is either linkage position 3 or 4, x is either linkage position 3 or 4, and a is either alpha or beta, and Hex is either Gal or Glc.
so that
when p is 1, Hex is Glcβ and r=1, and a is β, (the terminal Gal is β3- or β4- linked to GlcNAcβ3)
when p is 0, then preferably
Hex is Gal, r is 0 and a is alpha (terminal structure is Galα3) or
Hex is Glc, r is 1 and a is beta (terminal structure is GlcNAcβ3)

In a preferred embodiment the lactose/lactosamine type general receptors are according to the formula:

[Galβy]ₚ[GlcNAcβ3]ₛGalβx[Glc(NAc)ᵤ]ᵥ (IX)

wherein p, r, s, u and v are each independently 0 or 1, and y is either linkage position 3 or 4, x is either linkage position 3 or 4, so that
at least p is 1 or v is 1,
when p is 1, s is 1
When u is 0 and s is 0 and p is 0, x is 4 and the reducing end Glc is linked to ceramide comprising a hydroxylfatty acid.

Most preferred lactose/lactosamine general receptors include
the human milk tetrasaccharides Galβ4GlcNAcβ3Galβ4Glc and Galβ3GlcNAcβ3Galβ4Glc and lactosylceramides.
The preferred lactosamine structures also include oligosaccharide sequences and oligosaccharides from the group Galβ4GlcNAc, Galβ3GlcNAc, Galβ4Glc, Galβ4GlcNAcβ3Gal, Galβ3GlcNAcβ3Gal, And GlcNAcβ3Galβ4Glc, GlcNAcβ3Galβ4GlcNAc, Galβ4GlcNAcβ3Galβ4GlcNAc, and Galβ3GlcNAcβ3Galβ4GlcNAc.

For effective treatment of emerging and present diarrheagenic pathogens the all eight receptor specificities are useful. When the pathogen is not *E. coli* the general presence of mannose binding is demonstrated in the prior art works with Salmonella. The present invention allows to predict that the Galα4Gal-oligosaccharide sequence including binding specificities and even the Fucosyl-receptor type binding specificities will be found in human intestinal pathogen causing diarrheas.

When considering together the preferred receptor groups, the Lacto-receptors and Neolacto-receptors belong to first contact receptors, frequent receptos and general receptors. This makes the use and combined use of the Lacto- and Neolacto-receptors especially in activated forms such as LNT and LNnT especially preferred according to the invention.

The inventors characterized eight different binding specificities to a large number of diarrhea causing *E. coli* bacteria and several corresponding receptors in human intestinal tissues. The oligosaccharide sequences include one or several of the receptor oligosaccharide sequences selected from the following groups:

### Eight separate receptor oligosaccharide sequences of intestinal pathogens,:

a) Lactosylceramide receptors: for example binding to lactosylceramide and isoglobotriaosylceramide when the ceramides comprise hydroxylfatty acids.
b) Ganglio-receptors: for example binding to gangliotriaosylceramide and gangliotetraosylceramide.
c) Gala4Ga1-receptors: for example binding to galabiaosylceramide, globotriaosylceramide, globotetraosylceramide and the Forssman glycosphingolipid.
d) Lacto-receptors: for example binding to lactotetraosylceramide.
e) Neolacto-receptors: for example binding to neolactotetraosylceramide, neolactohexaosylceramide, NeuGcα3-neolactohexaosylceramide and oligosaccharide sequences comprising GlcNAcβ3Gal, especially GlcNAcβ3Gal/β4GlcNAc.
f) Fucosyl-receptors: for example binding to the Le^{a}-5 glycosphingolipid.
h) Sialic acid-receptors: for example binding to various oligosaccharide sequences with different sialic acid, especially N-acetylneuraminic acid NeuAcα- and/or N-glycolylneuraminic acid, NeuGcα.
g) Mannose receptors: represented by the Manα3(Manα6)Man-neoglycolipid.

### Preferred oligosaccharide sequences among the receptor groups

The present invention is preferentially directed to the use of a free oligosaccharide or derivatives thereof which are not glycolipids except for the hydroxylfatty acid comprising lactosylceramide glycolipids as described below. In general the glycolipids may diffuse to tissues and actually increase pathogen binding and the formulations to prevent this are considered difficult to produce. The hydroxyl group in the ceramides of the lactosylceramide glycolipids according to the present invention allows stronger contact between the glycolipids which would more effectively keep these together for example in membrane-like formulations and avoid diffusion to intestinal epithelium. The preferred polyvalent conjugates described by the invention are not neoglycoproteins such as albumin conjugates which are potent immunogens and can be used in causing immune responses. The polyvalent conjugates according to the present invention are preferably non-immunogenic and preferably do not contain immunogenic protein or peptide parts.

### Lactosylceramide receptors

The lactosylceramide receptors of the diarrhea causing *E*. *coli* depend on the presence of hydroxyl fatty acid on the ceramide. The present invention is directed to the use of lactosylceramides comprising hydroxy fatty acids against *E*. *coli* infections. The lactosylceramide receptors according to the present invention means a lactose residue comprising molecule in which lactosyl residue is linked to a ceramide structure comprising a natural type of hydroxylfatty acid or alternatively lactosylceramide receptor means mimetic structure of lactosylceramide in which the lactosyl residue is linked to a hydroxyl group comprising a ceramide-mimicking structure. The hydroxyl group of the hydroxyl fatty acid or ceramide mimicking structure preferentially forms a hydrogen bond with Glc-residues linked to ceramide or ceramide-mimicking structure. The lactosylceramide or mimetic structure can be substituted at position 3 or 4 of the Gal residue by natural type oligosaccharide sequences. The lactosylceramide receptor glycolipids also includes lacto-and neolactoseries glycolipids comprising a hydroxyl fatty acid. In other embodiments the present invention is also directed to the use of globo- and ganglioseries glycolipids comprising a lactosyl residue and a hydroxylfatty acid. The present invention is also directed to the use of analogs of lacto- or neolactoseries oligosaccharide sequences linked to the hydroxyl group comprising ceramide-mimicking structure. The present invention is also directed to the use of analogs of globo- or ganglioseries oligosaccharide sequences linked to the hydroxyl group comprising ceramide-mimicking structure. In a preferred embodiment the invention is directed to the use of non-sialylated forms of lactosylceramide receptors according to the present invention. The preferred embodiments include molecules according to the following Formula

R₁xGalβ4GlcβR₂ (X)

wherein x is linkage position 3 or 4,
R₂ is ceramide comprising a hydroxyl fatty acid or an analog of a ceramide comprising a hydroxyl fatty acid and
R₁ is Galα, Galβ, GalNAcβ, GlcNAcβ or longer oligosaccharide comprising one of these residues at the reducing end or Neu5Xα with the proviso that when R₂ is GlcNAcβ or Neu5Xα then x is 3 and Neu5X is sialic acid preferably Neu5Ac or Neu5Gc.

The present invention is directed to substances and compositions comprising polyvalent conjugates of lactosylceramide receptor and especially polyvalent conjugates of a mimetic structure of lactosylceramide according to the present invention. Especially polyvalent conjugates of mimetic structures of lactosylceramide are preferred when the lactosylceramide or mimetic structure of lactosylceramide is linked to a polysaccharide, optionally through a spacer group. In a specific embodiment the use of polyvalent conjugates are preferred over the use of lactosylceramide glycolipids. Use of glycolipids is more difficult as there is need to prevent the diffusion of the receptors to tissues. The prevention can be, however, achieved for example by incorporating the glycolipids in medical carbon matrix or in a stabile membrane or micellar structures.

It is realized that two or even three or more receptor binding specificities according to the invention can be presented by a single lactosylceramide receptor.

The present invention is also directed to the use of lactosylceramide comprising hydroxylfatty acids and analogs and derivatives thereof for therapy of gastrointestinal diseases, especially diarrheas and more specifically diarrheas caused by *E. coli* bacteria. In preferred embodiments the infection is caused by ETEC, EPEC, EHEC, EIEC, or EAEC, more preferentially by EHEC, EIEC or EAEC. In a preferred embodiment the present invention is directed to the use of a milk fraction comprising lactosylceramide comprising a hydroxylfatty acid. The milk is preferentially from a dairy animal such as a cow or any other dairy animal or milk producing animal which produces hydroxyl fatty acid-containing lactosylceramide. The prior art discussed above has been directed to the use of some milk glycolipids but the prior art does not realize the usefulness of the hydroxylfatty acid-containing glycolipids against diarrhea-causing *E. coli* bacteria. The lactosylceramide receptors according to the present invention are especially useful for functional food or feeds as nutritional additives.

### Ganglio-receptors

Preferred ganglioseries receptor comprises oligosaccharide sequences according to the Formula

[Galp3]ₙ₁GalNAc[β4Gal{β4Glc}ₙ₂]ₙ₃ (XI)

wherein n1 n2 and n3 are independently integers 0 or 1, preferably with the proviso that at least n1 or n3 is 1 and with the proviso that no sialic acids are linked to the oligosaccharide sequence.

The preferred oligosaccharide sequences are Galβ3GalNAcβ4Galβ4Glc, Galβ3GalNAcβ4Gal, Galβ3GalNAc, GalNAcβ4Gal and GalNAcβ4Galβ4Glc. Even GM1 oligosaccharide sequence can be used according to the present invention in novel combination therapies but it is less preferred due to complexity of the structure.
The screening of wide variety of ganglioseries and comparison of the structures in examples of the present invention allows the determination of Galβ3GalNAc as a novel preferred novel receptor oligosaccharide sequences of the ganglioseries receptor oligosaccharide sequences. The data indicates that even terminal Galβ3GalNAc in GM1-sequence can bind to diarrhea causing *E*. *coli.* The binding to the terminal disaccharide has previously not been demonstrated and the tetrasaccharide epitopes may be used in formulations which allows more effective presentation of the terminal disaccharide. According to one embodiment of the invention, the Galβ3GalNAc is preferably not β4 linked to lactose. The disaccharide epitope is in general cheaper to produce than the tetrasacharide epitope. More preferably the oligosaccharide sequence is Galβ3GalNAcβ with proviso that the disaccharide epitope is not linked to lactose or Galβ3GalNAcβ4Gal, with proviso that the reducing end Gal is not linked to glucose.

The novel ganglio receptors comprise the terminal disaccharide Galβ3GalNAc with the proviso that the disaccharide is not β4 linked to lactose. The disaccharide epitope is, in general, cheaper to produce than the tetrasacharide epitope. More preferably, the oligosaccharide sequence is Galβ3GalNAcβ with the proviso that the disaccharide epitope is not linked to lactose or Galβ3GalNAcβ4Gal, with the proviso that the reducing end Gal is not linked to glucose. The terminal disaccharide and trisaccharide sequences have not been previously described as receptors for diarrhea causing *E*. *coli* bacteria nor as receptors for EPEC-bacteria. The use of terminal disaccharides is preferred to the known tetrasaccharide receptors because of the more cost-effective synthesis.

### Galα4Gal- receptors

Preferred epitopes of the invention are Galα4Gal, Galα4Galβ4Glc and Galα4Galβ4GlcNAc. The present invention also shows that 3'-substituted forms of Galα4Gal-sequences such as the globoside and forssman antigen can be commonly recognized. Preferred Galα4Gal receptors comprise one or several oligosaccharide sequences according to the Formula

[GalNAcβ3]ₙ₁Galα4Gal {β4Glc(NAc)ₙ₂}ₙ₃ (XII)

wherein n1, n2, and n3 are independently integers 0 or 1, in a preferred embodiment with the proviso that either n1 is 1 or n3 is 1 and the GalNAc residue is optionally further substituted by other monosaccharide or oligosaccharide residues, preferably similar to natural oligosaccharide sequences such as Forssman antigen. More preferred oligosaccharide receptors are Galα4Gal, Galα4Galβ4Glc and Galα4Galβ4GlcNAc as these are synthetically more simple to produce, disaccharide Galα4Gal and pectin based oligosaccharide sequences according to the invention or other similar natural oligosaccharide sequences such as oligosaccharide sequence present in okra plant are especially preferred.

### Lacto-receptors

Preferred lacto series receptors comprise one or several oligosaccharide sequences according to the Formula

Galβ3GlcNAc[β3Gal{β4Glc(NAc)ₙ₁}ₙ₂]ₙ₃ (XIII)

wherein n1, n2, and n3 are independently integers 0 or 1. In preferred embodiments at least n3 is 1. Most preferred oligosaccharide sequences referred here as high affinity receptors include oligosaccharide sequences Galβ3GlcNAcβ3Gal, Galβ3GlcNAcβ3Galβ4Glc, Galβ3GlcNAcβ3Galβ4GlcNAc and Galβ3GlcNAcβ3Galβ3GlcNAc. The use of lactotetraose Galβ3GlcNAcβ3Galβ4Glc, optionally with other milk oligosaccharide such as Galβ4GlcNAcβ3Galβ4Glc and/or Galβ3(Fucα4)GlcNAcβ3Galβ4Glc and/or GlcNAcβ3Galβ4GlcNAcβ3Galβ4Glc, is especially preferred for therapeutical uses and especially for food, feed, and other nutritional uses.

The present invention finds out that the whole LNT sequence Galβ3GlcNAcβ3Galβ4Glc is preferably used for effective inhibition of the Lacto binding. Data in examples showed that the disaccharide epitope Galβ3GlcNAc, suggested in the prior art, alone could not support effective binding similarily as the epitope in the corresponding glycolipid. When the binding epitope was blocked by β6-GlcNAc in a neoglycolipid structure. The present invention is specifically directed to the use of Galβ3GlcNAcβ3Galβ4Glc as monovalent inhibitor and as soluble polyvalent inhibitor of diarrhea causing *E. coli.* It is realized that the substance can be useful even as a single substance as it is a frequent binding specificity.

### Neolacto-receptors

Preferred neolacto series receptors comprise one or several oligosaccharide sequences according to the Formula

[GlcNAcβ3]ₙ₁Galβ4GlcNAc[β3Gal{β4Glc(NAc)ₙ₂}ₙ₃]ₙ₄ (XIV)

wherein n1 n2, n3 and n4 are independently integers 0 or 1, when n1 is 1, the non-reducing terminal GlcNAc according to the formula can be further substituted by another monosaccharide residue or oligosaccharide residues, preferably by Galβ4 or GlcNAcβ3Galβ4. In preferred embodiments of the invention at least n4 is 1 or n1 is 1. Most preferred oligosaccharide sequences referred here as high affinity receptors include oligosaccharide sequences GlcNAcβ3Galβ4GlcNAc, Galβ4GlcNAcβ3Gal, Galβ4GlcNAcβ3Galβ4Glc, Galβ4GlcNAcβ3Galβ4GlcNAc, GlcNAcβ3Galβ4GlcNAcβ3Galβ4Glc, and GlcNAcβ3Galβ4GlcNAcβ3Galβ4GlcNAc. Preferred GlcNAcβ3Galβ4GlcNAc-structures include oligosaccharide sequences, which are β6-linked from the reducing end, especially GlcNAcβ3Galβ4GlcNAcβ6Gal, GlcNAcβ3Galβ4GlcNAcβ6GalNAc, GlcNAcβGalβ4GlcNAcβ6GlcNAc, GlcNAcβ3Galβ4GlcNAcβ6Glc and GlcNAcβ3Galβ4GlcNAcβ6Man. The use of neolactotetraose Galβ4GlcNAcβ3Galβ4Glc is especially preferred for therapeutical uses and especially for food, feed, and other nutritional uses.

The present invention finds out that the whole LNnT sequence Galβ4GlcNAcβ3Galβ4Glc is preferably used for effective inhibition of the Neolacto binding. Data in examples showed that the disaccharide epitope Galβ4GlcNAc, suggested in the prior art, alone could not support effective binding. The branched Galβ4GlcNAcβ3(Galβ4GlcNAcβ6)Galβ4GlcβCer could not support the binding even there is two of the disaccharide epitopes as the middle galactose is blocked by the branch. When the binding epitope was changed by a β6-structure in neoglycolipids GlcNAcβ3Galβ4GlcNAcβ3Galβ4Glc to GlcNAcβ3Galβ4GlcNAcβ6Galβ4Glc the binding was also very much weakened. The present invention is specifically directed to the use of Galβ4GlcNAcβ3Galβ4Glc and GlcNAcβ3Galβ4GlcNAcβ3Galβ4Glc as monovalent inhibitor and as soluble polyvalent inhibitor of diarrhea causing *E. coli.* It is realized that the substance can be useful even as a single substance as it is a frequent binding specificity.

A preferred embodiment of the invention is directed to uses of neolacto binding sequences comprising terminal-GlcNAc structures such as GlcNAcβ3Galβ4GlcNAc and GlcNAcβ3Galβ4GlcNAcβ3Galβ4Glc. It is realized that even the terminal disaccharide sequence GlcNAcβ3Gal can be used according to the invention, though with less activity.

It is also found for the first time that linear β3-linked poly-N-acetyllactoasmines, Galβ4GlcNAc[β3Galβ4GlcNAc]ₙβ3Galβ4Glc where in n is integer and n>=1, are receptors for diarrhea causing *E.coli* strains, the terminal Gal can be substituted by other monosaccharide residues, for example Neu5Xα3 or GlcNAcβ3. Preferred monovalent inhibitors comprises GlcNAcβ3Galβ4GlcNAcβ3Galβ4Glc, which has been reported from milk of buffalo, the common milk oligosaccharide Galβ4GlcNAcβ3Galβ4Glc and mixtures comprising GlcNAcβ3Galβ4GlcNAcβ3Galβ4Glc and Galβ4GlcNAcα3Galβ4Glc.

### Fucosyl-receptors

Preferred fucosyl receptors comprise one or several oligosaccharide sequences according to the Formula

Galβ3(Fucα4)GlcNAc[β3Gal{β4Glc(NAc)ₙ₁}ₙ₂]ₙ₃ (XV)

wherein n1, n2, and n3 are independently integers 0 or 1. In preferred embodiments at least n3 is 1. More preferred oligosaccharide sequences of the invention are Galβ3(Fucα4)GlcNAcβ3Gal, Galβ3(Fucα4)GlcNAcβ3Galβ4GlcNAc and Galβ3(Fucα4)GlcNAcβ3Galβ4Glc The use of Lewis a pentasaccharide Galβ3(Fucα4)GlcNAcβ3Galβ4Glc is especially preferred for therapeutical uses and especially for food, feed, and other nutritional uses.

### Sialic acid receptor

In the broadest sense the sialic acid receptor may be any sialic acid in natural type glycoconjugates. The sialic acid is preferably N-glycolyl-neuraminic acid or N-acetylneuraminic acid.

The present invention recognizes specific sialic acid which can bind effectively to the diarrhea causing pathogens, especially diarrhea causing *E. coli* bacteria.

The preferred sialic acid receptor oligosaccharide sequences are according to the Formula

Neu5XαpGalβr[(Fucαs)]ₙ₁Glc(NAc)ₙ₂ (XVI)

wherein independently X is either Ac or Gc meaning that the sialic acic is either Neu5Ac or Neu5Gc, n1 and n2 are either 0 or 1, p is linkage position 3 or 6,
r and s are linkage positions 3 or 4 with provision that when r is 3 then s is 4 and when r is 4 then s=3. More preferred oligosaccharide sequences includes one or several of the group:
Neu5Xα3Galβ3(Fucα4)GlcNAc, and Neu5Xα3Galβ4(Fucα3)GlcNAc,
Neu5Xα3Galβ4(Fucα3)Glc, Neu5Xα3Galβ3GlcNAc, Neu5Xa3Galp4GlcNAc,
Neu5Xα3Galβ4Glc, and Neu5Xα6Galβ4GlcNAc, Neu5Xα6Galβ4Glc wherein X is either Ac or Gc. The use of one or several of the milk type oligosaccharides such as Neu5Xα3Galβ3GlcNAcβ3Galβ4Glc, Neu5Xα3Galβ4GlcNAcβ3Galβ4Glc, sialyl-Lewis a hexasaccharide Neu5Xα3Galβ3(Fucα4)GlcNAcβ3Galβ4Glc or sialyl-Lewis x hexasaccharide Neu5Xα3Galβ4(Fucα3)GlcNAcβ3Galβ4Glc or sialyl-lactoses Neu5Xα3Galβ4(Fucα3)Glc, Neu5Xα3Galβ4Glc Neu5Xα6Galβ4Glc is especially preferred for therapeutical uses and especially for food, feed, and other nutritional uses. When the oligosaccharide sequences are used in human applications, it is preferred in a specific embodiment of the invention to use a natural human type of oligosaccharides
wherein X is Ac and Neu5X is therefore Neu5Ac. In another embodiment aiming for inhibition of human-animal cross-reactive strains with higher efficacy X is Gc and the sialic acid is NeuGc.

The present invention is specifically directed to exact sialic acid binding specificities toward sialyllactoses Neu5Xα3Galβ4Glc, Neu5Xα6Galβ4Glc, sialylactosamines Neu5Xα3Galβ4GlcNAc, Neu5Xα6Galβ4GlcNAc and their elongated forms Neu5Xα3Galβ4GlcNAcβ3Galβ4Glc, Neu5Xα6Galβ34GlcNAcβ3Galβ4Glc has not been previously characterized. The invention also showed effective inhibition of the binding specificities at reasonable low concentrations of oligosaccharides. In separate embodiment the present invention is specifically directed to use of Neu5Xα3- sialyllactose or sialyllactosamine, especially Neu5Xα3Galβ4Glc, Neu5Xα3Galβ4GlcNAc, Neu5Xα6Galβ4GlcNAc and Neu5Xα3Galβ4GlcNAcβ3Galβ4Glc for inhibition of diarrhea causing *E.coli.*
In separate embodiment the present invention is specifically directed to the use of Neu5Xα6- sialyllactose or sialyllactosamine, especially Neu5Xα6Galβ4Glc, Neu5Xα6Galβ4GlcNAc, and Neu5Xα6Galβ4GlcNAcβ3Galβ4Glc for inhibition of diarrhea causing *E.coli.* The Neu5Xα6-structures are especially preferred for their high activity. In a specific embodiment NeuNAc-containing oligosaccharides are used because their presence as natural sequence in human and human milk. In another embodiment NeuGc-containing oligosaccharides are used. The sialyl oligosaccharides from animal milks are especially preferred, especially sialyl-lactoses and Neu5Acα6Galβ4GlcNAc from bovine milk, furthermore a purified fraction comprising enriched amounts of one or several of the sialyl-oligosaccharides of bovine milk are preferred. A fraction containing Neu5Xα6-structures is especially preferred. The invention realizes for the first time the usefulness of the sialyl-oligosaccharides against human diarrhea, especially diarrheas according to the invention, especially when caused by *E. coli.* It is realized that the sialyloligosaccharides may be also used as single substances or as mixtures thereof.
It is realized that the sialyloligosaccharides are useful monovalent inhibitors of *E. coli* and can be used as polyvalent soluble conjugates. The sialic acid oligosaccharides may be used for inhibition of non-toxic *E.coli*.

In a separate embodiment the present invention is directed compositions comprising polysialic acid type sequences, preferably comprising oligosaccharide sequence Neu5NAcα8NeuNAc, called here polysialic acid compositions. The polysialic acid sequences in polysialic acid compositions may also or alternatively comprise oligosaccharide sequence Neu5NAcα9NeuNAc. Preferably the polysialic acid sequence is not present on a glycolipid type sequence. In another preferred embodiments the polysialic acid substance comprising the oligosaccharide sequences Neu5NAcα8NeuNAc and/or Neu5NAcα9NeuNAc also fulfil following criteria:
1. at least 95 % of sialic acid oligosaccharides are at least ten sialic acid residues long or
2. at least 95 % of sialic acid oligosaccharide are at least three sialic acid residues long or
3. at least 95 % of sialic acid oligosaccharides are less than ten sialic acid residues long and more preferably an oligosaccharide composition comprising at least 95 % of sialic acid oligosaccharides which are less than five sialic acid residues long or
4. at least 80 % of sialic acid oligosaccharides are at least two sialic acid residues long but less than less than six sialic acid residues long

Polysialic acid polysaccharide or oligosaccharides/precursors for oligosaccharide production can be produced by bacteria, for example by colomnic acid producing *E. coli.* The polysialic acid type oligosaccharide substances comprise Neu5NAcα8NeuNAc and/or Neu5NAcα9NeuNAc oligosaccharide sequences, preferably the polysialic acid-type oligosaccharide sequences comprises therapeutic oligosaccharides comprising Neu5NAcα8NeuNAc and/or Neu5NAcα9NeuNAc oligosaccharide sequences. The polysialic acid-type oligosaccharide substances comprise preferably two to ten sialic acid residues.
The present invention is also directed to polysialic acid-type oligosacharide substances or polysialic acid compositions for therapeutic uses or for use as medicine. The substances and compositions are especially directed for non-vaccine theraphautic uses and medicines. The present invention is also directed for use polysialic acid-type oligosacharide substances for preparation of medicines and therapeutic compositions against diarrheas and compositions for *ex vivo* uses as described by the present invention.

### Mannose receptor

The mannose receptor according to the present invention comprises ManaMan structures. The preferred mannose receptor oligosaccharide sequences are according to the Formula

Manαp[(Manαr)]ₙ₁Man (XVII)

wherein independently n is 0 or 1, p and r are linkage positions 3 or 6 between the Man residues, with proviso that when p is 3 then r is 6 and when p is 6 then r is 3. Preferred mannose receptor oligosaccharide sequences includes the structures: Manα3(Manα6)Man and Manα3Man. In a specific embodiment the oligosaccharide sequence is Manα3Manβ4GlcNAc or Manα3Manβ4GlcNAcβ4GlcNAc. In a preferred embodiment the reducing end residue of Manα3(Manα6)]Man is in open chain form, in reduced form or derivatized in open chain form, for example reductively aminated to a spacer or carrier. In a preferred embodiment mannose comprising mannan or phoshomannan oligosaccharide sequence is used. The mannan or phoshomannan comprises preferentially α-linked Mannose. The mannan or phosphomannan is preferably from non-harmful yeast such as baker's yeast (*S. cerevisiae*).

### Results about the binding specificities of diarrhea causing Helicobacter species

The major aim of the present invention is to provide therapies for diarrheas caused by various types of pathogens. The inventor chose diarrhea causing *Helicobacter* species to reveal receptor types which could be shared with totally different types of bacteria and could be involved even zoonotic infections spreading from other species. The zoonotic *Helicobacter* species are targets for developing also animal therapies, especially for preventing zoonotic infections. The present invention finds out special classes of receptors which are associated with risk of zoonotic infections. These include a family of galactose based receptors with possible sialic acid modifications.

The present invention is also related to *non-H.pylori Helicobacter* species, especially to enterohepatically infecting ones causing diarrheas and liver diseases. Typically these bacteria, referred as *zHelicobacter* (*zHe*/*icobacteria* in plural), are zoonotically active infecting both human and animals, such as cattle and pets, preferred pet animals are cats and dogs. In a separate embodiment the present invention is directed to the treatment of gastric infections caused by *zHelicobacteria.* The prior art is directed to different species of gastric bacteria such as *H. pylori, H. mustelae* (a non-zoonotic gastric pathogen of ferrets), and various non*-Helicobacter* species infecting the intestinal tract such as various types of *Escherichia coli* causing diarrheas. Different species of bacteria have different binding specificities and the receptors of *zHelicobacteria* are not known from prior art. Especially big differences could be expected between bacteria infecting different localizations in gastrointestinal tract or belonging to totally different families such as *Helicobacter* and *E. coli.* The present invention revealed different binding specificity profiles between *zHelicobacter* and *H. pylori.* The zoonotic bacteria reveal a specific group of receptors of zoonotic bacteria.

The group of *zHelicobacter* does not include species-specific human *Helicobacter pylori.* The present invention is further not directed to the infection of ferrets by *H. mustelae* as this is not an infection of a pet animal or cattle with a risk of zoonosis due to contact with human. The *zHelicobacteria* are inflecting human and/or, preferably and, pet animals of human and have zoonotic capacity to infect humans, especially persons with weak immune system. The present invention characterizes the carbohydrate binding specificities of *zHelicobacter* which are able to mediate the cross-species infective actions of the bacteria.

### Overview of results

The inventors analysed binding specificities of several *zHelicobacter* species towards a library of glycolipids in a TLC-overlay assay.

It has been established previously that both *H. pylori* and *H. mustelae* bind gangliotetraosylceramide binding was demonstrated for *H. felis, H. canis* and *H. hepaticus* and *H. bilis* (Table 3). Furthermore, in common with *H. pylori* we found that both gastric and enterohepatic *Helicobacter* spp. tested were capable of binding to lactotetraosylceramide, lactosylceramide with phytosphingosine and/or hydroxy fatty acids and isoglobotriaosylceramide. In contrast, binding to Le^{b} glycosphingolipid was only observed for *H. pylori* CCUG 17875 (Table 3).

The binding of certain *H. pylori* strains to slow-migrating gangliosides in the acid glycosphingolipid fraction of human granulocytes is sialic acid-dependent (Miller-Podraza *et al.,* 1999), and this fraction was therefore used as an indicator of sialic acid-recognition. The sialylated structures in human granulocytes are mainly NeuNAcα3Gal- and NeuNAcα6Gal. Binding to this fraction was noted for *H. hepaticus* CCUG 33637 (exemplified in Fig. 4B. lane 1) and *H. pylori* CCUG 17874 and occasionally for *H. mustelae* CCUG 25715 (Table 3). Sialic acid binding capacity assayed by other substances is also present in some species of *H. bilis.*

The *zHelicobacter* species were further observed to bind a linear polylactosamine glycolipid. The binding epitope is in the polylactosamine backbone as the removal of the specific terminal does not essentially effect the binding.

The present invention noticed that the carbohydrate specificities are also observable by various other methods in addition to the glycolipid assays. The binding were observable by assay involving protein type glycoconjugates even in cell based assay including traditional cell assay with cells from various species. These assays give results supporting the analysis of glycolipids.

### Preferred carbohydrate structures to be used against zoonotic infections of zHelicobacter according to invention

### β-Galactose based reseptors

According to the present invention the most common binding specificity of profile of the *zHelicobacter* species Galactoseβ3/4 -based receptor includes structures according to the formula:

[Galβy]ₚ[Hex(NAc)ᵣαz/βz]ₛGalβx[Glc(NAc)ᵤ]ᵥ (VI)

wherein p, r, s, u and v are each independently 0 or 1, and y is either linkage position 3 or 4, x is either linkage position 3 or 4, and z is either linkage position 3 or 4, and Hex is either Gal, or Glc,
so that
when v is 1 and u is 0 then x is 4,
when v is 0 then s is 1 and preferably also p is 1
when s is 0 the also p is 0 and v is 1
when p is 1, and y=3, Hex is Galβ or Glcβ and r=1, or p is 1 and y=4 and Hex is Glcβ and r=1 so that the terminal Gal is β3- or β4- linked to GlcNAcβ or the terminal Gal is β3-linked to GalNAcβ),
when p is 0 and z is 4, then Hex is Galβ and r is 1 so that the terminal monosaccharide structure is GalNAcβ4, or p =0 and z=3 so that the terminal is HexNAc/Hexα/β3),
when there is nonreducing terminal Galβ3/4, this can be further substituted by SAa3/6,
wherein SA is a sialic acid, preferably NeuNAc, N-acetylneuraminic acid.

### β-Galactose based reseptors, a combination formula:

Collectively the Galactoseβ3/4 -based receptors is an oligosaccharide sequence according to formula

[Galβy]ₚ[Hex(NAc)ᵣαz/βz]ₛGalβx[Glc(NAc)ᵤ]ᵥ (XVIII)

wherein p, r, s, u and v are each independently 0 or 1, and y is either linkage position 3 or 4, x is either linkage position 3 or 4, and z is either linkage position 3 or 4 or 6, and Hex is either Gal, Glc or SA (sialic acid),
so that
when v is 1 and u is 0 then x is 4
when v is 0 then s is 1 and preferably also p is 1,
when s is 0 the also p is 0 and v is 1
when p is 1, and y=3, Hex is Galβ or Glcβ and r=1, or p is 1 and y=4 and Hex is Glcβ and r=1 (the terminal Gal is β3- or β4- linked to GlcNAcβ or the terminal Gal is β3-linked to GalNAcβ),
when Hex is SA, z is either 3 or 6, preferably 3,
when p is 0 and z is 4, then Hex is Galβ and r is 1 (the terminal monosaccharide structure is GalNAcβ4), or p =0 and z=3 (the terminal is HexNAc/Hexα/β3), or
Hex is SA, z is 3 or 6 and the terminal structure is SAa3Gal or SAα6Gal.

In a preferred embodiment the Galβ-type receptor activity is a neutral oligosaccharide sequence not comprising sialic acid. In an embodiment the terminal p =0, Hex is sialic acid (SA), preferably, NeuNAc (N-acetylneuraminic acid) α3- or α6-linked.

### Preferred neutral galactose based receptors according to the invention

According to the present invention the most common binding specificity profile of the *zHelicobacter* species Galactoseβ3/4 -based receptor includes structures according to the formula:

[Galβy]ₚ[Hex(NAc)ᵣαz/βz]ₛGalβx[Glc(NAc)ᵤ]ᵥ (VII)

wherein p, r, s, u and v are each independently 0 or 1, and y is either linkage position 3 or 4, x is either linkage position 3 or 4, and z is either linkage position 3 or 4, and Hex is either Gal, or Glc ,
so that
when v is 1 and u is 0 then x is 4,
when v is 0 then s is 1 and preferably also p is 1
when s is 0 the also p is 0 and v is 1
when p is 1, and y=3, Hex is Galβ or Glcβ and r=1, or p is 1 and y=4 and Hex is Glcβ and r=1 so that the terminal Gal is β3- or β4- linked to GlcNAcβ or the terminal Gal is β3-linked to GalNAcβ),
when p is 0 and z is 4, then Hex is Galβ and r is 1 so that the terminal monosaccharide structure is GalNAcβ4, or p =0 and z=3 so that the terminal is HexNAc/Hexα/β3).

### Major receptor types According to the invention

The formula above is further divided to major structure groups including
4. Lactose/lactosamine type carbohydrate receptor
   This group further includes Lactose- receptors, and lactosamine receptors including Lacto-receptors, and Neolacto receptors
5. Ganglio-receptors
6. Sialic acid receptor

### Preferred lactose/lactosamine type receptors for zHelicobacter

[Galβy]ₚ[Hex(NAc)ᵣa3]ₛGalβx[Glc(NAc)ᵤ]ᵥ (VIII)

wherein p, r, s, u and v are each independently 0 or 1, and y is either linkage position 3 or 4, x is either linkage position 3 or 4, and a is either alpha or beta, and Hex is either Gal or Glc.
so that
when p is 1, Hex is Glcβ and r=1, and a is β (the terminal Gal is β3- or β4- linked to GlcNAcβ3)
when p is 0, then preferably
Hex is Gal, r is 0 and a is alpha (terminal structure is Galα3) or
Hex is Glc, r is 1 and a is beta (terminal structure is GlcNAcβ3)

In a preferred embodiment the lactose/lactosamine type receptors for *zHelicobacter* are according to the formula:

[Galβy]ₚ[GlcNAcβ3]ₛGalβx[Glc(NAc)ᵤ]ᵥ (IX)

wherein p, r, s, u and v are each independently 0 or I, and y is either linkage position 3 or 4, x is either linkage position 3 or 4, so that
at least p is 1 or v is 1,
when p is 1, s is 1
When u is 0, x is 4 and the reducing end Glc is preferably linked to hydroxyl.

Most preferred lactose/lactosamine structures include
the human milk tetrasaccharides Galβ4GlcNAcβ3Galβ4Glc and Galβ3GlcNAcβ3Galβ4Glc and lactosylceramides.
The preferred lactosamine structures also include
oligosaccharide sequences and oligosaccharides from the group Galβ4GlcNAc, Galβ3GlcNAc, Galβ4Glc, Galβ4GlcNAcβ3Gal, Galβ3GlcNAcβ3Gal,
And GlcNAcβ3Galβ4Glc, GlcNAcβ3Galβ4GlcNAc, Galβ4GlcNAcβ3Galβ4GlcNAc, and Galβ3GlcNAcβ3Galβ4GlcNAc.

The five receptor subgroups according to the invention for prevention of zoonotic pathogen especially for *zHelicobacter*
a) Lactose receptors
b) Lacto-receptors
c) Neolacto-receptors
d) Ganglio-receptors
e) Sialic acid-receptors

The present invention is also directed to the use of the five receptor types in combination so that at least 2 receptors are used. It is also preferred to use any of the receptor subtypes together with another receptor type. It is preferred to use Lactose receptor together with lactosamine receptor and/or ganglio-receptor and/or sialic acid receptor. It is further preferred to use Lactose/lactosamine receptor together with a ganglioreceptor and/or sialic acid receptor.

The present invention relates to a therapeutical composition comprising a purified fraction(s) of at least one, and in another embodiments of at least two or at least three compounds being or containing a pathogen inhibiting oligosaccharide sequence. When several oligosaccharide sequences are used, these are preferably selected from at least two, and in another embodiment from at least two, of the groups of pathogen receptors described above.

### Lactose receptors

In broadest sense lactose receptors are structures comprising oligosaccharide sequence Galβ4Glc. In a preferred embodiment lactose receptors are lactosylceramide receptors wherein the lactose structure is linked to a ceramide. More preferably there is a hydroxyl fatty acid structure present on the ceramide. The present invention is especially directed to the use of lactose receptors especially lactosylceramides comprising hydroxy fatty acids against *zHelicobacter* infections.

The lactosylceramide receptors according to the present invention means a lactose residue comprising molecule in which lactosyl residue is linked to a ceramide structure comprising a natural type of hydroxylfatty acid or alternatively lactosylceramide receptor means a mimetic structure of lactosylceramide in which the lactosyl residue is linked to a hydroxyl group comprising a ceramide-mimicking structure. The hydroxyl group of the hydroxyl fatty acid or ceramide mimicking structure preferentially forms a hydrogen bond with Glc-residues linked to ceramide or ceramide-mimicking structure. The lactosylceramide or mimetic structure can be substituted at position 3 or 4 of the Gal residue by natural type oligosaccharide sequences. The lactosylceramide receptor glycolipids also includes lacto-and/or neolactoseries glycolipids comprising a hydroxyl fatty acid. In other embodiments the present invention is also directed to the use of lacto- and/or neolacto- and/or ganglioseries glycolipids comprising a lactosyl residue and a hydroxylfatty acid. The present invention is also directed to the use of analogs of lacto- or neolactoseries oligosaccharide sequences linked to the hydroxyl group comprising ceramide-mimicking structure. The present invention is also directed to the use of analogs of ganglioseries oligosaccharide sequences linked to the hydroxyl group comprising ceramide-mimicking structure. In a preferred embodiment the invention is directed to the use of non-sialylated forms of lactosylceramide receptors according to the present invention. The preferred embodiments include molecules according to the following Formula

R₁xGalβ4GlcβR₂ (XIX)

wherein x is linkage position 3 or 4,
R₂ is ceramide comprising a hydroxyl fatty acid or an analog of a ceramide comprising a hydroxyl fatty acid and
R₁ is Gala, Galβ, GalNAcβ, GlcNAcβ or longer oligosaccharide comprising one of these residues at the reducing end or Neu5Xα with the proviso that preferably when R₁ is GlcNAcβ or Galα or Neu5Xa then x is 3 and Neu5X is sialic acid preferably Neu5Ac or Neu5Gc.

The present invention is also directed to substances and compositions comprising polyvalent conjugates of lactose receptor according to the invention and especially polyvalent conjugates of a mimetic structure of lactosylceramide according to the present invention. Especially polyvalent conjugates of mimetic structures of lactosylceramide are preferred when the lactosylceramide or mimetic structure of lactosylceramide is linked to a polysaccharide, optionally through a spacer group. In a specific embodiment the use of polyvalent conjugates are preferred over the use of lactosylceramide glycolipids. Use of glycolipids is more difficult as there is need to prevent the diffusion of the receptors to tissues. The prevention can be, however, achieved for example by incorporating the glycolipids in medical carbon matrix or in a stabile membrane or micellar structures.

It is realized that two or even three or more receptor binding specificities according to the invention can be presented by a single lactosylceramide receptor.

The present invention is also directed to the use of lactosylceramide comprising hydroxylfatty acids and analogs and derivatives thereof for therapy of gastrointestinal diseases, especially diarrheas and hepatobiliary diseases and more specifically diseases caused by *zHelicobacter* bacteria. In a preferred embodiment the present invention is directed to the use of a milk fraction comprising lactosylceramide comprising a hydroxylfatty acid. The milk is preferentially from a dairy animal such as a cow or any other dairy animal or milk producing animal which produces hydroxyl fatty acid-containing lactosylceramide. The prior art discussed above has been directed to the use of some milk glycolipids but the prior art does not realize the usefulness of the hydroxylfatty acid-containing glycolipids against diarrhea-causing *zHelicobacter* bacteria. The lactosylceramide receptors according to the present invention are especially useful for functional food or feeds as nutritional additives.

### Use of partial oligosaccharide sequences

In a separate embodiment one or several of the oligosaccharide sequences according to the present invention is/are replaced by a partial oligosaccharide sequences. The partial oligosaccharide sequence is in general less effective but can be used in higher concentrations. The partial oligosaccharide sequences are preferentially monosaccharides and more preferentially non-reducing pyranose formed monosaccharide residues having the same anomeric sructure as a terminal monosaccharide residue in a oligosaccharide sequence according to the present invention, more preferably the non-reducing pyranose formed monosaccharide residue is linked to a polyhydroxyl substance partially mimicking next monosaccharide of the corresponding oligosaccharide sequence. In a preferred embodiment the polyhydroxyl susbtance is a non-carbohydrate substance and most preferreably the polyhydroxyl substance is a flexible hydrophilic linker described by Formula 2 in this invention. Preferred partial oligosaccharide sequences include polyvalent conjugates and soluble polyvalent conjugates of the partial oligosaccharide sequences as described for the other receptor oligosaccharide sequences.

The partial oligosaccharide sequence is preferentially Manα, and more preferentially non-reducing pyranose formed Manα linked to a polyhydroxyl substance partially mimicking next monosaccharide of the corresponding oligosaccharide sequence. In another embodiment the partial oligosaccharide sequences is chosen from the group NeuNAcα, Galβ, Galα, Fucα, GlcNAcβ and terminal oligosaccharide sequence Fucα4GlcNAc optionally linked to a polyhydroxyl substance partially mimicking next monosaccharide of the corresponding oligosaccharide sequence. The partial oligosacharide sequences are preferably used together with low cost oligosaccharide sequences. Preferably one partial oligosaccharide sequence in pyranose form is used together with at least one, and preferably with two oligosaccharide sequences, and most preferably with three oligosaccharide sequences, according to the present invention. In another embodiment at least two partial oligosaccharide sequences are used with at least one oligosaccharide sequence according to the present invention. The partial oligosaccharide sequences are preferred for therapeutic uses according to the present invention, especially for feed and food uses.

### Defining most relevant carbohydrate binding specificities with regard to the natural infection cascade

As described below any carbohydrate specificity or specificities present on a pathogen cell surface can be used to inhibit the binding of a pathogen, for example by soluble polyvalent carbohydrates using the covering method as described by the present invention.
However, it is especially preferred to target such carbohydrate binding specificities which are directed to relevant receptors on the tissue which is infected. This is a preferred method when monovalent substances according to the invention are used. When soluble polyvalent conjugates are used for inhibition of a pathogen cell, and the most relevant carbohydrate specificities are used the polyvalent or even oligovalent conjugate need not be large like the conjugates which are used for achieving the sterical inhibition of other receptor interactions according to the invention. The present invention demonstrates several novel carbohydrate receptor structures on glycoproteins of human intestine and connects these to the binding specificitities shown by assays. In some cases the binding specificity of a certain intestinally pathogenic *E*. *coli* has been described but only the present invention shows its relevance to the infection by characterizing the natural receptor saccharides in human intestine. In a few cases combination of receptor structures and possible binding have been separately indicated to a certain extent. However, in these cases the characterization of potential receptors and binding specificities allow design of more effective receptor oligosaccharide sequences.

### Most relevant carbohydrate binding specificities of human intestine

Analysis of glycoproteins from human intestine revealed unexpectedly several interesting carbohydrate receptor structures. Combination of bacterial binding data and the presence of receptor allows defining of the biologically most useful therapeutic and diagnostic structures. The six binding specificities under this category also aim to use receptor specificities which are not so common in the normal useful bacterial flora.

### Mannose comprising N-glycans

Extraordinary structures such as N-glycan type structures comprising several mannoses and phosphate were characterized from glycoprotein samples of human gastrointestinal tract. Multi-mannose comprising N-glycans have not been characterized from human intestine. Presence of a phosphate residue is also a surprising feature. Phospho-mannans have been reported to bind certain biological carbohydrate receptors, but so far such structures have not been characterized to be present in human intestine nor as natural receptors in human intestinal tissues. The present data shows that a branched multi-mannose structure is a binding receptor for diarrhea causing *E.coli* bacteria. Previous data also indicates that certain bacteria such as *Escherichia coli* or *Salmonella typhimurium* can bind multi-mannose containing N-glycans. The present data concerning the presence of the mannose N-glycans in the intestine reveal the relevance of mannose binding to the pathogenesis. Substances inhibiting this binding, such as mannose or mannose analogues comprising carbohydrate oligomers or polyvalent carbohydrate conjugates, are especially effective because they can inhibit the relevant carbohydrate binding between the bacterium and human.

It is also realized that the novel multi-mannose receptors, especially phosphorylated multi-mannose receptors, can be used in analysis of pathogen binding to the receptor.

In a specific embodiment it is also realized that the multi-mannose receptors, especially phosphorylated multi-mannose receptors, can be used as receptors or substrates for probiotic bacteria, which adhere and bind or is able to degradate the receptor structure.

In a specific embodiment it is also realized that the multi-mannose receptors, especially phosphorylated multi-mannose receptors, can be used for diagnostic or analytical methods to analyze the bindings of intestinal pathogens to the receptor structure and smaller derivatives or anlogues thereof.

### Sialic acid comprising receptors and sialic acid binding specificities

Potential sialic acid comprising structures have not been characterized from human intestinal glycoproteins. The present invention shows several new sialylated structures and binding of diarrhea-causing *E*. *coli* to these structures. The sialic acid binding specificity of any diarrhea-causing *E*. *coli* has not been characterized in detail. The minor reports with only a few strains do not reveal the major sialic acid binding specificities according to the present invention and these specificities have not been connected with the receptor structures.

The present invention surprisingly shows that even N-glycolyl-neuraminic acid, not synthesized by human body, in various oligosaccharide chains can be effectively bound by *E.coli* bacteria infecting humans. It has been suggested that N-glycolyl neuraminic acid derived from foods can be present on human tissues. Even in case of vegans who do not eat animal based foods, the NeuGc binding is useful for the inhibition of the sialic acid binding or can be used as a polyvalent conjugate for sterical inhibition of other bindings as described by the present invention.

Surprisingly it could be shown that the sialic acid dependent binding specificity could be effectively inhibited by monovalent sialyl-lactose oligosaccharide.

Present invention was able to demonstrate the presence of protein linked sialylated first contact receptors in human gastrointestinal tract. The data show that the sialic acid-receptors are present and available for pathogen binding, showing the relevance of the receptors for pathogenesis, especially with regard to diarrhea causing E. *coli* infections.

### Galα4Gal- receptors of diarrhea causing E. coli.

This binding specificity has not been characterized for diarrhea-causing E. *coli* bacteria. Use of this oligosaccharide sequence has been known alone or as polyvalent non-soluble conjugates for prevention shiga-like toxins of EHEC. The failure of the approach depends probably on the failure to effectively inhibit the bindings of the EHEC. The non-soluble carrier does not allow the polyvalent inhibition as described by the present invention using the soluble polyvalent conjugates.

The difference in shiga-like toxin binding to adhesion is also shown by the ability of monovalent structures to inhibit and by the fine specificity of the binding. Preferred epitopes for shiga-like toxin inhibition are trisaccharides Galα4Galβ4Glc and Galα4Galβ4GlcNAc. According to the present invention the adhesin of diarrhea-causing *E. coli* also recognizes the disaccharide Galα4Gal, as this sequence can be produced more economically from natural polysaccharides than the trisaccharides. The present invention also shows that 3'-substituted forms of Galα4Gal such as the globoside and Forssman antigen can be commonly recognized by the adhesin while the recognition properties towards substituted Galα4Gal vary with toxins. The adhesin can be switched-on and switched-off in a bacterium.

In contrast to prior art with toxins the present invention shows effective inhibition of the Galα4Gal- binding by monovalent oligosaccharides. Inhibition of shiga-like toxin binding has been specifically reported not to be inhibitable monovalent Galα4Gal. The prior art does not describe the inhibition of one or several binding activities of EHECs together with the use of Galα4Gal. The present invention also shows that several binding specificities are also involved with EHEC infections. The prior art has not described the use of said sequence for treatment of other diarrheal diseases caused by other diarrhea causing *E.coli* bacteria. The roles of toxins, of which shiga-like toxins are only one class, vary in carbohydrate recognition specificities and infections caused by different types of *E.coli* such as EPEC, ETEC, etc.

The relevance of the epitope to natural infection is somewhat controversial, but the receptor may be present in the intestine or on the intestinal epithelium. The present invention is directed to the search of the epitope from intestinal proteins to confirm the relevance to the natural infections. Even if only small amounts of natural receptors would be present, the Galα4Gal-structures can be used as soluble polyvalent conjugates according to the invention to cover the bacterial surface and stererically block other adhesins of the bacterium.

### The fucosyl-receptors

The present invention also desribed a novel binding to fucosylated sequences such as the Lewis a structure. Such a binding has not been previously described for a diarrhea-causing *E.coli* bacterium. The Lewis a binding has not been previously described, but potential receptor structures are known from glycolipids of human intestine. Preferred inhibitors of the binding includes the human milk oligosacharide Galβ3(Fucα4)GlcNAcβ3Galβ4Glc.

Present invention was able to demonstrate the presence of protein linked fucosylated first contact receptors in human gastrointestinal tract. The data show that the fucosyl-receptors are present and available for pathogen binding, showing the relevance of the receptors for pathogenesis, especially with regard to diarrhea causing *E. coli* infections.

### Lacto-receptors and Neolacto-receptors

Present invention was able to demonstrate the presence of protein linked lacto- and neolacto-type first contact receptors in human gastrointestinal tract. The data show that the lacto-receptors and neolacto-receptors are present and available for pathogen binding, showing the relevance of the receptors for pathogenesis, especially with regard to diarrhea causing *E*. *coli* infections.

### General binding specificities also commonly present in normal flora

Lactosylceramide and ganglio-receptors are known to bind normal bacterial flora. The use of these receptors may also reduce normal flora or probiotic bacteria and are therefore more preferred to be used in combination with probiotic bacteria or probiotic substances. These receptors belong to the second contact receptor category and are most useful in connection to the other receptors described to be in the first contact receptors when the most effective treatment is needed. Galα4Gal structures can be also considered partially as normal flora binding structures. In a separate embodiment Galα4Gal structures are used together with probiotic bacteria.

### The lactosylceramide binding

The glycolipid receptor lactosylceramide comprising hydroxyl fatty acids is a novel receptor activity for diarrhea causing *E*. *coli.* This specificity includes 3'modified lactosylceramides, structures having modification or the elongation of the oligosaccharide chain on carbon 3 of the Gal residue in lactosylceramide. Lactosylceramide comprising hydroxyl fatty acids is known from intestinal tissue and considered as a general receptor for diarrhea causing *E*. *coli.*

### Refinement of the lacto-binding specificity and novel indications

Previously Galβ3GlcNAc has been proposed for EPEC inhibition by using neoglycoprotein comprising this disaccharide as a chemical non-natural conjugate. The present invention showed effctive binding to the longer oligosaccharide sequence Galβ3GlcNAcβ3Galβ4Glc. This tetrasaccharide can be used for inhibition of the lacto-binding of the diarrhea causing *E*. *coli* also in the monovalent state. The present invention shows that inhibiting the lacto binding of EHEC can be used for treatment of diseases caused by EHEC such as HUS, haemolytic uremic syndrome. Inhibition of the lacto-binding is also useful against ETEC, EIEC, and EAEC.

### Refinement of the neolacto-binding of E. coli and novel indications.

Previously Galβ4GlcNAc has been proposed for EPEC inhibition by using neoglycoprotein comprising this disaccharide as chemical non-natural conjugate. Lacto-N-neotetraose can inhibit binding of EPEC to a cultured epithelial cell line, but based on this finding the relevance of the binding cannot be shown. The glycosylations of the cultured cells vary and are not necessarily even close to natural glycosylation of the tissue from which it originates. According to the present invention it is possible to use lacto-N-neotetraose to inhibit EPEC binding. According to present invention the disaccharide sequence Galβ4GlcNAc and oligosacharide sequences comprising this disaccharide sequence can be used to inhibit EHEC, ETEC and other diarrhea-causing *E.coli*. The present invention also shows a novel variation for neolacto binding comprising terminal GlcNAc structures such as GlcNAcβ3Galβ4GlcNAc and GlcNAcβ3Galβ4GlcNAcβ3Galβ4Glc. It is also found for the first time that linear β3-linked poly-N-acetyllactoasmines, Galβ4GlcNAc[β3Galβ4GlcNAc]ₙβ3Galβ4Glc where in n is integer and n>=1, are receptors for diarrhea causing *E.coli* strains, the terminal Gal can be substituted by other monosaccharide residues. Preferred monovalent inhibitors comprises GlcNAcβ3Galβ4GlcNAcβ3Ga1β4Glc, which has been reported from milk of buffalo, and mixtures comprising GlcNAcβ3Galβ4GlcNAcβ3Galβ4Glc and Galβ4GlcNAcβ3Galβ4Glc.

### Novel indications for ganglio-receptor inhibitors of pathogens

Gangliobinding has been shown for several strains of EPECs and ETECs. The present invention widens the binding spectrum of the ganglio-saccharides to the EHEC type and especially to the EIEC and EAEC types of *E*. *coli.*

### Inhibition of pathogens by monovalent receptors

It is generally believed that the carbohydrate bindings to their receptors and especially the bindings of pathogenic bacteria are quite weak as monovalent interactions. It has been shown that for example binding of the Shiga-like toxin of *E*. *coli* to cultivated cells, can be only inhibited by very high density polyvalent carbohydrate conjugates of the Galα4Gal-sequence.

The present invention shows that especially the frequent binding specificities of diarrhea causing *E*. *coli* to Neolacto-receptors, Globo-receptors, Lacto-receptors and Sialic acid-receptors are specifically inhibitable. The inhibition can be achieved by relatively low concentrations of monovalent oligosaccharides. The oligosaccharides have inhibitory activity even at 0.3 mM final concentration under the in vitro testing conditions with natural glycolipid receptors. General useful concentration ranges estimated from the experiments are as follows. Useful concentration range is under about 3 mM more preferably under 2 mM, and even more preferably 1.5 mM or under. The invention is further directed to preferred concentrations under 1.0 mM and under 0.5 mM, a preferred concentration is of about 0.3 mM. The preferred lower limit of the concentration is above 0.005 mM more preferably above 0.010 mM and more preferably above 0.1 mM and most preferably above 0.1 mM. The invention is specifically directed to use of monovalent oligosaccharides at useful concentration as described, especially at concentrations under 2 mm and 1 mM. The concentration or the amount of the oligosaccharide can be further optimised for a specific oligosaccharide to avoid excessive use of the saccharides. The oligosaccharides may be used within preferred concentration ranges for monovalent inhibition so that the concentrations are adjusted closer to concentrations present in human milk or for example bovine milk. Some preferred concentrations and amount based on milk data are described elsewhere by the invention. The use of the oligosaccharides at milk concentrations is especially useful as these concentrations may be regarded as safe.
1. The bacterial adhesion binding specificities are inhibitable with monovalent oligosaccharides.
2. The bacterial adhesion binding specificities are inhibitable by relatively low concentrations of free monovalent oligosaccharide sequences.

The specific inhibition of the receptor binding specificities, especially the frequent binding specificities described by the examples further indicates that:
1. The binding specificities are separate and
2. The frequent binding specificities are independent of the glycolipid carrier or any other carrier structure
3. For therapeutic application each binding specificity toward receptor family described by the invention need to be inhibited separately.

The inventors further show by using combinations of the frequent binding specifities that the oligosaccharide inhibitors inhibit the bacterial binding simultaneously. The data indicates that
1.A single bacterial strain or batch can simultaneously express multiple inhibitable binding specificities. The prior art lacks experiments showing simultaneous binding and inhibition of the bacterial binding. As the invention also revealed that the binding specificities can be swithed on a single strain of and vary randomly between strains, any attempt to combine past data from different experiment performed at different times has no scientific relevance.
2.The tested oligosaccharides do not have interactions with each other which could prevent the simultaneous inhibition.

An approach using monovalent oligosaccharide sequences could save costs of synthesis when the construct is prepared. Polyvalent conjugates may also comprise non-natural and non-biodegradable linker structures which may cause side effects or regulatory problems. In general it is desired that the monovalent oligosaccharide should be active at low concentrations that would allow cost effective use of the oligosaccharide. The monovalent oligosaccharide means here also monovalent conjugates of the oligosaccharide, for example glycosylamines or glycosylamides or methyl glycosides or other glycosides including other N-glycosides, C-glycosides or S-glycosides, or for example active derivatives in which the reducing end is modified by reduction or reductive amination. If the reducing -end monosaccharide residue is reduced, it may be used as a spacer outside of the binding active carbohydrate epitope. Such an approach would require the use of an oligosaccharide which is at least one monosaccharide residue longer than the desired receptor epitope in the oligosaccharide sequence.

The present invention demonstrates that unexpectedly high affinity monovalent binding activities can be found and that monovalent carbohydrates can be used in relatively low concentrations to inhibit the bindings. Preferred monovalent substances comprise one or several terminal non-reducing end sequences chosen from the group: Galα4Gal, Galα4Galα4Gal, Galα4Galβ4Glc, Galα4Galβ4Glc, alpha-linked sialic acid, Neu5Acα, Neu5Acα3, Neu5Acα6, Neu5Acα3Gal, Neu5Acα6Gal, Neu5Acα9Neu5Ac, Neu5Acα8Neu5Ac, Galβ3GalNAc, GalNAcβ4Gal, Galβ3GlcNAc, Galβ3(Fucα4)GlcNAc, Galβ4GlcNAc, GlcNAcβ3Gal, and GlcNAcβ3Galβ4GlcNAc. More preferentially the monovalent substance or substances comprise(s) one or several terminal non-reducing end sequences chosen from the group: Galα4Gal, Galα4Galα4Gal, Galα4Galβ4Glc, Galα4Galβ4GlcNac, GalNAcβ3Galα4Gal, GalNAcβ3Galα4Galβ4Glc, Neu5Acα3Gal, Neu5Acα6Gal, Neu5Acα3Galβ4Glc, Neu5Acα6Galβ4Glc, Neu5Acα8Neu5Acα8Neu5Ac, Neu5Acα8Neu5Ac, Neu5Acα8/9Neu5Acα8/9Neu5Ac, Galβ3GalNAcβ4Galβ4Glc, GalNAcβ4Galβ4Glc, Galβ3GlcNAcβ3Galβ4Glc, Galβ3(Fucα4)GlcNAcβ3Galβ4Glc, Galβ4GlcNAcβ3Galβ4Glc, GlcNAcβ3Galβ4GlcNAc, Neu5Xα3Galβ3GlcNAcβ3Galβ4Glc, Neu5Xα3Galβ4GlcNAcβ3Galβ4Glc, Neu5Xα3Galβ3(Fucα4)GlcNAcβ3Galβ4Glc, Neu5Xα3Galβ4(Fucα3)GlcNAcβ3Galβ4Glc, Neu5Xα3Galβ4(Fucα3)Glc, Neu5Xα3Galβ4Glc Neu5Xα6Galβ4Glc.
Most preferentially the monovalent substance one or several terminal non-reducing end sequences chosen from the group: Galα4Gal, Galα4Galβ4Glc, Galα4Galβ4GlcNAc, GalNAcβ3Galα4Gal, GalNAcβ3Galα4Galβ4Glc, Neu5Acα3Galβ3GlcNAcβ3Galβ4Glc, Neu5Acα3Galβ4GlcNAcβ3Galβ4Glc, Neu5Acα3Galβ3(Fucα4)GlcNAcβ3Galβ4Glc, Neu5Acα3Galβ4(Fucα3)GlcNAcβ3Galβ4Glc, Neu5Acα3Galβ4(Fucα3)Glc, Neu5Acα3Galβ4Glc, Neu5Acα6Galβ4Glc, Galβ3GalNAcβ4Galβ4Glc, GalNAcβ4Galβ4Glc, Galβ3GlcNAcβ3Galβ4Glc, Galβ3(Fucα4)GlcNAcβ3Galβ4Glc, Galβ4GlcNAcβ3Galβ4Glc, and GlcNAcβ3Galβ4GlcNAc3Galβ4Glc.
This group comprises natural Galα4Gal sequences, natural type asialo ganglioside sequences and oligosaccharides which are present in animal or human milk. The preferred monovalent substances also comprise Manα3Man and Manα3(Manα6)Man oligosaccharide sequence structures.

In another embodiment the oligosaccharide sequences are chosen from cheap natural sources. Pectin oligosaccharides in which the carboxylic acid groups has been reduced is an example of low cost oligosaccharides, the reduced pectin oligosaccharides have the sequences Gal[α4Gal]n, wherein n is an integer from 1-about 10, it is noted that even larger oligosaccharides could be used but these are not so effective in general. Methods to reduce pectin in ester form, for example as a natural methanol ester, or by a carbodiimide have been reported. Large pectin polysaccharides can be degraded to oligosaccharides for example by chemical hydrolysis or enzymatically by pectinases. Galα4Gal oligosaccharide sequences or analogs or partial oligosaccharide sequences from natural sources for example from okra plant are also preferred for uses according to the present invention. The cheap natural sources also include polysialic acid produced by bacteria. These have polymeric sequences Neu5Ac[α8Neu5Ac]ₙ or Neu5Ac[α9Neu5Ac]ₙ or Neu5Ac with alternating α9- and α8-bonds. Polysialic acid may comprise intrachain binding and a specific embodiment is targeted to the use of polysialic acid as polymeric inhibitor. Polysaccharides can be degraded to oligosaccharides or lower molecular weight polysaccharides by methods known in art. Yeast mannan and phosphomannan or oligosaccharides derived thereof are preferred from low-cost natural sources for uses according to the present invention. The low-cost natural oligosaccharide sequences are especially preferred for nutritional, food and feed applications.

### Treatment of unknown pathogens

The carbohydrate compositions and substances are especially aimed for treatment of pathogen infections when the pathogen or pathogens causing the infection is or are not known. In many cases it is not possible to diagnose the pathogen and treatment has to be started before the results from diagnosis can be obtained. In under developed countries the diagnostics may not be available or may be too expensive. The availability of diagnostics may be also limited under war conditions or in distant regions with low populations. The compositions and substances according to the invention can be used to relieve symptoms of infections caused by numerous different pathogens. The present invention is especially directed to the treatment of diseases, preferentially gastrointestinal diseases such as diarrheas, when the pathogen is non-typable pathogen or pathogenic *E*. *coli.*

### Synergistic effects of manipulating several carbohydrate receptor bindings

The first synergistic effect is the unexpectedly high efficiency in inhibition or binding to a single pathogen which represent several adhesins binding to cell surfaces of a patient. In traditional inhibition attempts with single oligosaccharide epitopes the pathogen usually has additional carbohydrate binding specificities which may allow it to survive in the tissue. The prevention or inhibition of the binding is more effective when as many binding components as possible are inhibited. When a polyvalent conjugate is used the highest affinity part of the conjugate targets possible receptor oligosaccharide sequences with lower affinity to the surface of the pathogen. When the inhibition cover most of the binding mechanisms of the pathogen, the inhibition exeeds a threshold value allowing the pathogen mass to be flushed away by liquids of the tissue, causing a dramatic preventive effect against the pathogen. When the invention is used to inhibit simultaneously a microbe and a toxin involved in the same disease, the disease is relieved by two means, i.e. removal of both the bacterium and the toxin.

The use of two or more oligosaccharide sequences has also a synergistic effect against the development of resistance against the inhibition theraphy. The development of resistance is a common problem in current antibiotic theraphies. When there are limited amount of potential carbohydrate receptors for a pathogen in a target tissue, the therapies with two or more oligosaccharides can be used so that the bacteria have no choice left for the adhesion to the tissues.

When two or more oligosaccharides are used against several pathogens, synergistic inhibition effects are produced. When several pathogens are infecting simultaneously, the pathogens/infections are often supporting each other.
Besides the effects between pathogen and host tissues or between pathogens or pathogens and normal flora, the synergistic effect for inhibition of pathogens may occur by interaction with the immune defence of the patient. The pathogens may weaken the cells of the immune defence.

The coinfection situation may involve several carbohydrate interactions which can be manipulated. For example, cells infected by influenza virus are more effectively coinfected by several pathogenic bacteria of lungs. It has been suggested that sialidase of the influenza virus could reveal non-sialylated receptors for the bacteria on the infected lung cells. The virus may also use its hemagglutinin receptors for binding to granulocytes creating an interaction which can lead to dysfunction of the leukocyte.

### Methods involving synergistic effects to inhibit binding of pathogens

### 1.Synergistic effects of at least two receptor carbohydrates against a single pathogen which has several binding activities.

a) Simultaneous inhibition of at least two binding specificities present on the same pathogen effectively inhibits alternative binding specificities of the pathogen when at least two binding specificities are present at the same time.
b) Similarly, the inhibition of at least two binding specificities of a pathogen is desired when second binding specificity may arise in a situation when first binding specificity is inhibited. A cell pathogen like a bacterium may even be able to switch on the first binding specificity. While the first binding specificity may be switched off by inhibition using the covering method, which is described for polyvalent soluble carbohydrates in 3 below, it is not possible by single polyvalent carbohydrate against the first specificity. Such switching may occur for example by a phase variation of a bacterium. Switching the binding would be useful for a pathogenic cell which can use several carbohydate receptors since production of carbohydrate adhesins for receptors which are not present would consume energy unnecessarily. The inventors noticed that the binding specificities according to the invention can be switched off in strains of *E*. *coli* causing diarrhea. Therefore it is useful to use several binding oligosaccharide sequences, especially oligosaccharide sequences according to the invention to inhibit the binding of the bacteria causing diarrheas and other intestinal diseases. The invention reveals that any of the binding specificities of the numerous bacterial strains studied may be switched of by random fashion. During longer cultivation all the binding specificities may be switched off.
c) The invention reveals that the binding specificities are not stabily expressed in different strains of bacteria even among the same indication. This further emphasizes the need of multiple inhibitors, especially monovalent inhibitors for effective therapy.
d) Inhibition of the receptor on different levels of the infection. As a special case about the inhibition of pathogens, especially bacteria and viruses causing intestinal diseases such as diarrheas, the invention shows that it is useful to inhibit the pathogen binding on two receptor levels. The first binding interactions occur on the outer part of the carbohydrate matrix covering cell surfaces. This outer part comprises oligosaccharide sequences of glycoproteins, and possibly some polyglycosylceramide type of structures. The first binding interactions are here called first contact and the receptors involved in the first binding are here called first contact receptors. The second binding interactions, here called second contact, occur with medium sized and small glycolipids on the cell membrane surface. The small and medium sized glycolipid receptors are here called the second contact recptors. The prior art does not describe these structurally characterized oligosaccharide sequences as first contact bacterial receptors from human intestine or gastrointestinal tract.

The invention shows that several novel first contact receptors among the receptor types according to the invention are present on human intestinal mucin glycoproteins. The novel receptors include mannose-comprising oligosaccharide sequences, Galβ3GlcNAc, Galβ4GlcNAc, Lewis a, and sialylated glycoprotein oligosaccharide sequences. More preferred receptors are involved in the first binding interactions. The binding to the glycoprotein receptors is on a different level of the binding interactions than the binding to shorter chain oligosaccharide sequences of the cell surface glycolipids. It is noticed that Galβ3GlcNAc, Galβ4GlcNAc, Lewis a, and sialylated glycoprotein oligosaccharide sequences may also be present on long chain polylactosamine glycolipids and on shorter chain glycolipids. The carbohydrate structures which are totally or at least mostly expressed as second contact receptors include the lactosylceramide receptors, the ganglio-binding receptors and the globo-binding receptors.

According to a specific embodiment it is preferred to use the primary contact receptors, preferentially at least two of these to inhibit effectively primary contact and the infections. The present invention shows for the first time several first contact receptors from human intestine:
multi-mannose receptor, neolacto, lacto-, Lewis a and sialic acid binding receptors.

It is also realized that the novel first contact receptors are useful for search of other pathogen bindings towards these. When a binding structure has been found the receptor saccharide can be used for inhibitor design as described here for *E*. *coli* bacteria.

### Underfucosylated receptors

A preferred group among the preferred first contact receptors are underfucosylated receptors such as neolacto, lacto and Lewis a oligosaccharide sequences. These are more common in persons who are negative for fucosyltransferases like secretor α2-Fuc-T and Lewis-blood group fucosyltransferase. Persons with underglycosylated gastrointestinal tracts are more prone to infections. The present invention shows a reason for that and a potential therapy. Pathogenic *E. coli* can be inhibited by one or more of several of the following oligosaccharides or conjugates, preferentially polyvalent conjugates, thereof: Lacto-N-tetraose, Lacto-N-neotetraose, Galβ3(Fucα4)GlcNAcβ3Galβ4Glc.

The present invention describes for the first time theraphies for a novel indication, increased infections due to under modified lactosamine sequences, especially underfucosylation of epithelial lactosamine sequences. The invention is especially directed to treatment of persons who are Lewis fucosyltransferase (fucosyltransferase III) negative and or secretor fucosyltransferase negative. Similar underfucosylated sequences acting as pathogen receptors on epithelial cells can occur when a human patient is negative for other fucosyltransferases, especially fucosyltransferase V and/or fucosyltransferase VI. The present invention is directed to prevent intestinal pathogen adhesion by inhibiting pathogen or pathogens by carbohydrates comprising one or more oligosaccharide sequences chosen from the group neolacto receptors, lacto receptors and fucose receptors when the structures in a patient has increased.
In a preferred embodiment one or several more active elongated oligosaccharide sequences according to the formula Galβx(Fucα4)ₙGlcNAcβR, wherein independently x is linkage position 3 or 4, n is = 0 or 1, with the provision that when x=4, then n=0; and R is a monosaccharide residue or oligosaccharide or conjugate thereof, preferentially R, linked to GlcNAcβ, comprises 3Gal(NAc)ₘ and m is independently 0 or 1, are used. Preferred sequences are Galβx(Fucα4)ₙGlcNAcβ3Gal, Galβx(Fucα4)ₙGlcNAcβ3Galβ4Glc, Galβ3x(Fucα4)ₙGlcNAcβ3Galβ4GlcNAc, Galβx(Fucα4)ₙGlcNAcβ3Galβ3GlcNAc, Galβx(Fucα4)ₙGlcNAcβ3Galβ4GlcNAcβ3Galβ4Glc, Galβx(Fucα4)ₙGlcNAcβ3Galβ3GlcNAcβ3Galβ4Glc and Galβx(Fucα4)ₙGlcNAcβ3GalNAc.

Preferentially two oligosaccharide sequences are used. Preferred combinations of the two oligosaccharide sequences include non-reducing end terminal oligosaccharide sequences Galβ4GlcNAcβ3Gal and Galβ3GlcNAcβ3Gal, where these sequences represent both undermodified or underfucosylated type 1 and type 2 N-acetyllactosamines and serve as receptors for intestinal pathogens. Another preferred combination is Galβ3GlcNAcβ3Gal and Galβ3(Fucα4)GlcNAc comprising type 1 lactosamines which are especially common in intestine. In a preferred embodiment all three oligosaccharide sequences are used.

In the present invention it is realised for the first time that
1. Single pathogens, especially pathogenic bacteria infecting human gastrointestinal tract such as intestinal diarrheagenic E. *coli* bind to the limited number of specific oligosaccharide receptors present on the target tissue. Several receptor binding specificities are simultaneously functional.
2. The oligosaccharide sequences as polyvalent conjugates or in immunologically active compositions may also activate the immune defense, for example in intestine, which may target several types of pathogens such as bacteria or fungi. Carbohydrates can especially be used to activate non-specific immune defence reactions.
3. Polyvalent soluble carbohydrates comprising common carbohydrate receptor or receptors for carbohydrate binding activity present on a pathogen, which has possibility for several types of binding interactions with patient, can be used to coat the bacterium. When the surface of the bacterium is covered by the polyvalent soluble carbohydrate, the other binding interactions are sterically inhibited. The steric inhibition requires suitable molecular weight, in general the molecular weight should be high enough to be able to effectively inhibit, on the other hand the molecular weight in certain applications should be low enough to allow effective diffusion of the soluble carbohydrate. The covering soluble polyvalent carbohydrate can bind several pathogens together making an agglutinate which is removed for example with mucin secretion on lung or intestinal epithelium. Several pathogens can comprise several different species or strains of pathogens or several cells or several protein pathogens of the same species, strain or type.
4. The use of an inhibiting carbohydrate against a single pathogen in a coinfection situation can enhance the infections of other coinfecting pathogens which are not inhibited but are getting more room to expand. Prevention of one pathogen has also a synergistic effect against a coinfecting pathogen when there is an adhesion, which may be inhibited or used to flush several bacteria together. When using polyvalent soluble carbohydrates complexes may be formed between the coinfecting pathogens. When one or preferably at least two carbohydrates are used against all the coinfecting pathogens the infection is weakened much more effectively than when only one interaction is targeted. The synergistic effect of inhibiting coinfection by at least two carbohydrate sequences which can inhibit all the coinfecting bacteria is useful for the situation with at least two co-infecting pathogens for prevention of for example severe pneumonias or diarrheas.

### Preferred combinations of inhibitors of diarrhea causing E.coli

Because of the different contact levels in infections, combinations of first contact receptor oligosaccharides can be preferred. The first contact receptors are more easily available for bacterial binding and preferred targets for inhibition. The present invention is also directed to the treatment or prevention of infections, wherein the first contact receptors of the pathogen adhesion to human gastric epithelium is blocked, especially by using at least two of oligosaccharide receptors chosen from the group: lacto receptors, neolacto receptors, fucose-receptors, sialic acid receptors, and mannose receptors, more preferably at least three oligosaccharide receptors from the group are chosen and most preferably at least four oligosaccharide from the group are chosen. In another embodiment, at least one of the first contact receptors and more preferably at least two of the first contact receptors is/are used together with at least one of the second contact receptors in the group Galα4Gal-receptors, lactosylceramide-receptors and ganglio receptors. In a separate embodiment at least two second contact receptors chosen from the group Galα4Gal-receptors, lactosylceramide-receptors and ganglio receptors are used optionally with a probiotic bacterium or with a prebiotic substance.

The prevention or treatment of infections such as diarrhea caused by E. *coli* and diagnosis of diarrheagenic *E*. *coli* is also preferred by using at least two of oligosaccharide receptors chosen from the group: Galα4Gal-receptors, lacto receptors, neolacto receptors, fucose receptors, sialic acid receptors, and mannose receptors. These are preferred in certain cases because lactosylceramide-receptors and ganglio receptors are also associated with normal flora interactions.

In a preferred embodiment the prevention or treatment of infections such as diarrhea caused by *E*. *coli* and diagnosis of diarrheagenic *E*. *coli* is performed by using at least two oligosaccharide receptors chosen from the groups
a. lacto receptors, neolacto receptors and mannose receptors;
b. fucose receptors,Galα4Gal-receptors, and sialic acid receptors.

So that at least one oligosaccahride receptor is from group a and one is from group b. More preferentially at least two oligosaccharide receptors are used so that at least two oligosaccharide sequences are chosen from the group b. These are some preferred combinations of the pathogenesis specific receptors, which are especially aimed for treatment against specific diarrhea strains or disease types after analysis of the pathogen(s) causing the disease.

In a preferred embodiment the prevention or treatment of infections such as diarrhea caused by *E*. *coli* and diagnosis of diarrheagenic *E*. *coli* is performed by using at least two substances comprising different oligosaccharide sequences chosen from the oligosaccharides present in human milk or in milk of a dairy animal. Preferred free oligosaccharides from milks include several lacto receptors, neolacto receptors, fucose receptors, sialic acid receptors and the lactosylceramide receptor glycolipids. In a preferred embodiment the milk type oligosaccharide sequences are used together with one or several non-milk oligosaccharide sequences, more preferentially with one oligosaccharide sequences selected from the group consisting of Neu5Acα8Neu5Ac, Galα4Gal, and mannose receptor oligosaccharide sequences.

In a preferred embodiment the prevention or treatment of infections such as diarrhea caused by *E*. *coli* and diagnosis of diarrheagenic *E. coli* is performed by using at least two low cost substances chosen from the group Galα4Gal-receptors, lacto receptors, neolacto receptors, and mannose receptors, more preferably the low cost substances are a Galα4Gal-receptor, and a mannose receptor.

Use of at least two receptor oligosaccharide sequences according to the present invention when one of the oligosaccharide sequences is a fucose receptor according to the present invention is preferred because the sequence is a common natural first contact receptor.

Use of at least two receptor oligosaccharide sequences according to the present invention when one of the oligosaccharide sequences is a Galα4Gal receptor according to the present invention is preferred because the sequence is an especially effective receptor for *E*. *coli.*

Use of at least two receptor oligosaccharide sequences according to the present invention when one of the oligosaccharide sequences is a high affinity type neolacto-receptors according to the present invention is preferred because the sequences are an especially effective receptor for human diarrheagenic *E*. *coli.*

Use of at least two receptor oligosaccharide sequences according to the present invention when one of the oligosaccharide sequences is a high affinity type of lacto-receptors receptors according to the present invention is preferred because the sequences are an especially effective receptor for human diarrheagenic *E. coli.*

Use of at least two receptor oligosaccharide sequences according to the present invention when one of the oligosaccharide sequences comprises a Neu5Gc receptor according to the present invention is preferred because the sequence is an especially effective receptor for human diarrheagenic *E. coli.*

The combinations of the lacto receptors, neolacto receptors and fucose receptors are preferred as under-modified sequences as described above.

### Polyvalent carriers and conjugates

In a preferred embodiment the pathogen inhibiting/pathogen receptor oligosaccharide sequence or sequences are linked to a polyvalent carrier, more preferentially at least two pathogen inhibiting oligosaccharide sequences. In a specific embodiment at least two pathogen inhibiting oligosaccharide sequences are linked to the same polyvalent carrier. The polyvalent carrier is preferentially a carbohydrate carrier such as polysaccharide or an oligosaccharide, in a preferred embodiment the carbohydrate carrier is soluble carbohydrate carrier. The carbohydrate carrier is in a preferred embodiment a bacterial polysaccharide.

In another embodiment the pathogen inhibiting oligosaccharide sequence or pathogen inhibiting oligosaccharide sequences are expressed on a particle carrier. The particle carrier is preferably a carbohydrate particle, a synthetic polymer particle or a cell. The cell is preferably a bacterial cell or a yeast cell. The preferred diameter of the particle is between 10 nm and 10 micrometers.

The polyvalent conjugates are preferentially designed to be non-antigenic, and non-immunogenic, so that the only minor immune reactions or no immune reactions at all are caused by the conjugates. Other preferred properties of the polysaccharides include low toxicity of the polysaccharide and/or its degradation products.

The polyvalent conjugates which are aimed to inhibit bacteria are designed to avoid carbohydrates binding specificity or specificities of the epithelium when the carbohydrate binding specificity can attach the conjugate to the epithelium and increase the pathologic binding of the pathogen to the tissue.

Bacterial exopolysaccharides or capsular polysaccharides from bacteria are preferred, especially when the bacterium is a non-pathogenic bacterium such as lactic acid bacterium. Several of the oligosaccharide receptors according to the invention are known from bacterial polysaccharides. The invention is also directed to the engineering of the receptor oligosaccharide epitopes on bacterial polysaccharides, especially polysaccharides of non-pathogens such as lactic acid bacteria. The engineering may be done genetically or by chemical modification of the polysaccharides, for example by specific hydrolysis or glycosyltransferase reactions. According to present invention it is possible to use a bacterial polysaccharide or mixture of bacterial polysaccharides which comprises at least two receptor oligosaccharides according to the present invention. It is more preferred to use a bacterial polysaccharide or mixture of bacterial polysaccharides which comprises at least three receptor oligosaccharide sequences and in another embodiment at least four receptor oligosaccharide sequences according to the present invention.
Preferred polysaccharide substances comprising NeuNAcα6Gal- or NeuNAcα3Gal comprise NeuNAcα6Galβ4/3GlcNAc, NeuNAcα3Galβ4/3GlcNAc, NeuNAcα6Galβ4/3Glc or NeuNAcα3Galβ4/3Glc linked to a polysaccharide. Such polysaccharides with NeuNAc3Gal are already present on certain exopolysaccharides of type B *Streptococcus-* species. Similar polysaccharides can be expressed on non-pathogens such as lactic acid bacteria. NeuNAcα6Gal- containing species are more preferred since these can be produced by desialylating totally or partially NeuNAcα3Galβ4GlcNAc-containing polysaccharides and resialylating with a transferase sialylating at 6-position of Gal such as a α6-sialyltransferase. Alternatively a non-sialylated polysaccharide comprising terminal Galβ4/3GlcNAc or Galβ4/3Glc can be sialylated. Non-sialylated bacterial polysaccharides or polysaccharide derivatives comprising oligosaccharide sequences according to present invention such as Galβ4/3GlcNAc, Galβ4Glc, and Galβ3GalNAc or larger oligosaccharide sequences according to present invention are also preferred for use according to the present invention. In preferred embodiment partially sialylated polysaccharide comprising Galβ4/3GlcNAc or/and Galβ4Glc-sequences are used.

In another embodiment an antigenic or immuno stimulating or modulating carbohydrate conjugate is included. The antigenic or immunogenic carbohydrate conjugate is in a specific embodiment covalently conjugated to one or several oligosaccharide sequences according to the invention.

### Polyvalent conjugates for cross-linkingpathogens to immune cells or to immune defence proteins

Alternatively the carbohydrate compositions can be used to cross-link the pathogens to immune cells such as various types of leukocytes, or immune defence proteins such as antibodies, immune lectins or other pathogen inhibiting agents and thus inhibit the pathogen.

Several receptors for pathogens have been reported from the surfaces of immune cells. Preferred receptors on immune cells are aimed for destruction of the pathogen, such as phagosytosis receptors. The polyvalent or oligovalent oligosaccharide sequences are preferably not so large that they could prevent the phagosytosis or destruction of infecting pathogen. Such receptor includes mannose receptor on macrophages, receptors of natural killer cells which bind N-acetylglucosamine. It is obvious that several natural and synthetic carbohydrates can be used as analogs of these sequences. The preferred carbohydrates binding to the mannose receptors comprise terminal monosaccharide or monosaccharide analogs containg at least two free axial hydroxyl groups. The polyvalent carbohydrate sequences to be used for binding to immune cells include polyvalent conjugates comprising mannose, fucose, N-acetylglucosamine, N-acetylmannosamine or glucose. More preferentially the monosaccharides are chosen so that these are natural components present in human biology, mannose is D-mannopyranose, fucose is L-fucopyranose, N-acetyl-D-glucosaminopyranose, N-acetyl-D-mannosaminopyranose and glucose is D-glucopyranose. In the most preferred embodiment the monosaccharide residues are linked by natural type glycosidic bonds to neighboring monosaccharides such as Manα1-3, Manα1-6, or Manα1-2, GlcNAcβ1-3, GlcNAcβ1-2, GlcNAcβ1-6, Fucα1-2, Fucα1-3, Fucα1-4, Fucα1-6. Preferred oligosaccharide sequences comprise the terminal disaccharides Manα1-3Man, Manα1-6Man, Manα1-2Man, GlcNAcβ1-3Gal, GlcNAcβ1-2Man, GlcNAcβ1-6Gal, Fucα1-2Gal, Fucα1-3GlcNAc, Fucα1-4GlcNAc, or Fucα1-6GlcNAc.

In a specific embodiment a polymeric carbohydrate which has binding specificity towards both the pathogen or several pathogens and a pathogenesis inhibiting immune cell or leukocyte is used to cross-link a pathogen and an immune cell. Specifically an alpha-mannose containing carbohydrate is used for binding of bacteria such as *Salmonella* species or *E. coli* and leukocytes/complement system simultaneously.

In another embodiment the polyvalent substances comprising at least two different oligosaccharide sequences according to the invention is used for simultaneous binding of one or more types of pathogens and one or more types of immune cells capable of inhibiting the pathogen or the pathogens. More preferably one of the oligosacharide sequences in the polyvalent substance comprising at least two different oligosaccharide sequences according to the invention is a Manα- comprising oligosaccharide sequence.

### Soluble polyvalent conjugates covering and agglutinating the pathogens

Preferred polyvalent conjugates include soluble or gel forming polyvalent conjugates. More preferably the polyvalent conjugate is soluble and can cover the surface of the bacterium. Preferrably the bacterium covering soluble polyvalent conjugate has at least a molecular weight of 5000 daltons, more preferably at least about 10 000 daltons and most preferably at least about 20 000 daltons. For several applications higher molecular weights should be limited because of the effective diffusion of the conjugates in the gastric mucosa. Preferred upper limits of the polyvalent conjugates are under about 300 000 daltons, more preferably under about 150 000 daltons and most preferably under 50 000 daltons. More preferred molecular weight ranges include from about 5 000 to about 50 000 daltons, from about 10 000 daltons to 50 000 daltons and most preferably from about 20 000 daltons to about 100 000 daltons. The molecular weight limits indicate that about at least 70 % of the molecules are within the desired range and more preferably at least 80 % in the desired range.

The polyvalent conjugates that can diffuse to the surface of the pathogen and cover it are especially effective in prevention pathogens when several types of bindings should be inhibited. The polyvalent conjugate or conjugates comprises carbohydrate corresponding to the most common binding activity on the pathogen or pathogens present. The covering of the surface by the polyvalent conjugate blocks sterically the other carbohydrate binding receptor or receptors on the surface of the pathogen or the pathogens. Preferably at least two pathogen covering polysaccharides are used. More preferably two different receptor oligosaccharide sequences are conjugated to the same polymer.

Most preferably the soluble polyvalent conjugate comprises a polysaccharide backbone.

The present invention is thus directed to polyvalent substances, especially soluble polyvalent substances comprising at least two receptor oligosaccharide sequences, more preferably at least three receptor oligosaccharide sequences according to the present invention. The present invention is also directed to the polyvalent substances comprising at least four receptor oligosaccharide sequences according to the present invention.

### Polyvalent conjugates which can induce carbohydrate binding towards itself

The present invention is also directed to carbohydrate binding specificities which can be induced in pathogen cells by polyvalent conjugates which mimic the polyvalent natural surfaces to which the pathogens aim to attach. The inducible binding specificities are not active all the time but can be activated when pathogen needs to bind the receptor. According to the present invention pathogen cells, especially bacteria such as *Escherichia coli,* are able to activate such inducible receptor carbohydrate binding by contact with the receptor. A mechanism for the induction is presence of low amounts of the indicible receptors on the cell surface, which signals induction of the receptors in higher amounts.

For the induction of the receptor the polyvalent conjugates as described above can be used. High molecular weight conjugates are prefererred when the target pathogen cell is accessible for higher molecular weight molecules in the mucin layer. For this application even non-soluble polymeric conjugates can be used when the target pathogen cell is accessible for these.

### Therapeutic targets

The present invention is preferrably targeted for treatment of intestinal infections or lung infections. The term treatment means also preventive or prophylaxis treatments. Similarly, the invention could be used for treatment of oral or other gastrointestinal infections or treatment of infections of other epithelia or surfaces of the body of the patient, such as skin, or on genital surfaces such as the vagina. The invention can be even used in blood circulation of the patient, but then special care must be taken for the suitability of the substances and compositions for such use.

The invention is especially and preferentially directed to treatment of intestinal pathogens. The preferred intestinal pathogens cause diarrhea diseases. Preferred diarrhea causing pathogens includes all types *Escherichia coli* which cause intestinal diseases. The use of the compositions against *Escherichia coli* species including EPEC (enteropathogenic *Escherichia coli*), ETEC (enterotoxigenic *Escherichia coli*), EHEC (enterohemorrhagic *Escherichia coli),* EAEC (enteroaggregative *Escherichia coil*) and EIEC (enteroinvasive *Escherichia coli).*

### Treatment and prevention of other non- E.coli infections and co-infections

It is realized that several other pathogens live in similar receptor environment, in human or even in animals, especially in gastrointestinal tract, especially in intestinal receptor environment, comprising carbohydrate receptors according to the present invention. Other, non-*E. coli,* pathogens infecting human especially human gastrointestinal tract, more specifically ones infecting the human intestine, are likely to use one or several of the receptor oligosaccharide sequences according to the present invention. Treatment of other pathogen or other pathogens according to the present invention is preferred when the pathogens bind to at least two, more preferably at least three, receptor oligosaccharide sequences according to the present invention and when there is specifical benefits for using the oligosaccharide, sequences as described by the present invention. Thus, the present invention is generallyt directed to the use of compositions comprising of compounds which comprise at least two receptor oligosaccharide sequences according to the present invention against pathogens in human gastrointestinal tract, especially in intestine.

When the pathogen is a not *E. coli* the pathogen may use or bind other receptors or analogous oligosaccharide sequences, the other oligosaccharide sequences, referred here as other receptor oligosaccharide sequences, including preferentially other oligosaccharides such as sequences Fucα2Gal, Fucα3GlcNAc, Fucα3Glc, ganglioseries gangliosides, and/or NeuNAcα8NeuNAc, more preferably the fucosylated sequences are Fucα2Galβ3/4GlcNAc, Fucα2Galβ4Glc, Fucα2Galβ4(Fucα3)Glc, Galβ4(Fucα3)GlcNAc, Fucα2Galβ3/4(Fucα4/3)GlcNAc. The present invention is directed to the use of compositions comprising of compounds which comprise at least two receptor oligosaccharide according to the present invention together with at least one of the other receptor oligosaccharide sequences against pathogens, especially non-*E. coli* pathogens in human gastrointestinal tract, especially in intestine. The present invention is also directed to the simultaneous treatment of infections caused by at least one diarrheagenic *E*. *coli* and at least one non-*E.coli* pathogen.

Fucα2Gal-structures bind also to other, non-*E. coli,* pathogens and they are useful for use in combinations with the substances according to the present invention. In a preferred embodiment Fucα2Gal-structure or Fucα2Gal-Xyl structure is derived from plant hemicellulose. The present invention is also directed to therapeutic substance comprising Fucα2Gal-structure derived from plant hemicellulose. The therapeutic substance can be used in nutritional compositions including foods, feeds, beverages or in medicines or medicine like therapeutic compositions.

Other bacteria which can be targeted by the receptor carbohydrate combinations and polyvalent conjugates according to the invention include for example *Vibrio* species, including *Vibrio cholerae, Campylobacter* species, including *Campylobacter jejuni, Salmonella* species, including *Salmonella typhimurium, Listeria* species, *Shigella* species, *Aeromonas* species, intestinal viruses, especially rota virus, and intestinal eukaryotic parasites including the *Entamobae* species. The other intestinal pathogens have similar binding profiles with diarrhea causing *E. coli* as shown for example by studies with hemagglutination patterns with varios red cells. The other pathogens live in similar environment and use at least partially the same receptors as the diarrhea causing *E. coli.*

Many infections for example in intestine and lungs involve several pathogens. Such infections are difficult to treat and may turn into chronic infections. Many, diarrheas and lung diseases which may be mild infections in the beginning may develop into lethal forms of diseases. Complicated diseases are often caused by coinfection of several pathogens. It is especially preferred to use the carbohydrate compositions for treatment of two or more pathogenics, which infect or are considered to be infecting the patient. The compositions according to invention can be used to inhibit two or more pathogens from the group pathogenic bacteria, toxins, viruses, fungi, or parasites, simultaneously. More preferably the compositions according to the invention inhibit at least two pathogens from the group pathogenic bacteria, toxins, and viruses, simultaneously. A preferred combination of toxins and pathogens includes toxins *of Escherichia coli* and the *Escherichia coli-* bacteria. Toxin proteins comprises one or usually several lectins sites presented in ordered oligomeric manner. For example bacterial toxins such as cholera toxin or shigalike toxins contain five lectin domains in a ring-shaped protein pentamer. On bacterial surfaces the adhesion lectins or adhesins are presented in polyvalent manner. Also bacterial carbohydrates represent bioactive carbohydrate epitopes as large polyvalent conjugates like on exopolysaccharides or capsular polysaccharides or lipopolysaccharides or peptiglycans or like. No effective inhibitors are described for inhibition two or more different lectin representations. The preferred polyvalent or oligovalent conjugates are special carbohydrate conjugates.

According to the present invention it is also preferred to inhibit simultaneously two different pathogens. In a preferred embodiment the invention is targeted for treatment of coinfection by a virus and a bacterium. Preferably the bacterium or bacteria belong to species *Escherichia, Vibrio, Salmonella, Listeria, Shigella, Aeromonas,* or *Campylobacter* and it is most preferably a diarrhea causing *Escherichia coli* or several strains of *Escherichia coli* and the virus is a diarrhea causing virus such as a rotavirus. Other preferred combinations include lung pathogenic bacteria, preferably *Haemophilus influenzae, Klebsiella pneumoniae, Streptococcus pneumonia, Pseudomonas aeruginosa* and a lung infecting virus such as influenza virus.

The surfaces and adhesion mechanisms of the viruses and bacteria are different. The viral surface contains infective lectins in ordered surface structures on a relative small and curved surface of while the larger bacterial surface contains adhesive lectins usually in linear ordered structures like pili or flagella. Therefore the effective simultaneous prevention of viruses and bacteria is especially difficult. The present invention describes the use of special oligovalent or polyvalent compositions or substances which can be used for treatment of coinfections by viruses and bacteria. The oligovalent or polyvalent substances or compositions preferably comprise the active carbohydrates in special carbohydrate conjugates which can inhibit the bindings of two or more different lectin presentations on pathogen surface or pathogen surfaces.

### Use of the receptor oligosaccharide sequences alone or in combinations for the novel indication for carbohydrate

It is realized that the receptor oligosaccharide sequences can be used as or in single substances for therapy or other applications with regard to diarrhea causing *E. coli.* The present invention describes novel general diarrhea indication in which diarrhea is caused by one of the five major types of diarrhea causing *E. coli,* namely EPEC (enteropathogenic *Escherichia coli),* ETEC (enterotoxigenic *Escherichia coli),* EHEC (enterohemorrhagic *Escherichia coli),* EAEC (enteroaggregative *Escherichia coli)* and EIEC (enteroinvasive *Escherichia coli)* and even by non-typed or non-typable wild-type strains of diarrhea causing *E. coli.*

According to present invention the receptor oligosaccharides according to the present invention can be used as single substances or as parts of single substances for treatment of infections caused by any type of diarrhea causing *E. coli* and in a preferred embodiment for treatment of infections caused by all of the five major types of diarrhea causing *E. coli,* and in a more preferred embodiment embodiment for treatment of infections caused by at least four, and in a separate embodiment by at least three, of the major types of diarrhea causing *E. coli.* The oligosaccharide sequences are also preferred for preparation of therapeutical compositions for treatment of diarrheas caused by several types of diarrhea causing *E. coli,* in case a first indication for an oligosaccharide sequence has been suggested. When the oligosaccharide sequences are used alone, the theraphy is not as effective as according to present invention when combinations of the oligosaccharide sequences are used. As the general indication of using carbohydrate substances against infections caused by all or major types of diarrhea causing *E.* coli is new and inventive, the use of combinations of the receptor oligosaccharide sequences is even more new and inventive.

### Use of the most novel receptor oligosaccharides alone for therapies

Several oligosaccharide sequences has been suggested as inhibitors of specific types diarrhea causing *E. coli* in prior art, the data includes contradictory results and does not allow which of the substances could be used alone. The prior art does not show the relevance of the possible binding with regard to therapeutical use of even single binding specificity to make inhibitors of carbohydrate mediated pathogen binding. The prior work does not fulfil the simple primary criteria for theraphautically most relevant carbohydrate binding. The theraphautically useful carbohydrate mediated pathogen binding could be considered,
1) if a certain strain of pathogenic bacterium (or pathogen cell) has reprodicible binding specificity and
2) the binding specificity is present on the pathogen and
3) the corresponding receptor oligosaccharide sequence is present on the relevant target tissue and
4) the relevant receptor oligosaccharide sequence on target tissue is available for the binding specificities of the pathogen.

When considering usefulness of the therapeutics, the effects of the possible inhibitor oligosaccharide sequence must be established. The present invention shows useful substances and compositions for inhibition of pathogens. The prior art about potential bindings does not allow to determine effective inhibitors of pathogen binding according to the present invention. The present invention shows for the first time relevant first contact lacto-, neolacto-, fucosyl-, sialic acid-, and mannose receptors for diarrhea causing *E. coli* in human gastrointestinal tract. Lactosylceramide receptor and Galα4Gal-receptors for tissue binding of diarrhea causing *E. coli* has not been previously described, nor specific Galβ3GalNAc-binding. The approaches previously described for toxins does not cure the disease but can only possible relieve symptoms of the disease with specific strains of *E. coli.* The present invention is directed to use following groups of oligosaccharide sequences according to the invention also as single substances or as part of single substances for treatment of general and specific indications of diarrhea causing *E. coli.*
a) Lactosylceramide receptors
b) Galβ3GalNAc -receptors
c) Galβ4Gal-receptors
d) Lacto-receptors, preferably Galβ3GlcNAcβ3Gal-receptors
e) Neolacto-receptors, preferably (GlcNAcβ3)_{0 or 1}Galβ4GlcNAcβ3Gal-receptors
f) Fucosyl-receptors
g) Sialic acid-receptors
h) Mannose receptors

The oligosaccharide sequences are also preferred for preparation of therapeutical compositions for treatment of diarrheas caused by several types of diarrhea causing *E. coli,* in case a first indication for an oligosaccharide sequence has been suggested. When the oligosaccharide sequences are used alone, the theraphy is not as effective as according to present invention when combinations of the oligosaccharide sequences are used. The invention of relevance of the specific oligosaccharide sequences also adds the inventiveness of approaches using specific combinations of the oligosaccharide sequences. The relevant oligosaccharide sequences can be also used as monovalent and polyvalent inhibitors as described by the invention.

### Preferred receptor oligosaccharide sequences for therapy, prevention or treatment in connection to separate diarrhegenic E. coli indications

### Preferred and novel substances and compositions to be used against EHEC-infections

Present invention is also directed to the treatment of diseases caused by enterohemorrhagic *Escherichia coli.* Previously Galα4Gal-comprising substances have been described for blocking the shiga-like toxin of *E. coli.* Methods to use several oligosaccharide sequences especially to block the binding of the bacteria have not been previously described. In the present invention several strains of EHEC were screened. Special binding profile of preferred binding specificities were found. According to the present invention these specificities among the group of eight specificities are preferred for treatment of EHEC. Substances comprising following oligosaccharide sequences are preferred for the treatment of EHEC-infections: lactosylceramide receptors, ganglio receptors, lacto receptors, neolacto receptors, fucose receptors, and mannose receptors; more preferably lactosylceramide receptors, lacto-receptors, neolacto-receptors, and fucose receptors. The substances are also preferred in compositions comprising at least two receptor oligosaccharide sequences as described by the invention.

It is realized that the oligosaccharide sequences can be used together with toxin blocking oligosaccharide sequences such as Gala4Gal-type receptors of shiga like toxin. Several oligovalent and polyvalent oligosaccharides has been described to be effective inhibitors of the toxins especially when using Galα4Galβ4Glc and Galα4Galβ4GlcNAc-type sequences. In the light of previous studies the toxin blocking alone is not enough for effective treatment. According to the present invention the Galα4Gal-type oligosaccharides are not major adhesion receptors for EHEC-adhesion and for effective treatment.

In a preferred embodiment for EHEC prevention, treatment or diagnostics at least one of the receptors in the group of lacto-receptors, neolacto- receptors and fucose-receptors are used. More preferably at least two of the receptors are used. It is also preferred to use at least three or at least four receptors. These are preferred due to higher speficifity towards pathogenic organism. It is especially preferred to use the high affinity forms of the receptors selected from the group consisting of: lacto receptors, neolacto receptors and fucose receptors. The present invention is also directed to the treatment of EHEC infections by blocking the first contact receptors of the EHEC-adhesion to human gastric epithelium, especially at least one of the receptors selected from the group consisting of lacto receptors, neolacto receptors, fucose receptors and mannose receptors are used. More preferably at least two of the receptors are used. In another embodiment at least one of the first contact receptors and more preferably at least two of the first contact receptors is/are used together with at least one of the second contact receptors from the group of lactoreceptors or ganglioreceptors. In a preferred embodiment high affinity variant of the preferred receptor oligosaccharide sequences according to the present invention are used.

### Preferred and novel substances and compositions to be used against EPEC-infections

Present invention is also directed to the treatment of diseases caused by enteropathogenic *Escherichia coli.* Multiple non-characterized binding specificities have been suggested for EPEC. Therapeutical usefulness of these has not been demonstrated. The relevance of the bindings to the infection has not been shown and previous data does not allow to define useful compositions or substances among the ones indicated. Reports are contradictory and some reports indicate that the substances would not be useful for treatment. Methods to use several oligosaccharide sequences especially to block the binding of the bacteria have not been previously described. In the present invention several strains of EPEC were screened. Substances comprising following oligosaccharide sequences are preferred for the treatment of EPEC-infections: lactosylceramide receptors, Galα4Gal-receptor, sialic acid receptors, high affinity neolacto receptors and novel ganglio receptors.

The high-affinity neolacto-receptors comprise the terminal oligosaccharide sequences according to the Formula

[GlcNAcβ3]ₙ₁Galβ4GlcNAc[β3Gal{β4Glc(NAc)ₙ₂}ₙ₃]ₙ₄ (XIV)

wherein n1, n2, n3, and n4 are independently integers 0 or 1, when n1 is 1,
the non-reducing terminal GlcNAc according to the formula can be further substituted by other monosaccharide residue or oligosaccharide residues, preferably by Galβ4 or GlcNAcβ3Galβ4,
with the proviso that at least n4 is 1 or n1 is 1.

The novel ganglio receptors according to the present invention comprise the terminal disaccharide Galβ3GalNAc with the proviso that the disaccharide is preferably not β4 linked to lactose. The disaccharide epitope is in general cheaper to produce than the tetrasacharide epitope. More preferably the oligosaccharide sequence is Galβ3GalNAcβ with the proviso that the disaccharide epitope is not β4-linked to lactose or Galβ3GalNAcβ4Gal, with the proviso that the reducing end Gal is not β4-linked to glucose. The terminal disaccharide and trisaccharide sequences have not been previously described as receptors for diarrhea causing *E. coli* bacteria nor as receptors for EPEC-bacteria. The use of these is preferred to the known tetrasaccharide receptors because of the more cost-effective synthesis.

The substances are also preferred in compositions comprising at least two of the receptor oligosaccharide sequences as described by the present invention.

The present invention is specifically directed to the inhibition of the EPEC-binding and theraphy against EPEC infections by using at least one preferred EPEC-inhibiting oligosaccharide sequence chosen from the group: lactosylceramide receptors, Galα4Gal-receptors, lacto receptors, neolacto receptors, sialic acid receptors, and fucose receptors and more preferably chosen from the group: Galα4Gal-receptors, lacto receptors, neolacto receptors and fucose receptor, when the receptors are as described by the present invention. Furthermore the present invention is directed to the inhibition of EPEC-binding and theraphy against EPEC infections by using compositions comprising at least two or at least three of the preferred oligosaccharide sequences according to the present invention.

In a preferred embodiment high affinity variant of the preferred receptor oligosaccharide sequences according to the present invention are used. It is also preferred to use monovalent receptors and polyvalent receptor conjugates according to the present invention in connection with EPEC.

The present invention is also directed to combination of the receptor oligosaccharide sequences to be used in theraphy against EPEC together with an oligosaccharide sequence or oligosaccharide sequences which can inhibit the intimin receptor involved in the later stage of the infection cascade. The receptors of intimin have been described to be oligosaccharides with terminal structure Fucα2Gal, especially fucosyllactose Fucα2Galβ4Glc and Fucα2Galβ4GlcNAcβ3Galβ4Glc.

### Preferred and novel substances and compositions to be used against ETEC-infections

Present invention is also directed to the treatment of diseases caused by enterotoxigenic *Escherichia coli.* Multiple non-characterized binding specificities have been suggested for ETEC. Therapeutical usefulness of these has not been demonstrated. The relevance of the bindings to the infection has not been shown and previous data does not allow to define useful compositions or substances among the ones indicated. Reports are contradictory and some reports indicate that the substances would not be useful for treatment. Methods to use several oligosaccharide sequences especially to block the binding of the bacteria have not been previously described. In the present invention several strains of ETEC were screened. Substances comprising following oligosaccharide sequences are preferred for the treatment of ETEC-infections: lactosylceramide receptors, Galα4Gal-receptors, lacto receptors, neolacto receptors, sialic acid receptors, fucose receptors and the novel ganglio receptors. More preferably for the treatment or diagnostics of ETEC at least one oligosaccharide sequence is chosen from the group Galα4Gal-receptors, lacto receptors, neolacto receptors, and fucose receptors, when the receptors are as described by the present invention. Furthermore the present invention is directed to the inhibition of ETEC-binding and theraphy against ETEC infections by using compositions comprising at least two or at least three of the oligosaccharide sequences according to the present invention.

In a preferred embodiment high affinity variant of the preferred receptor oligosaccharide sequences according to the present invention are used.

It is also preferred to use monovalent receptors and polyvalent receptor conjugates according to the present invention in connection with ETEC.

### Preferred and novel substances and compositions to be used against EAEC-infections

The present invention describes novel binding specificities for enteroaggregative *Escherichia coli* (EAEC). It is realized that substances comprising the oligosaccharide sequences according to the each of the eight binding specificities of the present invention can be used for the treatment of infections caused by EAEC or diagnostics of the EAEC even as single substances. The present invention is also especially directed to the use one of the of the receptors in group Galα4Gal-receptors, lacto receptors, neolacto receptors, sialic acid receptors, and mannose receptors for infections caused by EAEC or diagnostics of the EAEC. More preferably at least two of the receptors are used. These are preferred due to higher speficificty towards pathogenic organism. It is especially preferred to use the preferred or high affinity forms of the receptor or receptors chosen from the group group lacto-receptors, neolacto receptors and fucose receptors.

The present invention is also directed to the treatment of EAEC infections by blocking of the first contact receptors of the EAEC-adhesion to human gastric epithelium, especially by using at least one of the receptor oligosaccharide chosen from the group: lacto receptors, neolacto receptors, fucose-receptors, sialic acid receptors, and mannose receptors. More preferably at least two of the receptors are used. In another embodiment at least one of the first contact receptors and more preferably at least two of the first contact receptors is/are used together with at least on of the second contact receptors in the group Galα4Gal-receptors, lactosylceramide-receptors and ganglio receptors.

In a preferred embodiment high affinity variant of the preferred receptor oligosaccharide sequences according to the present invention are used.

It is also preferred to use monovalent receptors and polyvalent receptor conjugates according to the present invention in connection with EAEC.

### Preferred and novel substances and compositions to be used against EIEC-infections

The present invention describes novel binding specificities for enteroinvasive *Escherichia coli* (EIEC). It is realized that substances comprising the oligosaccharide sequences according to the each of the eight binding specificities of the present invention can be used for the treatment of infections caused by EIEC or diagnostics of the EIEC even as single substances. The present invention is also especially directed to the use of at least one of the of the receptors chosen from the group: Galα4Gal-receptors, lacto receptors, neolacto receptors, sialic acid receptors, fucose-receptors, or mannose-receptors for infections caused by EIEC or diagnostics of the EIEC. More preferably at least two of the receptors are used. These are preferred due to higher speficificty towards pathogenic organism. It is especially preferred to use the high affinity forms of the receptors in group lacto receptors, neolacto receptors and fucose receptors.

The present invention is also directed to the treatment of EIEC infections by blocking of the first contact receptors of the EIEC-adhesion to human gastric epithelium, especially by using at least one of the receptor oligosaccharide chosen from the group: lacto receptors, neolacto receptors, fucose-receptors, sialic acid receptors, and mannose receptors. More preferably at least two of the receptors are used. In another embodiment at least one of the first contact receptors and more preferably at least two of the first contact receptors is/are used together with at least on of the second contact receptors in the group Galα4Gal-receptors, lactosylceramide-receptors and ganglio receptors.

In a preferred embodiment high affinity variant of the preferred receptor oligosaccharide sequences according to the present invention are used.

It is also preferred to use monovalent receptors and polyvalent receptor conjugates according to the present invention in connection with EIEC.

### Use of the methods and compositions according to the invention for animal theraphies

In a specific embodiment the invention is used for treatment of infections of cattle or pet animals. The binding specificities of animal infecting bacteria are different from the human pathogens. However, the general mechanisms using several specificities at the same time, and use of polyvalent conjugates, especially soluble polyvalent conjugates according to the invention, are also preferred for use with animals. The binding specificities are also partially cross-reactive and some of the receptor combinations described by the present invention are also useful for animal theraphies, and some bacterial strains spread from animals like cows. According to the present invention several of the receptor oligosaccharide sequences are present in gastrointestinal tract of animals such as cats and dogs and even in pigs. The cross-reactivity between a specific animal species or between a human and a specific animal for a specific strain cannot be known before the binding specificities are analysed. The most common *E. coli* bacteria causing infections in animals such as K99 or K88-strains do not infect humans.

The present invention is especially directed to prevention of the transfer of the infections from animals to human beings and vice versa. Such transfer is an important mechanism in pathogenesis of many diarrheas such as diseases caused by EHEC including the so called hamburger disease. When cattle or food products from cattle or pets or other animals are transferred between countries there are also risks for spreading infectious diseases such as diarhhea.

### Replacement of traditional antibiotics

The need of anti-infective therapies for animals is urgent as use of traditional antibiotics is not acceptable and even getting prohibited. The therapies aim to replace totally or partially the traditional antibiotics in animal nutrition and treatments.

The present invention show receptor sequences which are also described for animals living in proximity to humans and they probably have a role in the transfer of the infection from cattle to human. The present invention also shows actual binding of human diarrhea-causing *E. coli* bacteria to animal glycolipds. Some of the glycolipid receptors are same between animals and human intestinal tissues. The present invention is also directed to the receptors which are specific for animals, several animal specific receptor or receptors more common in animals. Preferred animals to which the invention is directed are major cattle or farm animals such as cows and other domestic ruminants, pigs, sheep, horses, poultry including for example hens, ducks and turkeys and rabbits or pet animals such as dogs, cats or rodents species including mice and rats or hamsters or guinea pigs and rabbits. Most of the common pet animal can be also used as laboratory animals, whose healthy is important for the experiments. Animals may also be in need of theraphy in nature or in sanctuaries or in zoos for example. Primates, especially chimpanzees and apes, are especially at risk of being infected by human pathogens as they are the most close human relatives. Most preferred animal species to be treated according to the invention are dogs, cats, pigs and cows.

The present invention is also directed to the search of animal specific receptors for diarrhea causing bacteria.

The N-glycolyl-neuraminic acid containing oligosaccharide sequences are mostly animal specific as biosynthetic enzymes making this structure are not present in humans. The human receptors comprising this monosaccharide are probably synthesized from the monosaccharide arising from animal foods. NeuGc is a common monosaccharide in many animals. Glycopeptides comprising this monosaccharide has been used against diarrhea in calves against animal specific K99 *E. coli.* Present invention describes several NeuGc-oligosaccharide sequences which can be used in animals when the animal is infected by cross-reactive *E.coli.*

### Ex vivo uses of the present invention

It is realized that the present invention can be used for inhibition of pathogens especially diarrhea causing *E*. *coli ex vivo* and such method has use in disinfection and preservation type applications. It is preferred to use the receptor oligosaccharide sequences according to the present invention as part of single substances or as single substances or more preferably as composition comprising at least two receptor oligosaccharide sequences from different groups according to the present invention for inhibition pathogens, preferably *E. coli ex vivo.* Polyvalent conjugates according to the present invention, especially soluble polyvalent conjugates which can agglutinate pathogens, preferably diarrheagenic *E. coli,* are preferred for *ex vivo* uses. One special *ex vivo* embodiment of the invention is the cleansing or disinfection of surfaces, e.g., of tables, medical devices and packages, in hospital or hospital-like enviroment with a cleanser or disinfectant containing the receptor oligosaccharide sequences described in the present invention. The receptor saccharides described by the invention can also be used as ingredients in a soap or detergent used for washing or bathing of patients in hospital or hospital-like enviroment.

### Oral infections and oral health products

It is realized that infections targetted by the present invention spread through oral route, possibly also from nose to the oral cavity. The present invention is directed to the prevention of the infections already in human mouth. The present invention is directed to the treatment of oral infections by at least two different oligosaccharide sequences which can inhibit at least two different binding specificities of pathogen, preferably orally infecting bacterium and more preferably a diarrhea causing bacterium. It is preferred to use the receptor oligosaccharide sequences according to the present invention as part of single substances or as single substances or as composition comprising at least two receptor oligosaccharide sequences from different groups according to the present invention for inhibition of oral or nasal infections. According to the present invention the receptor oligosaccharide sequences according to the present invention are used as compositions or as separate substances in products inhibiting pathogens, called here mouth hygiene products, in human mouth.

It is realized that human mouth comprises similar receptors as human intestine especially on proteins at least neolacto-receptors, mannose receptors and oligosaccharide receptors resembling fucose receptors according to the present invention. As a separate embodiment it is realized that the substances and compositions according to the present invention are also useful in inhibiting pathogens causing caries. In a specific embodiment the present invention is also directed to the compositions according to the present invention for treatment of other orally spreading infections such as infection causing otitis media or lung infections including influenza, bronchitis or pneumonia. The mouth hygiene products according to the present invention can also be directed against caries, otitis media, bronchitis and pneumonia. In a specific embodiment the composition to used in mouth hygiene product or for inhibition of a pathogen infecting orally comprises at least oligosaccharide sequences Neu5Acα3Galβ4GlcNAc and/or Neu5Acα3Galβ4Glc or more preferably Neu5Acα6Galβ4GlcNAc and/or Neu5Acα6Galβ4Glc and it is directed at least against human influenza virus, preferably for prophylaxis of influenza virus.

The present invention is especially directed to mouth hygiene products comprising substances or compositions comprising pathogen inhibiting oligosaccharide sequences, especially oligosaccharide sequences according to the invention. The mouth hygiene product is preferably selected from the group consisting of tooth pastes, mouth wash solutions, mouth tablets, chewing tablets, and chewing gums. It is preferred to use either monovalent receptor oligosaccharide sequences or polyvalent receptor oligosaccharide sequences. In another preferred embodiment the mouth hygiene product comprises polyvalent oligosaccharide sequences according to the present invention. Due to size of human mouth and volume of liquid saliva on its surface relatively small amount of oligosaccharides is enough to obtain saturating rating concentrations of pathogen inhibiting receptors in mouth. The typical amounts of receptor active monovalent epitopes varies from about 100 nmol to 100 µmol of the receptor active oligosaccharide, (at molecular weight 1000 Da this would be 100 µg to 100 mg). More generally useful amounts are estimated to be between about 1-10 µmol. In a separate embodiment the present invention about therapeutical composition is also directed to pathogen inhibiting nasal sprays. The nasal sprays can be directed against otitis media or lung infections.

### Topical, washing and cosmetic products

It is realized that the common pathogens can spread on human surfaces such as human skin, genital epithelia, hair, household surfaces, and other surfaces in human environment. The oligosacchride sequences according to the present invention are also useful for prevention of the pathogens also in these environments. It is therefore also preferred to use the oligosaccharide sequences according to the present invention as single substances, as part of single substances, or as composition comprising at least two receptor oligosaccharide sequences from different groups according to the present invention in topical or cosmetic products, for example as creams, lotions, or gels. It is also preferred to use the substances or compositions according to the present invention products aimed for washing human skin, hair or genital epithelia, (which can be also called as personal hygiene products), or for household surfaces, dishes or clothes. Traditional antibiotics have been aimed for use of household washing solutions, but are not useful because of resistance problems which are not likely with the substances according to the present invention. In preferred embodiment polyvalent oligosaccharide sequences are used for washing solutions, in another preferred embodiment monovalent oligosaccharide sequences are used for washing solutions.

### Food safety products to be applied to foods or feeds, beverages, drinks and water

Besides the therapeutic uses in humans or in animals the invention is also directed to the use of receptors and compositions according to the invention for the prevention of the infections by using the invention to neutralize pathogens or bacteria inside or on surfaces of food products. Carbohydrates according to the present invention can for example be applied on the surfaces of meat products or animal bodies, body parts in meat production to prevent the spreading of pathogens. Use of soluble and other polyvalent conjugates to cover and agglutinate the bacteria are preferred. A specific method to be used on a surface of a solid or semi-solid food product involves contacting the bacteria with the carbohydrates receptors described by the invention and optionally washing away the pathogen carbohydrate complexes. This kind of method is not acceptable with traditional antibiotics. The carbohydrates according to the invention can be also applied to liquid food products or concentrates or powder to make these including milk and liquid milk like products, various beverages including juices, soft drinks, sport drinks, alcoholic beverages and the like.

In a specific embodiment the carbohydrate according to the invention in polymeric form is applied to a liquid food product or a beverage product, potential pathogens are agglutinated by the polyvalent conjugate and the agglutinated complex is removed by a method based on size or solubility of the complex. Non-soluble agglutinates can be removed when these precipitate by standard methods like decanting the solution above the precipitate or more usually more effectively by filtration methods.
Filtration methods can be used to remove larger agglutinated complexes.

Preferred foods to be treated with carbohydrates according to the invention include various animal food products, especially meat products and middle products in processing. Many pathogens including diarrhea causing *E. coli* bacteria are transmitted effectively from vegetables, fruits, salads and other plant foods which are not properly washed. The food stuffs which need washing, but are not washed properly or washed with contaminated water are especially problematic in developing countries. The present invention is also directed to methods for increasing food safety of plant foods and other foods in need of washing to control the amount of pathogens, especially pathogenic *E. coli* bacteria in the food products. The invention is especially directed to home customer products and products aimed for the food industry to prevent infections from food. The product is preferentially in solid form as powder or pill or in a capsule containing solutions of the receptors according to the invention, which can be applied to food under processing. Such product can be used to prevent diarrheas in developing countries especially diarrheas in children. The food safety product is also directed to the prevention of travellers diarrheas. The food safety products and feed safety products below can be considered as novel safe preservatives.

### Filter products to puri beverages and water

Contaminated drinks and water are major cause of gastrointestinal diseases, especially diarrheas.

The receptors according to the present invention can be also used to make filters to purify pathogens, especially bacteria from liquid food and beverages and water, especially water used for drinking and preparing foods. Preferentially at least two recptor structures are used. Methods are known to produce solid phase materials to which carbohydrate sequences are conjugtated to be used as filters for example from cellulose or plastics or agarose and similar materials. The filters according to the invention also include affinity chromatography material known in the art. Methods to remove bound materials from such filters are known and in a specific embodiment the filter is regenerated by removing the contaminant and optionally sterilizing the filter by heat or other sterilizing means.

### Feed safety products

The food safety products described above can be also applied to animal solid and liquid feeds and drinking water of animals. Preferred target animals to be protected includes pet animals, especially cats and dogs and cattle or farm animal such as cows and other domestic ruminants, pigs, sheep, horses, poultry including for example hens, ducks and turkeys, and rabbits.

### Water, food and feed safety analytics.

Standard analytic and diagnostic methods in combination with the receptor carbohydrates according to the invention can be applied to water, beverages, foods and feeds to measure presence pathogens binding to the receptor carbohydrates. The knowledge of the binding specificities of contaminating pathogens can be applied to design of theraphy when patients are infected or to methods for food safety remove or control pathogens as described above.

### Other carbohydrate based interactions which can be inhibited according to the invention

Besides inhibiting different types of adhesin presentations the invention can be also used to inhibit carbohydrate-carbohydrate interactions and carbohydrate-lectin interactions.

The carbohydrate compositions and substances comprises of oligosaccharide sequences. The oligosaccharides inhibit one or several pathogens by binding one or several pathogens and/or by binding the receptors of one or several pathogens. Preferentially at least two pathogen inhibiting oligosaccharide sequences are used and more preferentially at least three pathogen inhibiting oligosaccharide sequences. In other embodiments at least four, five, six, or seven pathogenesis inhibiting oligosaccharide sequences are used.

In specific theraphies one or several of the oligosaccharide sequences are given separately at different time points. This is especially useful when the administration of all the oligosaccharide sequences would have negative effects on the normal flora. The separate administration of the therapeutic compositions can be useful also because of effect of nutritional situation in the gastrointestinal tract could change differently the stability of the on the oligosaccharide sequences according to the inventionin the gastrointestinal tract.

### Use of the invention together with probiotic bacteria

When the invention is used to inhibit bacterial binding, especially multiple bacterial bindings, also some beneficial bacterial bindings can be prevented. The normal bacterial flora has many important functions for example in the human gastrointestinal system. The destruction of the normal bacterial flora is however an even larger problem with use of traditional antibiotics.

In a separate embodiment at least two pathogen inhibiting oligosaccharides are administered together with a probiotic microbe and/or a prebiotic substance. The probiotic microbe according to the invention represent a non-harmful bacteria with beneficial functions, for example in digestion of food, providing nutrients and vitamins or covering tissue surfaces from pathogenic bacteria. The probiotic bacteria comprise preferentially one or several or multitude of normal bacterial flora. In a preferred embodiment the probiotic bacterium comprise one or several types, strains, or species of lactic acid bacteria.

The prebiotic substance is a substance supporting the normal flora or probiotic microbe. Preferred prebiotic substances include prebiotic carbohydrates, such as galactose oligosaccharides, xylose oligosaccharide, or fructose oligosaccharides used as prebiotic substances, the prebiotic substances also include polysaccharides and fibers with prebiotic acticities such as inulin or midified starches. The present invention is also directed to the use of other polysaccharides which are used in food or for nutritional purposes such as chitosan or beta-glucans for example glucan from oats, which are used to reduce cholesterol and fats. In a preferred embodiment one or several pathogen inhibiting carbohydrates are chosen so that they are also prebiotic substances like carbohydrates with a non-reducing terminal beta linked galactose residue. In a preferred form of therapy
a) pathogens and potentially part of the normal flora are first removed by one or more preferentially at least two carbohydrates according to the invention
b) probiotic microbe and/or prebiotic substance are applied.
Steps 1 and 2 may also be applied in reversed order, preferably with a large amount of the probiotic microbe and/or prebiotic substance and then step one. According to the invention it is also possible to repeat steps 1 and/or 2 several times while varying the order of the steps. Steps 1 and 2 may be applied at the same time. The substances according to the invention can be administered together with probiotic microbe and/or prebiotic substance or alternatively probiotic microbe and/or prebiotic substance can be included in the compositions according to the invention, and then steps 1 and 2 above can be performed simultaneously.

Some of the oligosaccharide sequences according to the invention are known to have prebiotic effects, these includes N-acetyl-lactosamine type oligosaccharide sequences, and fucosylated oligosaccharides, especially human milk oligosaccharides. Administration human milk oligosaccharides together with a probiotic microbe and/or prebiotic substance, especially N-acetyllactosamine containing for example one or several from the group Lacto-N-neotetraose, Lacto-N-tetraose, Lacto-N-hexaose, Lacto-N-neohexaose, para-Lacto-N-hexaose, para-Lacto-N-neohexaose, and/or fucosylated oligosaccharides derived from these such as mono-di- or trifucosylated Lacto-N-tetraose (LNT) or/or Lacto-N-neotetraose (LNnT) and/ or fucosyl-lactose oligosaccharides such as 2'fucosyl-lactose, and /or 3-fucosyllactose, and/or difucosyllactose is one embodiment of the invention.

### Other useful substances to be used with the substances and/or compositions according to the invention

According to the present invention it is also useful to use the pathogenesis preventing carbohydrate together with a glycosidase inhibitor.

According to the present invention it is also useful to use the pathogenesis preventing carbohydrate together with a lectin or another carbohydrate binding protein. The lectin can be used to block carbohydrate receptors, for example on the bacterial exopolysaccharides.

Hydroxyl substance means ceramide comprising hydroxyl fatty acid or more preferrably an analog thereof. The analog is preferably a spacer conjugating the oligosaccharide sequence to the carrier.

A preferred composion comprises mixtures human milk oligosaacharide backbones such as LNT and LNnT, and optimally with elongated or branched structures and/or natural sialic acid and fucose modifications.

*E. coli* means herein bacterium *Escherichia coli.* The *E.coli* or *Escherichia coli* which is targeted by the present invention means diarrhea causing *E*. *coli* or in other words diarrheagenic *E. coli.* The diarrheagenic *E. coli* means all types of *E. coli* including non-typed wild type strains of *E. coli* which cause diarrheas especially to humans. In more limited embodiments the diarrheagenic *E. coli* specifically includes the five major types of the diarrhea causing *E. coli,* namely EPEC (enteropathogenic *Escherichia coli*)*,* ETEC (enterotoxigenic *Escherichia coli*), EHEC (enterohemorrhagic *Escherichia coli),* EAEC (enteroaggregative *Escherichia coli)* and EIEC (enteroinvasive *Escherichia coli).* The abbreviations such as EHEC also mean multiple strains of the specific type of *E*. *coli,* multiple strains can be also indicated by letter s after the abbreviation like in "EHECs".

In this invention the terms "analog" and "derivative" are defined as follows. According to the present invention it is possible to design structural analogs or derivatives of the *Escherichia coli* binding oligosaccharide sequences. Thus, the invention is also directed to the structural analogs of the substances according to the invention. The structural analogs according to the invention comprise the structural elements important for the binding of *Escherichia coli* to the oligosaccharide sequences. For design of effective structural analogs it is important to know the structural element important for the binding between *Escherichia coli* and the saccharides. The important structural elements are preferably not modified or these are modified by very close mimetics of the important structural element. These elements preferably include the 4-, and 6-hydroxyl groups of the Galβ4 residue in the trisaccharide and oligosaccharide epitopes. Also the positioning of the linkages between the ring structures is an important structural element. For a high affinity binding the acetamido group or acetamido mimicking group is preferred in the position corresponding to the acetamido group of the reducing end-GlcNAc of the di- or trisaccharide epitopes. Acetamido group mimicking group may be another amide, such as alkylamido, arylamido, secondary amine, preferentially N-ethyl or N-methyl, O-acetyl, or O-alkyl for example O-ethyl or O-methyl.

The structural derivatives according to the invention are oligosaccharide sequences according to the invention modified chemically so that the binding to the *Escherichia coli* is retained or increased. According to the invention it is preferred to derivatize one or several of the hydroxyl or acetamido groups of the oligosaccharide sequences. The invention used to describe several positions of the molecules which could be changed when preparing the analogs or the derivatives. Preferred derivatives of the receptor oligosaccharide sequences according to the present invention include reducing-end derivatives of the oligosaccharide sequences. Multiple derivatization methods are known to link oligogosaccharides to other carbohydrates, aglycon molecules or various carriers. The C1-carbon of the reducing end monosaccharide residue can be linked through a sulphur, carbon or nitrogen atoms to other carbohydrates, aglycon molecules or various carriers, especially polyvalent carriers. Methods such as reductive amination can be used when the pathogen binding carbohydrate epitope is not destroyed by opening the reducing end monosaccharide residue. Derivatives of acetamido groups are also preferred.

Acetamido- groups can be deacetylated and derivatized for example by other carboxylic acids, the acetamido-derivatives can be screened for better pathogen binding. The derivatives can also be produced from precursors of the oligosaccharide to be derivatized for example from oligosaccharide sequences comprising hexosamine-residues. Methods to produce oligosaccharide analogs for the binding of a lectin are well known. For example, numerous analogs of sialyl-Lewis x oligosaccharide have been produced, representing the active functional groups on different scaffolds, see page 12090 Sears and Wong 1996. Similarly, analogs of heparin oligosaccharides has been produced by Sanofi Corporation and sialic acid-mimicking inhibitors such as Zanamivir and Tamiflu (Relenza) for the sialidase enzyme by numerous groups. Preferably the oligosaccharide analog is built on a molecule comprising at least one six- or five-membered ring structure, more preferably the analog contains at least two ring structures comprising 6 or 5 atoms.

In mimicking structures monosaccharide rings may be replaced rings such as cyclohexane or cyclopentane, aromatic rings including benzene ring, heterocyclic ring structures may comprise besides oxygen for example nitrogen and sulphur atoms. To lock the active ring conformations the ring structures may be interconnected by tolerated linker groups. Typical mimetic structures may also comprise peptide analog-structures for the oligosaccharide sequence or part of it.

The effects of the active groups to binding activity are cumulative and lack of one group could be compensated by adding an active residue on the other side of the molecule. Molecular modelling, preferably by a computer can be used to produce analog structures for the *Escherichia coli* binding oligosaccharide sequences according to the invention. The results from the molecular modelling of several oligosacharide sequences are given in examples and the same or similar methods, besides NMR and X-ray crystallographic methods, can be used to obtain structures for other oligosaccharide sequences according to the invention. It is also noted that the monovalent, oligovalent or polyvalent oligosaccharides can be activated to have higher activity towards the lectins by making derivatives of the oligosaccharide by combinatorial chemistry. When the library is created by substituting one or a few residues in the oligosacharide sequence, it can be considered as derivative library, alternatively when the library is created from the analogs of the oligosaccharide sequences described by the invention. A combinatorial chemistry library can be built on the oligosaccharide or its precursor or on glycoconjugates according to the invention. For example, oligosaccharides with variable reducing ends can be produced by so called carbohydrid technology. In a preferred embodiment a combinatorial chemistry library is conjugated to the *Escherichia coli* binding substances described by the invention. In a more preferred embodiment the library comprises at least 6 different molecules. Such library is preferred for use of assaying microbial binding to the oligosaccharide sequences according to the invention. Amino acids or collections of organic amides are commercially available and can be used for the synthesis of combinatorial library of acetamido analogs. A high affinity binder could be identified from the combinatorial library for example by using an inhibition assay, in which the library compounds are used to inhibit the bacterial binding to the glycolipids or glycoconjugates described by the invention. Structural analogs and derivatives preferred according to the invention can inhibit the binding of the *Escherichia coli* binding oligosaccharide sequences according to the invention to *Escherichia coli.*

### Neolacto-receptor analog trisaccharide epitopes comprising glucose at the reducing end

Steric hindrance by the lipid part or the proximity of the silica surface probably may limit the measurement of the neolacto-analogous epitope GlcNAcβ3Galβ4Glc in current TLC-assay. Considering the contribution of the terminal monosaccharide to the binding indicates that Glc could be allowed at the reducing end of the epitope. The trisaccharide epitopes with Glc at reducing end are considered as effective analogs of the *Escherichia coli* binding substance when present in oligovalent or more preferably in polyvalent form. One embodiment of the present invention is the saccharides with Glc at reducing end, which are used as free reducing saccharides with high concentration, preferably in the range 1-100 g/l, more preferably 1- 20 g/l. It is realized that these saccharides may have minor activity in the concentration range 0,1-1 g/l.

In the present invention the pathogen receptor or pathogen inhibitor by other words, especially receptors for diarrheagenic *Escherichia coli,* are described as oligosaccharide sequences. The oligosaccharide sequence defined here can be a part of a natural or synthetic glycoconjugate or a free oligosaccharide or a part of a free oligosaccharide. Such oligosaccharide sequences can be bonded to various monosaccharides or oligosaccharides or polysaccharides on polysaccharide chains, for example, if the saccharide sequence is expressed as part of a bacterial polysaccharide. Moreover, numerous natural modifications of monosaccharides are known as exemplified by O-acetyl or sulphated derivative of oligosaccharide sequences. The *Escherichia coli* receptor oligosaccharide sequence defined here can comprise the oligosaccharide sequence described as a part of a natural or synthetic glycoconjugate or a corresponding free oligosaccharide or a part of a free oligosaccharide. The *Escherichia coli* receptor oligosaccharide sequence can also comprise a mix of the *Escherichia coli* receptor oligosaccharide sequences. In a preferred embodiment the the oligosaccharide sequences according to the present invention are non-reducing terminal oligosaccharide sequences, which means here that the oligosaccharide sequences are not linked to other monosaccharide or oligosaccharide structures except optionally from the reducing end of the oligosaccharide sequence. The oligosaccharide sequence when present as conjugate is preferably conjugated from the reducing end of the oligosaccharide sequence, though other linkage positions which are tolerated by the pathogen binding can be also used. In a more specific embodiment the oligosaccharide sequence according to the present invention means the corresponding oligosaccharide residue which is not linked by natural glycosidic linkages to other monosaccharide or oligosaccharide structures. The oligosaccharide residue is preferably a free oligosaccharide or a conjugate or derivative from the reducing end of the oligosaccharide residue.

The pathogen receptor oligosaccharide sequences can be synthesized enzymatically by glycosyltransferases, or by transglycosylation catalyzed by glycosidase or transglycosidase enzymes (Ernst *et al.,* 2000). Specifities of these enzymes and the use of co-factors can be engineered. Specific modified enzymes can be used to obtain more effective synthesis, for example, glycosynthase is modified to do transglycosylation only. Organic synthesis of the saccharides and the conjugates described herein or compounds similar to these are known (Ernst *et al.,* 2000). Saccharide materials can be isolated from natural sources and modified chemically or enzymatically into the pathogen receptor compounds. Natural oligosaccharides can be isolated from milks produced by various ruminants. Transgenic organisms, such as cows or microbes, expressing glycosylating enzymes can be used for the production of saccharides.

In a separate embodiment the pathogen receptor substance, when the oligosaccharide is not an asialo-gangliosacharide or lacto-receptor or neolacto-receptor, may be conjugated to an antibiotic substance, preferably a penicillin type antibiotic. The pathogen receptor substance targets the antibiotic to pathogen. Such conjugate substance is beneficial in treatment because a lower amount of antibiotic is needed for treatment or therapy against *Escherichia coli,* which leads to lower side effect of the antibiotic. The antibiotic part of the conjugate is aimed at killing or weakening the bacteria, but the conjugate may also have an antiadhesive effect as described by the invention. Present invention is specifically directed to composition comprising at least two receptor oligosaccharide sequences according to the present invention as conjugates with a traditional antibiotic or several traditional antibiotics. The receptor oligosaccharide sequences and the antibiotic may be linked to a polyvalent carrier. The compositions are preferably targeted against gastrointestinal infection, more preferably agains diarrhea causing *E. coli.*

The pathogen receptor substances, preferably in oligovalent or clustered form, can be used to treat a disease or condition caused by the presence of the pathogen, preferably diarrhea causing *Escherichia coli.* This is done by using the *Escherichia coli* receptor substances for anti-adhesion, i.e. to inhibit the binding of *Escherichia coli* to the receptor epitopes of the target cells or tissues.

When the *Escherichia coli* binding substance or pharmaceutical composition is administered it will compete with receptor glycoconjugates on the target cells for the binding of the bacteria. Some or all of the bacteria will then be bound to the *Escherichia coli* receptor substance instead of the receptor on the target cells or tissues. The bacteria bound to the *Escherichia coli* receptor substances are then removed from the patient (for example by the fluid flow in the gastrointestinal tract), resulting in reduced effects of the bacteria on the health of the patient. Preferably the substance used is a soluble composition comprising the *Escherichia coli* receptor substances. The substance can be attached to a carrier substance which is preferably not a protein. When using a carrier molecule several molecules of the *Escherichia coli* receptor substance can be attached to one carrier and inhibitory efficiency is improved.

It is shown in the present invention that *Escherichia coli* can bind several kinds of oligosaccharide sequences. Some of the binding by specific strains may represent more symbiotic interactions which do not lead to severe conditions. Therefore total removal of the bacteria may not be necessary for the prevention of the diseases related to *Escherichia coli.* The less pathogenic bacteria may even have a probiotic effect in the prevention of more pathogenic strains of *Escherichia coli.*

According to the invention it is possible to incorporate the *Escherichia coli* receptor substance, optionally with a carrier, in a pharmaceutical composition, which is suitable for the treatment of a condition due to the presence *of Escherichia coli* in a patient or to use the *Escherichia coli* binding substance in a method for treatment of such conditions. Examples of conditions treatable according to the invention are and related gastrointestinal diseases, all, at least partially, caused by the *Escherichia coli* infection.

The pharmaceutical composition containing the pathogen receptor preferably diarrhegenic *Escherichia* coli-receptor substance may also comprise other substances, such as an inert vehicle, or pharmaceutically acceptable carriers, preservatives etc, which are well known to persons skilled in the art. The pathogen receptor, preferably diarrhegenic *Escherichia* coli-receptor-substance, can be administered together with other drugs such as antibiotics used against the pathogen or specifically *Escherichia coli.*

The pathogen receptor, preferably diarrheagenic *Escherichia* coli-receptor substance or pharmaceutical composition containing such substance, may be administered in any suitable way, although an oral administration is preferred.

The receptor oligosaccharide sequences according to the present invention are aimed for use in inhibition against pathogens, especially pathogenic bacteria, and the receptor oligosaccharide sequences are also referred as pathogen inhibiting oligosaccharide sequences. In more specific embodiments the pathogen is diarrhea causing *E. coli* and the receptor oligosaccharides are also referred as pathogen inhibiting oligosaccharide sequences or as *E. coli* receptor substances. The naming of the specific receptor oligosaccharide sequences and other longer terms may vary with regard to use of dash or capital letter as first letter, for example "lacto-receptor" and "lacto receptor" and "Lacto-receptor" and "Lacto receptor" mean the same.

The term "purified fraction" used herein relates purified or isolated oligosaccharide fraction from natural or synthetic sources. In a preferred embodiment the amount of the active oligosaccharide sequnce or oligosaccharide sequences is analysed and/or controlled from the fraction, optionally the amounts of other related carbohydrate structures are also analysed. The purified fraction has reduced amount of inactive substances originating from the source of the fraction, for example protein, monosaccharide precursors, lactose, or fat. Potentially harmful substances, such as harmful chemicals from synthesis, allergenic proteins, or substances considered ethically harmful, for example by religious or diet culture reasons, are removed to a level where these are not harmful in the final product. For medical use the purified fraction is preferably essentially pure (i.e. a purity of 98 % or better), or non-relevant substances are controlled and comprise preferably at least less than half of the mass of the purified fraction, more preferably less than 20% of the mass of the purified fraction and most preferably less than 5 % of the mass of the purified fraction. In a preferred embodiment of the invention, the production of the purified fraction from animal milk or milks involves at least partial removal of milk protein and/or fat. The purification may comprise filtration methods, such as gelfiltration or ultrafiltration, as well as drying and/or concentrating steps. For non-medical use the purified fraction is preferably essentially pure or the non-relevant substances comprise preferably at least less than 95 % of the mass of the purified fraction, more preferably less than 75% of the mass of the purified fraction and most preferably less than 25 % of the mass of the purified fraction. The purified fraction may be used as such or together with other ingredients of the desired product.

The term "treatment" used herein relates both to treatment in order to cure or alleviate a disease or a condition, and to treatment in order to prevent the development of a disease or a condition. The treatment may be either performed in a acute or in a chronic way.

The term "patient", as used herein, relates to any human or non-human mammal in need of treatment according to the invention. The present infection is especially directed for the treatment of intestinal infections, especially diarrheas, when the patient is a human patient.

It is also possible to use the pathogen receptor preferably diarrhegenic *Escherichia coli-*receptor substance in screening for substances that bind to the receptor substance, for example for screening of carbohydrates (by carbohydrate-carbohydrate interactions) that bind to the *Escherichia coli* receptor substance. The screening can be done for example by affinity chromatography.

Furthermore, it is possible to use substances specifically binding or inactivating the *Escherichia coli* receptor substances present on human tissues and thus prevent the binding of *Escherichia coli.* Examples of such substances include plant lectins such as *Erythrina cristagalli* and *Erythrina corallodendron* (Teneberg *et al.,* 1994). When used in humans, the binding substance should be suitable for such use such as a humanized antibody or a recombinant glycosidase of human origin which is non-immunogenic and capable of cleaving the terminal monosaccharide residue/residues from the *Escherichia coli* receptor substances. However, in the gastrointestinal tract, many naturally occuring lectins and glycosidases originating for example from food are tolerated.

### Nutritional, food and feed uses

Furthermore, it is possible to use the pathogen receptor oligosaccharide sequences or *Escherichia coli* receptor oligosaccharide as part of a nutritional composition including food- and feedstuff. It is preferred to use the receptor oligosaccharide sequences according to the present invention in isngle substances or as single substances and more preferably in composition comprising at least two receptor oligosaccharide sequences from different groups according to the present invention for nutritional compositions, foods or feed stuffs. It is preferred to use the *Escherichia coli* receptor oligosacharide sequences as substances or compositions as a part of so called functional or functionalized food. The said functional food has a positive effect on the person's or animal's health by inhibiting or preventing the binding of *Escherichia coli* to target cells or tissues. The *Escherichia coli* receptor substance or composition can be a part of a defined food or functional food composition. The functional food can contain other acceptable food ingredients accepted by authorities such as Food and Drug Administration in the USA. The *Escherichia coli* receptor substance or composition can also be used as a nutritional additive, preferably as a food or a beverage additive to produce a functional food or a functional beverage. The food or food additive can also be produced by having ,e.g., a domestic animal such as a cow or other animal produce the *Escherichia coli* receptor substance or composition in larger amounts naturally in its milk. This can be accomplished by having the animal overexpress suitable glycosyltransferases in its milk. A specific strain or species of a domestic animal can be chosen and bred for larger production of the *Escherichia coli* receptor substance or composition. The *Escherichia coli* receptor substance or composition for a nutritional composition or nutritional additive can also be produced by a microorganisms such as a bacteria or a yeast.

It is especially useful to have the *Escherichia coli* receptor substance or composition as part of a food for an infant, preferably as a part of an infant formula. Many infants are fed by special formulas in replacement of natural human milk. The formulas may lack the special lactose based oligosaccharides of human milk, especially the elongated ones such as lacto-N-neotetraose, Galβ4G1cNAcβ3Ga1β4G1c, lacto-N-tetraose, Galβ3GlcNAcβ3Galβ4Glc, and derivatives thereof. The lacto-N-tetraose, lacto-N-neotetraose para-lacto-N-hexaose (Galβ3GlcNAcβ3Galβ4GlcNAcβ3Galβ4Glc and para-lacto-N-neohexaose (Galβ4GlcNAcβ3Galβ4G1cNAcβ3Galβ4Glc) as well as Galβ3Galβ4Glc are known from human milk and can therefore be considered as safe additives or ingredients in an infant food. Sialylated and/or fucosylated human milk oligosaccharides and buffalo milk oligosaccharide GlcNAcβ3Galβ4GlcNAcβ3Galβ4Glc, described as pathogen receptors according to the present invention, are also preferred for functional foods and infant formulas. It is preferred to use combinations comprising at least two of the milk oligosaccharides. Diarrhea causing *Escherichia coli* is especially infective with regard to infants or young children, and considering the diseases it may later cause it is reasonable to prevent the infection.

Preferred concentrations for human milk oligosaccharides in functional food to be consumed (for example, in reconstituted infant formula) are similar to those present in natural human milk. It is noted that natural human milk contains numerous free oligosaccharides and glycoconjugates (which may be polyvalent) comprising the oligosaccharide sequence(s) described by the invention, wherefore it is possible to use even higher than natural concentrations of single molecules to get stronger inhibitory effect against *Escherichia coli* without harmful side effects. Natural human milk contains lacto-N-neotetraose at least in range about 10-210 mg/l with individual variations (Nakhla *et al.,* 1999). Consequently, lacto-N-neotetraose is preferably used in functional food in concentration range 0,01-10 g/l, more preferably 0,01-5 g/l, most preferably 0,1 - 1 g/l. Approximately 2-5 times higher amounts of lacto-N-tetraose can be used. Alternatively, the total concentration of the saccharides used in functional food is the same or similar to the total concentration of natural human milk saccharides, which bind *Escherichia coli* like the substances or composition described, or which contain the binding epitope/oligosaccharide sequence indicated in the invention.

Sialyl-lactoses and sialyllactosamines occur in bovine milk at concentrations from tens of microgram per ml to maximum of almost mg per ml of all three major oligosaccharides together NeuNAcα3Galβ4Glc, NeuNAcα6Galα4GlcNAc and NeuNAcα6Galβ4Glc in early colostrums (Nakamura et al 2003). The amounts of 0,01 - 10 g/l, more preferably 0,01 - 5 g/l, most preferably 0,1- 1 g/l are preferred for products. NeuNAcα6Galβ4Glc occurs in largest amount in bovine milk and it is also preferred as effective inhibitor against diarrheagenic *E. coli.*

Infant formulas also comprise, beside substances or compositions according to the present invention, other substances used in infant formulas such as fractions from ruminant milks such as proteins from whey or soy protein preparations or protein hydrolysates. The infant formula may also comprise other carbohydrates useful or accepted for infant formulas such as lactose or galactose oligosaccharides.

Preferably, the nutritional formulation of the present invention contains edible macronutrients, vitamins and minerals in amounts desired for a particular use. The amounts of such ingredients will vary depending on whether the formulation is intended for use with normal, healthy infants, children, adults or subjects having specialized needs such as those accompany certain pathological conditions (e.g., metabolic disorders). It will be understood by persons skilled in the art that the components utilized in a nutritional formulation of the present invention are of semi-purified or purified origin. By semi-purified or purified is meant a material which has been prepared by purification of a natural material or by synthesis. These techniques are well known in the art (See, e.g., Code of Federal Regulations for Food Ingredients and Food Processing; Recommended Dietary Allowances, 10th Ed., National Academy Press, Washington D.C., 1989).

In a preferred embodiment, the nutritional formulation of the present invention is an infant enteral nutritional product. Accordingly, in a further aspect of the invention, a nutritional formulation is provided that is suitable for feeding to infants. The formula comprises, in addition to the above described oligosaccharides, vitamins and minerals in amounts designed to provide the daily nutritional requirements of infants.

The macronutritional components include for example, edible fats, carbohydrates and proteins. Exemplary edible fats are coconut oil, soy oil, and mono- and diglycerides. Exemplary carbohydrates are glucose, food grade (edible) lactose and hydrolyzed cornstarch. A typical protein source would be for example, soy protein, electrodialysed whey or electrodialysed skim milk or milk whey, or the hydrolysates of these proteins, although other protein sources are also available and may be used. These macronutrients would be added in the form of commonly accepted nutritional compounds in an amount equivalent to those present in human milk on an energy basis, i.e., on a per calorie basis.

The infant formula would preferably include the following vitamins and minerals: calcium, phosphorous, potassium, sodium, chloride, magnesium, manganese, iron, copper, zinc, selenium, iodine, and Vitamins A, E, D, C, and the B complex.

The infant formula can be sterilized and subsequently utilized on a ready-to-feed (RTF) basis or stored in a concentrated liquid or a powder. The powder can be prepared for example, by spray drying the infant formula prepared as indicated above, and the formula can be reconstituted for example, by rehydrating the concentrate. Infant nutritional formulas are well known in the art and commercially available (e.g., Similac.RTM. and Alimentum.RTM. from Ross Products Division, Abbott Laboratories).

Examples of nutritional compositions of the present invention include but are not limited to infant formulas, dietary supplements, dietary substitutes, and rehydration compositions, the latter of which may also be considered as pharmaceutical compositions. Nutritional compositions of particular interest include but are not limited to those utilized for enteral and parenteral supplementation for infants, specialist infant formulas, supplements for the elderly, and supplements for those with gastrointestinal difficulties and/or malabsorption. Certainly the young, the elderly, and the immunocompromised are particularly suspectible to suffering serious, and even fatal, effects from the toxins.

The nutritional compositions of the present invention may also be added to food even when supplementation of the diet is not required. For example, the composition may be added to food of any type including but not limited to margarines, modified butters, cheeses, milk, yogurt, chocolate, candy, snacks, salad oils, cooking oils, cooking fats, meats, fish and beverages.

In a preferred embodiment of the present invention, the nutritional composition is an enteral nutritional product, more preferably, an adult or pediatric enteral nutritional product. For example, this composition may be administered to adults or children experiencing gastrointestinal distress or having specialized needs due to chronic or acute disease states. The composition may comprise, produced in accordance with the present invention, macronutrients, vitamins and minerals as described above. The macronutrients may be present in amounts equivalent to those present in human milk or on an energy basis, i.e., on a per calorie basis.

Methods for formulating liquid or solid enteral and parenteral nutritional formulas are well known in the art. The enteral formula, for example, may be sterilized and subsequently utilized on a ready-to-feed (RTF) basis or stored in a concentrated liquid or lyophilized powder form. The powder can be prepared by spray drying the formula prepared as indicated above, and reconstituting it by rehydrating the concentrate. Adult and pediatric nutritional formulas are well known in the art and are commercially available (e.g., Similac(R), Ensure(R), Jevity(R) and Alimentum(R) from Ross Products Division, Abbott Laboratories, Columbus, Ohio).

The energy density of the nutritional compositions of the present invention, when in liquid form, may range from about 0.6 Kcal to about 3 Kcal per ml. When in solid or powdered form, the nutritional supplements may contain from about 1.2 to more than 9 Kcals per gram, preferably about 3 to 7 Kcals per gm. In general, the osmolality of a liquid product should be less than 700 mOsm and, more preferably, less than 660 mOsm.

The nutritional formula may include macronutrients, vitamins, and minerals, as noted above, in addition to the monovalent oligosaccharides of the present invention. The presence of these additional components helps the individual ingest the minimum daily requirements of these elements. In addition, it may also be desirable to add zinc, copper, folic acid and antioxidants to the composition. It is believed that these substance boost a stressed immune system and will therefore provide further benefits to the individual receiving the composition. A pharmaceutical composition, as described above, may also be supplemented with these elements.

In a more preferred embodiment, the nutritional composition comprises, in addition to antioxidants and at least one monovalent oligosaccharide, a source of carbohydrate wherein at least 5 weight percent of the carbohydrate is indigestible oligosaccharide. In a more preferred embodiment, the nutritional composition additionally comprises protein, taurine, and carnitine.

### Diagnostic and analytical uses related to therapeutical uses

Furthermore, it is possible to use the *Escherichia coli* binding oligosaccharide receptors according to the present invention in the diagnosis of a condition caused by an *Escherichia coli* infection. Diagnostic uses also include the use of the *Escherichia coli* binding substance for typing of *Escherichia coli.* The typing of *E. coli* with regard to binding of the carbohydrate receptors according to the present invention can be used to determine effective combination of therapeutic carbohydrates for a specific diarrheagenic *E. coli* strain. This can be useful for making specific lower cost theraphies for local infections, the profiles of carbohydrate bindings of major diarrhea causing *E. coli* may differ in different geographic locations and during epidemies.

### Novel protein bound receptors in human gastrointestinal tract

Present invention shows novel receptors in human gastrointestinal tract. These receptors are present on glycoproteins and are therefore considered as first contact receptors for infecting pathogens. The present invention is directed to the use of the novel protein linked receptors for analysis for binding of pathogens to human gastrointestinal tract. The present invention is directed to the use of the novel protein linked receptors for diagnostics for pathogens of human gastrointestinal tract.

Samples of protein linked carbohydrates from different position on the gastrointestinal epithelia were analysed. The novel protein linked receptors include protein bound lacto-receptors, leolacto-receptor, fucosyl receptors, mannose receptors or sialic acid receptors according to the invention. The novel protein linked receptors can be used for binding assay as released oligosacharides or oligosaccharide derivatives, alternatively the protein linked oligosaccharide sequences can be used as isolated glycoproteins. Corresponding oligosaccharide sequences can be also produced synthetically. In a preferred embodiment at least part of O-glycan or N-glycan core structure of the natural protein linked receptor is included in diagnostic or analysis substances. It is especially preferred to use the sequence to analyse pathogen binding towards the novel protein linked receptor when the pathogen is infecting the part of the gastrointestinal epithelium where the novel protein linked receptor is abundant or especially found.

The novel protein linked receptors can be used for a search or design of analogous oligosaccharide substances. The analogous substances can be therapeutically useful or can be used for diagnostics of diarrhea. It is especially preferred to search or design structures for which there is available effective and economical synthesis.

Structural analysis revealed some preferred protein linked receptor to be used for analysis or diagnostics with regard to human infections. The mannose receptors are N-glycan type oligosaccharides. The present invention is directed to diagnostic and analytic uses of high-mannose or multimannose type N-glycans. The present invention is especially directed to the uses of high-mannose N-glycans comprising phophate esters. The mannose receptors are present in all major parts of human gastrointestinal tract. The neolacto-type protein linked oligosaccharide sequences are in a preferred embodiment N-linked glycans, the neolacto-type receptors are present in all parts of gastrointestinal tract. The lacto-receptor was especially observed on glycoproteins of intestinal tissue. The lacto-receptor is more preferentially present on O-glycan type sequence.
Several fucosylated novel protein bound receptors were found. Lewis a-type sequences were especially found in intestine and larynx. Other novel fucosylated receptors useful for analysis of human pathogen binding includes O-glycans comprising Fucα2Gal-structures, which are present especially on human stomach.
Sialylated novel protein linked receptors includes NeuNAcα3Gal- and NeuNAcα6Gal-structures. NeuNAcα3Gal- is in a preferred embodiment present on a N-linked glycan and NeuNAcα6Gal-structures are preferentially present on both N-linked and O-linked glycans.
The novel protein linked receptors can be also used for search of substances which can inhibit the binding of the pathogen to the novel protein bound receptor. The substance may be an antibody, for example an antibody present in milk, which can bind to carbohydrate receptor binding substance on pathogen. The inhibiting substance may also be a lectin binding to the novel protein linked receptor, the lectin may be for example a food lectin.
In a specific embodiment it is also realized that the novel protein linked receptors can be used as receptors or substrates for probiotic bacteria, which adhere and bind or is able to degradate the structure.

In a specific embodiment it is also realized that the novel protein linked receptors can be used for diagnostic or analytical methods to analyze the bindings of intestinal pathogens to the receptor structures and smaller derivatives or anlogues thereof.

When the substance is used for diagnosis or typing, it may be included in, e.g., a probe or a test stick, optionally constituting a part of a test kit. When this probe or test stick is brought into contact with a sample containing *Escherichia coli,* the bacteria will bind the probe or test stick and can be thus removed from the sample and further analyzed. In a preferred embodiment the test kit comprises at least two oligosaccharide receptors according to the present invention, more preferably the test kit comprises at least three and most preferably at least four oligosaccharide receptors according to the present invention. In a preferred embodiment the test kit comprises seven or all of the oligosaccharide receptors according to the present invention.

The glycolipid structures are naturally presented in a polyvalent form on cellular membranes. This type of representation can be mimicked by the solid phase assay described below or by making liposomes of glycolipids or neoglycolipids.

The present novel neoglycolipids produced by reductive amination of hydrophobic hexadecylaniline were able to provide effective presentation of the oligosaccharides. Most previously known neoglycolipid conjugates used for binding of bacteria have contained negatively charged groups such as phosphor ester of phosphadityl ethanolamine neoglycolipids. Problems of such compounds are negative charge of the substance and natural biological binding involving the phospholipid structure. Negatively charged molecules are known to be involved in numerous non-specific bindings with proteins and other biological substances. Moreover, many of these structures are labile and can be enzymatically or chemically degraded. The present invention is directed to the non-acidic conjugates of oligosaccharide sequences meaning that the oligosaccharide sequences are linked to non-acidic chemical structures. Preferably, the non-acidic conjugates are neutral meaning that the oligosaccharide sequences are linked to neutral, non-charged, chemical structures. The preferred conjugates according to the invention are polyvalent substances.

In the previous art bioactive oligosaccharide sequences are often linked to carrier structures by reducing a part of the receptor active oligosaccharide structure. Hydrophobic spacers containing alkyl chains (-CH₂-)ₙ and/or benzyl rings have been used. However, hydrophobic structures are in general known to be involved in non-specific interactions with proteins and other bioactive molecules.

The neoglycolipid data of the examples below show that under the experimental conditions used in the assay the hexadecylaniline parts of the neoglycolipid compounds do not cause non-specific binding for the studied bacterium. In the neoglycolipids the hexadecylaniline part of the conjugate forms probably a lipid layer like structure and is not available for the binding. The invention shows that reducing a monosaccharide residue belonging to the binding epitope may destroy the binding. It was further realized that a reduced monosaccharide can be used as a hydrophilic spacer to link a receptor epitope and a polyvalent presentation structure. According to the invention it is preferred to link the bioactive oligosaccharide via a hydrophilic spacer to a polyvalent or multivalent carrier molecule to form a polyvalent or oligovalent/multivalent structure. All polyvalent (comprising more than 10 receptor active oligosaccharide residues) and oligovalent/multivalent structures (comprising 2-10 receptor active oligosaccharide residues) are referred here as polyvalent structures, though depending on the application oligovalent/multivalent constructs can be more preferred than larger polyvalent structures. The hydrophilic spacer group comprises preferably at least one hydroxyl group. More preferably the spacer comprises at least two hydroxyl groups and most preferably the spacer comprises at least three hydroxyl groups.

According to the invention it is preferred to use polyvalent conjugates in which the hydrophilic spacer group linking the oligosaccharide sequences to polyvalent presentation structure is preferably a flexible chain comprising one or several -CHOH- groups and/or an amide side chain such as an acetamido NHCOCH₃ or an alkylamido. The hydroxyl groups and/or the acetamido group also protects the spacer from enzymatic hydrolysis in vivo. The term flexible means that the spacer comprises flexible bonds and do not form a ring structure without flexibility. A reduced monosaccharide residues such as ones formed by reductive amination in the present invention are examples of flexible hydrophilic spacers. The flexible hydrophilic spacer is optimal for avoiding non-specific binding of neoglycolipid or polyvalent conjugates. This is essential optimal activity in bioassays and for bioactivity of pharmaceuticals or functional foods, for example.

A general formula for a conjugate with a flexible hydrophilic linker has the following Formula 2:

[OS-O-(X)ₙ-L₁-CH(H/{CH₁₋₂OH}ₚ₁)-{CH₁OH}ₚ₂-{CH(NH-R)}ₚ₃-{CH₁OH}ₚ₄-L₂]ₘ-Z

wherein L₁ and L₂ are linking groups comprising independently oxygen, nitrogen, sulphur or carbon linkage atom or two linking atoms of the group forming linkages such as -0-, - S-, -CH₂-, -NH-, -N(COCH3)-, amide groups -CO-NH- or NH-CO- or -N=N- (hydrazine derivative) or hydroxylamine -0-NH- and NH-O-. L1 is linkage from carbon 1 of the reducing end monosaccharide of X or when n =0, L1 replaces -0- and links directly from the reducing end C 1 of OS.

p1, p2, p3, and p4 are independently integers from 0-7, with the proviso that at least one of p1, p2, p3, and p4 is at least 1. CH₁₋₂OH in the branching term {CH₁₋₂OH}ₚ₁ means that the chain terminating group is CH₂OH and when the p1 is more than 1 there is secondary alcohol groups -CHOH- linking the terminating group to the rest of the spacer. R is preferably acetyl group (-COCH₃) or R is an alternative linkage to Z and then L₂ is one or two atom chain terminating group, in another embodiment R is an analog forming group comprising C₁₋₄ acyl group (preferably hydrophilic such as hydroxy alkyl) comprising amido structure or H or C₁₋₄ alkyl forming an amine. And m > 1 and Z is polyvalent carrier. OS and X are defined in Formula 1.

Preferred polyvalent structures comprising a flexible hydrophilic spacer according to formula 2 include *Escherichia coli* binding oligosaccharide sequence (OS) β1-3 linked to Galβ4Glc(red)-Z, and OSβ6GlcNAc(red)-Z and OSβ6GalNAc(red)-Z., where "(red)" means the amine linkage structure formed by reductive amination from the reducing end monosaccharides and an amine group of the polyvalent carrier Z.

In the present invention the oligosaccharide group is preferably linked in a polyvalent or an oligovalent form to a carrier which is not a protein or peptide to avoid antigenicity and possible allergic reactions, preferably the backbone is a natural non-antigenic polysaccharide.

Therefore the some of optimal polyvalent non-acidic substances to be used according to the invention comprises a terminal oligosaccharide sequence
Gal(NAc)ᵣ₁/Glc(NAc)ᵣ₂β3Galβ4Glc(NAc)ᵤ
wherein r1 r2, and u are each independently 0 or 1,
More preferably u=0 and most preferably the oligosaccharide sequence presented in polyvalent form is
GlcNAcβ3Galβ4GlcNAc
or an analog or derivative thereof.

### Soluble polyvalent conjugates comprising hydroxylamine linkage

Effective production of soluble polyvalent oligosaccharide conjugates, which are biologically acceptable has been a problematic. The problem was solved by using a chemoselective O-hydroxylamine structure to be reacted with the reducing end aldehyde of the oligosaccharide to be coupled. The oxygen of the carrier is linked to the backbone or spacer and the nitrogen is linked to the C-of the reducing end of the oligosaccharide. The reaction can be produced under conditions where the polysaccharide backbone is soluble such as in aqueous buffer.
The present invention is specifically directed to oligosaccharides conjugated to polyvalent oligosaccharide or polysaccharide structures by O-hydroxylamine structue.

The present invention is further directed to diarrheagenic *E. coli* inhibiting substance according to
the formula

[OS-(y)ₚ-(S)_{q}-(Z)ᵣ-]ₙPO (SP1)

wherein PO is an oligomeric or polymeric carrier structure, OS is an oligosaccharide sequence according to the invention, PO is preferably oligosaccharide or polysaccharide structure, n is an integer ≥ 1 indicating the number of oligosaccharide groups covalently attached to the carrier PO, S is a spacer group, p, q and r are each 0 or 1, whereby at least one of p and r is different from 0, y and z are linking groups, at least one of y and z being an O-hydroxylamine residue -0-NH- or -O-N=, with the nitrogen atom being linked to the OS and/or PO structure, respectively, and the other y and z, if present, is a chemoselective ligation group, with the proviso that when n is 1, the carrier structure PO is an oligosaccharide or polysaccharide. The present invention is preferably directed to polyvalent constructs wherein the oligosaccharide is linked from the reducing end to the nitrogen atom of the O-hydroxylamine structure.

### Chemoselective ligation groups

The The chemoselective ligation group y and/or z is a chemical group allowing coupling of the OS- group to a spacer group or a OS-(y)ₚ-(S)_{q}-(z)ᵣ- group to the PO carrier, specifically without using protecting groups or catalytic or activator reagents in the coupling reaction. According to the invention, at least one of these groups y and z is a O-hydroxylamine residue -O-NH- or -O- N=. Examples of other chemoselective ligation groups which may be present include the hydrazino group - N-NH- or -N-NR₁-, the ester group -C(=O)-O-, the keto group -C(=O)-, the amide group -C(=O)-NH-, - O- , - S - , - NH-, - NR₁ -, etc., wherein R, is H or a lower alkyl group, preferably containing up to 6 carbon atoms, etc. A preferred chemoselective ligation group is the ester group -C(=O)-O-formed with a hydroxy group, and the amide group -C(=O)-NH- formed with an amine group on the PO or Bio group, respectively. In a preferred embodiment, y is an O-hydroxylamine residue and z is an ester linkage.

Preferably p, q, and r are 1. If q is 0, then preferably one of p and r is 0.

Preferred polysaccharide or oligosaccharide backbone (PO) structures include glycosaminoglycans such as chondroitin, chondroitin sulphate, dermantan sulphate, poly-N-acetylactosamine or keratan sulphate, hyaluronic acid, heparin, and heparin precursors including N-acetylheparosan and heparan sulphate; chitin, chitosan, starch and starch or glycogen fractions. A preferred backbone structure is a cyclodextrin. Useful starch fractions includes amylose and amylopectin fractions.

The invention is specifically directed to use of water soluble forms of the backbone structures such as very low molecular weight chitosan polysaccharide mixture or chitosan oligomer fraction containing hexamer and lerger chitosan oligosaccharides, called here chitomer.

Preferred spacer structure includes ones described for hydrophilic linker above, aminooxyacetic acid. According to an embodiment of the invention the spacer group, when present, is preferably selected from a straight or branched alkylene group with 1 to 10, preferably 1 to 6 carbon atoms, or a straight or branched alkenylene or alkynylene group with 2 to 10, or 2 to 6 carbon atoms. Preferably such group is a methylene or ethylene group. In the spacer group one or more of the chain members can be replaced by -NH-,-O-, -S-, -S-S-, =N-O-, an amide group -C(O)-NH- or -NH-C(O)-, an ester group -C(O)O-or -O-C(O)-, or -CHR₂, where R₂ is an alkyl or alkoxy group of 1 to 6, preferably 1 to 3 carbon atoms, or -COOH. Preferably a group replacing a chain member is NH-, -O-, an amide or an ester group.

The present invention is in a specific embodiment directed to the use of minimal disaccharide epitopes and partial epitopes described by the invention as soluble polyvalent conjugates according to the invention.

### Zoonotic Helicobacter species

The present invention is also directed to *Helicobacter* species causing gastric infections to human and animal living in close contact with human. The zoonotic species also cause other diseases as described by the invention. The species of bacteria have varying zoonotic potential. The bacteria from animals living in close contact with human includes the large group of enterohepatic Helicobacters from *H. pullorum* to *H. westmaedii* and gastric species from *H. suis* to *H.salomonis,* preferably also including bovine species (*H. bovis*) and monkey species fig. 1 Dewhurst et al. 2000. The species of bacteria form homologous groups known to zoonotically infect human. This grouping does not include *H. mustelae* type "wild animal" species, less interesting as therapy targets. These Helicobacters form homologous groups known to containg zoonotic activities. Moreover the present invention describes the carbohydrate binding activities allowing the bacteria to spread. The species are different from *H. pylori* having Lewis b and/ more pronounced sialic acid based infection mechanisms. The invention is preferably directed to inhibition to the Helicobacters known to cause zoonotic infections. The preferred species includes group of "gastrospirilla" bacteria, zoonotic cat and dog pathogens *H. felis- H. bizzezeronii-* and *H*. *salomonis,* which are same or very similar to a group of human infecting bacteria named in human *H. heilmannii* and another type of *H.heilmannii* resembles closely candidatus *H*. *suis,* a pig Helicobacter. Yet another zoonotic group includes species characterized as *Flexipira rappini* isolated from aborted lambs, dog and human faeces and pig intestineGroup of helicobacters called *H. rappini* has been also known to infect human, with similarity to *H. bilis* and *H. trogantum.* Especially zoonotic species include also *H. canis* and *H. pullorum* (from poultry to human) (On 2001) and *H. fenellilae, H. cinaedi, H. canadiens, H winghamensis* and *H westmaedi* (Fox 2002).

### Zoonotic enteric infections causing diarrhea and other enteric diseases

The present invention invention is also directed to treatment and/or prevention of diarrheas caused by zoonotic *Helicobacter* species. In a preferred embodiment one or several of the carbohydrates are used for acute or preventive treatment of infections in animals living in close contact with humans. The invention is specifically directed to treatments of pet animals infectable with zoonotically spreading *Helicobacter* species. Such infected pets have reported to infect human beings and cause diseases including diarrheas. In a specific embodiment the treatment is given to the human or animal that is suffering of weakened immune protection or immunodeficiency.

### Zoonotic Helicobacter infections causing hepatobiliary disease

The present invention is also directed to the treatment and/or prevention of hepatobiliary infection caused by zoonotic *Helicobacter* species. In a preferred embodiment one or several of the carbohydrates are used for acute or preventive treatments of infections in animals living in close contact with humans. The invention is specifically directed to the treatment of pet animals infectable with zoonotically spreading *Helicobacter* species. Such infected pets have been reported to infect human beings and cause diseases including hepatobiliary diseases. In a specific embodiment the treatment is given to the human or animal that is suffering of weakened immune protection or immunodeficiency.

### Zoonotic Helicobacter infections causing gastric or hepatic disease

The present invention is also directed to the treatment and/or prevention of gastric infections and diseases caused by zoonotic *Helicobacter* species. In a preferred embodiment one or several of the carbohydrates are used for acute or preventive treatments of infections in animals living in close contact with humans. The invention is specifically directed to the treatment of pet animals infectable with zoonotically spreading *Helicobacter* species. Such infected pets have been reported to infect human beings and cause diseases including gastric infections. In a specific embodiment the treatment is given to the human or animal that is suffering of weakened immune protection or immunodeficiency.

### Enterohepatic Helicobacteria

The invention is primarily targeted to common binding specificity shared with enterohepatic *non-H.pylori Helicobacter* species. These species includes *H. canis, H. bilis* and *H. hepaticus.* The similar galactose based binding specificity profile towards human and animal glyconjugates is also observable with *H. fenelliae, H. rappini* and *H.pullorum.* In general the ecologic niches in enterohepatic system allows an effective use of limited amount of receptor carbohydrates. The present invention identifies the major receptor carbohydrates useful for binding enterohepatic system of human and animals. In a specific embodiment the galactose binding specificity is further applicable for inhibition and binding assays with other enterohepatic *Helicobacter* species having the same infectivity profile in enterohepatic system of human and animals.

### Zoonotic Helicobacteria causing gastric infection

The present invention is further directed to treatment of *non-H. pylori Helicobacteria* which are primarily infecting animals including preferably pets, preferably cats and dogs, and which also zoonotically infect human. Examples of zoonotic gastric pathogens includes *H. felis* and *H. heilmannii.* The present invention is not directed to binding specificities of *H. mustellae* included only as control which is not known to infect human or common pet animals such as cats and dogs.

Glycolipid and carbohydrate nomenclature is essentially according to recommendations by the IUPAC-IUB Commission on Biochemical Nomenclature (Carbohydrate Res. 1998, 312, 167; Carbohydrate Res. 1997, 297, 1; Eur. J. Biochem. 1998, 257, 29).

It is assumed that Gal, Glc, GlcNAc, and Neu5Ac are of the D-configuration, Fuc of the L-configuration, and all the monosaccharide units in the pyranose form. Glucosamine is referred as GlcN or GlcNH₂ and galactosamine as GalN or GalNH₂. Glycosidic linkages are shown partly in shorter and partly in longer nomenclature, the linkages of the Neu5Ac-residues α3 and α6 mean the same as α2-3 and α2-6, respectively, and with other monosaccharide residues α1-3, β1-3, β1-4, and β1-6 can be shortened as α3, β3, β4, and β6, respectively. Lactosamine refers to N-acetyllactosamine, Galβ4GlcNAc, and sialic acid is N-acetylneuraminic acid (Neu5Ac) or N-glycolylneuraminic acid (Neu5Gc) or any other natural sialic acid. The sialic acid are referred together as NeuNX, wherein preferably X is Ac or Gc. Ocassionally Neu5Ac/Gc/X may be referred as NeuNAc/NeuNGc/NeuNX. Term glycan means here broadly oligosaccharide or polysaccharide chains present in human or animal glycoconjugates, especially on glycolipids or glycoproteins. In the shorthand nomenclature for fatty acids and bases, the number before the colon refers to the carbon chain lenght and the number after the colon gives the total number of double bonds in the hydrocarbon chain. Abbreviation GSL refers to glycosphingolipid. Abbreviations or short names or symbols of glycosphingolipids are given in the text and Table 2. *Escherichia coli* refers also to the bacteria similar to *Escherichia coli.*

The expression "terminal oligosaccharide sequence" indicates that the oligosaccharide is not substituted to the non-reducing end terminal residue by another monosaccharide residue.

The term "α3/β3" indicates that the adjacent residues in an oligosaccharide sequence can be either α3- or β3- linked to each other.

The publications and other materials used herein to illuminate the background of the invention, and in particular, to provide additional details with respect to its practice, are incorporated herein by reference.

The present invention is further illustrated by the following examples, which in no way are intended to limit the scope of the invention:

### EXAMPLES

### EXAMPLE I

### Experimental procedures

*Culture Conditions and Labeling -* The *E. coli* strains were cultured on Luria-agar with the addition of 10 µl ³⁵S-methionine (400 µCi; Amersham Pharmacia Biotech, U.K.) at 37 °C for 12 h. The bacteria were harvested by scraping, washed three times with phosphate-buffered saline (PBS), pH 7.3, and thereafter resuspended in PBS (with or without 1% mannose (w/v)) to 1x10⁸ CFU/ml. The specific activities of the suspensions were approximately 1 cpm per 100 bacteria.

*Reference Glycosphingolipids* - Total acid and non-acid glycosphingolipid fractions were obtained by standard procedures (1). The individual glycosphingolipids were isolated by repeated chromatography on silicic acid columns of the native glycosphingolipid fractions, or acetylated derivatives thereof. The identity of the purified glycosphingolipids was confirmed by mass spectrometry (2), proton NMR spectroscopy (3), and degradation studies (4, 5).

*Preparation of neoglycolipids.* Oligosaccharides with terminal GlcNAc were synthethic oligosaccharides GlcNAcβ3Galβ4GlcNAc, GlcNAcβ3Galβ4GlcNAcβ3Galβ4Glc and GlcNAcβ3Galβ4GlcNAcβ6GlcNAc from Carbion Oy, Finland, and mannose oligosaccharide was from Dextralabs, UK were reductively aminated with 4-hexadecylaniline (abbreviation HDA, from Aldrich, Stockholm, Sweden) by cyanoborohydride (Halina Miller-Podraza, to be published later). The products were characterized by mass spectrometry and were confirmed to be reductively aminated conjugated of the oligosacharides and HDA.

*Thin-Layer Chromatography -* Thin-layer chromatography of glycosphingolipids was performed on glass- or aluminum-backed silica gel 60 HPTLC plates (Merck, Darmstadt, Germany), using chloroform/methanol/water 60:35:8 (by volume) as solvent system. Chemical detection was done with anisaldehyde (6).

*Glycosphingolipid Binding Assays* - Binding of ³⁵S-labeled bacteria to glycosphingolipids on thin-layer chromatograms was done as reported (7). Dried chromatograms were dipped for 1 min in diethylether/*n-*hexane (1:5, by volume) containing 0.5% (w/v) polyisobutylmethacrylate (Aldrich Chem. Comp. Inc., Milwaukee, WI). After drying, the chromatograms were soaked in PBS containing 2% bovine serum albumin (w/v), 0. 1 % NaN₃ (w/v) and 0.1% Tween 20 (by volume) for 2 h at room temperature. The chromatograms were subsequentely covered with radiolabeled bacteria diluted in PBS (2-5 x 10⁶ cpm/ml). Incubation was done for 2 h at room temperature, followed by repeated washings with PBS. The chromatograms were thereafter autoradiographed using XAR-5 X-ray films (Eastman Kodak, Rochester, NY) for 12 h. Autoradiograms were replicated using a CCD camera (Dage-MTI, Inc., Michigan City, In) , and analysis of the images was performed using the public domain NIH Image program (developed at the U.S. National Institutes of Health, and available at http://rsb.info.nih.gov/nih-image/).

*Inhibition with Soluble Oligosaccharides* - As a test for possible inhibition of binding by soluble sugars ³⁵S-labeled *E. coli* strains were incubated for 1 h at room temperature with approximately 1.5 mM of globotriaose, globotetraose, lacto-N-tetraose, lacto-N-neotetraose, 3'-sialyllactose and 6'-sialyllactose in PBS. The final concentrations of the inhibiting oligosaccharides on the TLC-plate were 0.3 mM. Lactose was tested at final concentrations from 1 mg/ml and 2 mg/ml. Thereafter the chromatogram binding assay was performed as described above.

*Analysis of glycosylation from human gastrointestinal system* - Human mucosa samples were obtained from surgical operations. They represented epithelial tissues of the larynx and the gastrointestinal tract, namely stomach and colon.

Reducing oligosaccharides were isolated by non-reductive β-elimination. After purification, they represented all kinds of cellular glycans mainly from proteins.

MALDI-TOF MS was performed with a Voyager-DE STR BioSpectrometry Workstation, essentially as in (Saarinen et al., 1999; Papac et al., 1996; Harvey, 1993). Neutral oligosaccharides were analysed with 2,5-dihydrobenzoic acid matrix in positive ion reflector mode, and acidic oligosaccharides were analysed with 2',4',6'-trihydroxyacetophenone matrix in negative ion linear mode.

All exoglycosidase reactions were performed essentially as described previously (Nyman et al., 1996; Saarinen et al., 1999) and analysed by MALDI-TOF MS. The enzymes and their specific control reactions with characterised oligosaccharides were: β-N-acetylglucosaminidase (*Streptococcus pneumoniae,* recombinant, *E. coli;* Calbiochem, USA) digested GlcNAcβ1-6Gal-R in β1,4-galactosidase treated lacto-N-hexaose but not GalNAcβ1-4GlcNAcβ1-3/6Gal-R in a synthetic oligosaccharide; *Arthrobacter ureafaciens* sialidase (recombinant, *E. coli;* Glyko, UK) digested both Neu5Acα2-3Galβ1-4GlcNAc-R and NeuSAcα2-6Galβ1-4GlcNAc-R in synthetic oligosaccharides; α2,3-sialidase (*Streptococcus pneumoniae,* recombinant, *E. coli;* Glyko, UK) digested Neu5Acα2-3Ga1β1-4GlcNAc-R but not Neu5Acα2-6Galβ1-4GlcNAc-R in synthetic oligosaccharides; α1,2-fucosidase *(Xanthomonas manihotis;* Glyko, UK) digested Fucαl-2Galβ1-3GlcNAc-R in monofucosyllacto-N-hexaose I but not Galβ1-4(Fucα1-3)GlcNAc in lacto-N-fucopentaose III; α1,3/4-fucosidase (*Xanthomonas* sp.; Calbiochem, USA) digested Galβ1-4(Fucα1-3)GlcNAc-R in lacto-N-fucopentaose III but not Fucαl-2Galβ1-3GlcNAc-R in monofucosyllacto-N-hexaose I; β1,4-galactosidase *(Streptococcus pneumoniae,* recombinant, *E. coli;* Calbiochem, USA) digested Galβ1-4GlcNAc-R but not Galβ1-3GlcNAc-R in lacto-N-hexaose; β1,3-galactosidase (recombinant, *E. coli;* Calbiochem, USA) digested Galβ1-3GlcNAc-R but not Galβ1-4GlcNAc-R in lacto-N-hexaose; α-mannosidase (Jack bean; Glyko, UK) transformed a mixture of high-mannose N-glycans to the Man₁GlcNAC₂ N-glycan core trisaccharide. Control digestions were performed in parallel and analysed similarly to the analytical exoglycosidase reactions.

### Results

### Screening for Carbohydrate Binding Activity of Diarrheagenic E. coli

*Using Mixtures of Glycosphingolipids -* During the initial studies the potential carbohydrate recognition of a number of diarrheagenic *E. coli* strains (summarized in Table 1) was evaluated using well characterized mixtures of glycosphingolipids in the chromatogram binding assay, in order to expose the bacteria to a large number of variant carbohydrate sequences. Thereby, a selective binding to some glycosphingolipids was detected, while other compounds were not recognized by the bacteria. The binding patterns obtained varied between the strains as exemplified in Fig. 1.

*Binding of CCUG Type Strains to Pure Glycosphingolipids* - To further define the binding characteristics, two type strains (CCUG 38068 and 38077) were selected for binding assays using pure glycosphingolipids on thin-layer chromatograms, as exemplified in Fig. 2. The results are summarized in Table 2. Thus, both strains bound to lactosylceramide. The binding to lactosylceramide was only obtained when this glycosphingolipid had a ceramide with sphingosine or phytosphingosine and hydroxy fatty acids (No.S in Table 2), whereas lactosylceramide with sphingosine and non-hydroxy fatty acids (No. 4) was consistently non-binding.

Further glycosphingolipids recognized by both bacteria were galabiaosylceramide (No. 6), isoglobotriaosylceramide (No. 7), globotriaosylceramide (No. 8), gangliotriaosylceramide (No. 10), gangliotetraosylceramide (No. 11), globotetraosylceramide (No. 12), Forssman glycosphingolipid (No. 14), neolactotetraosylceramide (No. 15), lactotetraosylceramide (No. 23), neolactohexaosylceramide (No. 22) and NeuGcα3-neolactohexaosylceramide (No. 36). The binding to these glycosphingolipids was not dependent on ceramide structure.

The strain CCUG 38077, but not strain CCUG 38068, also bound to a number of gangliosides (Nos. 28, 29, 31-36; exemplified in Fig. 2). Binding-active gangliosides had both *N*-acetyl- and *N*-glycolyl neuraminic acid, and the neuraminic acid could be both α3-linked and α6-linked. However, all gangliosides were not recognized, e.g. no binding to the GD1a ganglioside (No. 30) was obtained.

The strain CCUG 38068 on the other hand bound to the Le^{a}-5 glycosphingolipid (No. 24), which was not recognized by strain CCUG 38077.

The two strains of *E. coli* were also shown to bind to Manα3(Manα6)Man on thin-layer chromatograms. The saccharide was tested after coupling with a lipid tail through reductive amination. Further experiments with double branched mannose-containing neoglycolipids indicated that the binding was dependent on the presentation of the saccharide.

Neoglycolipids with terminal GlcNAcβ3LacNAc were also recognized by the two CCUG strains.

### Example of neoglycolipid binding experiment

The isomeric pentasaccharides were produced by specific β3-galactosidase (Calbiochem, La Jolla, CA) and β4-galactosidase (from *D.pneumonia,* Sigma, ST Louis, MO) digestions digestion from commercial hexasaccharides para-lacto-N-hexaose (from Dextra laboratories, Reding, UK) and from Lacto-N-hexaose (Isosep, Tullinge, Sweden) to obtain GlcNAcβ3Galβ4GlcNAcβ3Galβ4Glc and Galβ3GlcNAcβ3(GlcNAcβ6)Galβ4Glc. GlcNAcβ3Galβ4GlcNAcβ6Galβ4Glc was produced from GlcNAcβ6Galβ4Glc (Sigma St Louis, MO) by first β4-galactosyltransferase (Calbiochem, La Jolla, CA) reaction using UDP-Gal (Kyowa Hakko, Japan) as donor and then by β3-GlcNAc-transferase (human serum) and UDP-GlcNAc (Kyowa Hakko, Japan). The oligosaccharides were purified using gel filtration chromatography and characterized by NMR-spectroscopy and mass spectrometry. The pentasaccharides and Manα3(Manα6)Man were reductively aminated with 4-hexadecylaniline (abbreviation HDA, from Aldrich, Stockholm, Sweden) by cyanoborohydride (Halina Miller-Podraza, to be published later). The products were characterized by mass spectrometry were confirmed to be the corresponding GlcNAcβ3Galβ4GlcNAcβ3Galβ4Glc(red)-HDA, Galβ3GlcNAcβ3(GlcNAcβ6)Galβ4Glc (red)-HDA, GlcNAcβ3Galβ4GlcNAcβ6Galβ4Glc(red)-HDA, and Manα3(Manα6)Man(red)-HDA [where "(red)-" means the amine linkage structure formed by reductive amination from the reducing end glucoses of the saccharides and amine group of the hexadecylaniline (HDA)]. The compound GlcNAcβ3Galβ4GlcNAcβ3Galβ4Glc(red)-HDA and Manα3(Manα6)Man(red)-HDA had binding activity with regard to two strains of diarrheagenic *E.coli* (CCUG 38068 and 38077) in TLC overlay assay described above while the pentasaccharides Galβ3GlcNAcβ3(GlcNAcβ6)Galβ4Glc(red)-HDA, GlcNAcβ3Galβ4GlcNAcβ6Galβ4Glc(red)-HDA had much weaker or non-existent binding activities. The results indicated that GlcNAcβ3Galβ4GlcNAcβ3Galβ4Glc-type neolactostructures bind effectively to diarrheagenic *E. coli.* The binding is reduced much if the GlcNAcβ3 is changed to GlcNAcβ6- in the structure. The results also demonstrated similarily that blocking the middle galactose in lacto tetraose structure by a GIcNAc branch Galβ3GlcNAcβ3(GlcNAcβ6)Galβ4Glc diminished the binding on a TLC-overlay assay.

### Binding specificities in different types of diarrheagenic E. coli

Screening for binding specificities in different types of diarrheagenic E. coli, i.e. enterotoxigenic (ETEC), enteropathogenic (EPEC), enteroaggregative (EAGG), enteroinvasive (EIEC) and enterohemorrhagic (EHEC) showed that the majority of the described binding specificities were found in most strains (data about typical/type strains are summarized in Table 4. Thus binding to lactosylceramide, lacto- and neolacto was obtained with all strains tested, and globobinding was obtained by all strains with the exception of wild type EHEC strains. In this collection mainly the type strains, sialic acid binding was obtained with one enteroaggregative strain. However, several wild type diarrheagenic E. coli strains bind to sialic acid (see e.g. Fig 6 and Fig 3). The absence of binding of EHEC to globoseries glycosphingolipids and to gangliosides is illustrated in Fig. 7(lane 1, and lanes 3,5,6,8, and 9 respectively. The binding obtained in lane 7 is related to lactosylceramide binding.

### Preferential recognition of NeuGc-neolactotetraosylceramide

To dissect the sialic acid binding preferences of diarrheagenic *E. coli,* the binding of bacteria to variants of sialyl-neoleactotetraosylceramide was compared. The bacteria bound with highest affinity to NeuGcα3-neolactotetraosylceramide (NeuGcα3Galβ4GlcNAcβ3Galβ4GlcNAcβ3Galβ4GlcβCer), followed by NeuAcα6-neolactotetraosylceramide (NeuAcα6Galβ4GlcNAcβ3Galβ4GlcNAcβ3Galβ4GlcβCer), and finally NeuAcα3-neolactotetraosylceramide (NeuAcα3Galβ4GlcNAcβ3Galβ4GlcNAcβ3Galβ4GlcβCer). The comparison was performed by using dilution series of glycolipids and comparing the disappearence of the bindings when the amounts of glycolipids were reduced. Taken together the results indicate that even higher binding can be obtained with α6-linked NeuGc.

### Based on binding patterns and carbohydrate structures the binding-activities were classified into eight separate binding specificities:

a) Lactosylceramide-binding: represented by lactosylceramide (No. 5) and isoglobotriaosylceramide (No. 7).
b) Ganglio-binding: represented by gangliotriaosylceramide (No. 10) and gangliotetraosylceramide (No. 11).
c) Galα4Gal-binding: represented by galabiaosylceramide (No. 6), globotriaosylceramide (No. 8), globotetraosylceramide (No. 12) and the Forssman glycosphingolipid (No. 14).
d) Lacto-binding; represented by lactotetraosylceramide (No. 23).
e) Neolacto-binding: represented by neolactotetraosylceramide (No. 15), neolactohexaosylceramide (No. 22) and NeuGcα3-neolactohexaosylceramide (No. 36).
f) Binding to fucosylated glycosphingolipids: represented by the Le^{a}-5 glycosphingolipid (No. 24).
g) NeuAc/NeuGc-X: represented by the gangliosides Nos. 28,29,31-36.
h) Mannose-X: represented by the Manα3(Manα6)Man-neoglycolipid.

Each wild type strain (Table 1) exhibited two or more of the binding specificities listed above, and the combination of binding specificities varied among the strains. Most strains had even three or more binding specificities. Four and more binding specificities were observed often and most strains may probably have capacity to express all or almost all of the specificities, though the specificities may necessarily not be active all the time. The need of use two or more oligosaccharide sequences at the same time is emphasized by the fact that the expression of the bindings varies between the strains.

### Inhibition Experiments -

### Inhibition by a mixture of globotetraose and 3'sialylactose

The ability of soluble oligosaccharides to interfere with the binding of diarrheagenic *E. coli* to glycosphingolipids on thin-layer plates was examined by incubating the bacteria with a mixture of globotetraose and 3'sialyllactose before binding on chromatograms. The results are shown in Fig. 3. Thus, by incubation of the bacteria with a mixture of oligosaccharides an inhibition of the binding to both globotetraosylceramide and 3'sialyl-paragloboside was obtained. Inhibition of binding to globotriaosylceramide was also obtained by preincubatiing the bacteria with 1.5 mM globotriaose, with final concentration of 0.3 mM.

### Inhibition by a mixture of globotriose, lacto-N-neotetraose and 6'sialylactose

The ability of soluble oligosaccharides to interfere with the binding of diarrheagenic *E. coli* to glycosphingolipids on thin-layer plates was further examined by incubating the bacteria with a mixture of globotetraose and 3'sialyllactose before binding on chromatograms. The results are shown in Fig. 6. Thus, by incubation of the bacteria with a mixture of oligosaccharides a simultaneous inhibition of the binding to globotriasylceramide, NeuNGcα3neolactohexaosylceramide (carrying a midchain neolactoepitope) and NeuGcα3neolactotetraosylceramide was obtained. The data showed simultaneous inhibitions against two epitopes present on the same molecule. A further observation was that binding to NeuGcα3neolactotetraosylceramide is inhibited by 6'sialyllactose, demonstrating that the α3-linked and α6-linked sialic acid is bound by the same bacterial adhesin.

*Other results from the inhibition studies.* In control experiments the monovalent free oligosaccharides described above specifically inhibited the binding to the glycolipids comtaining the same receptor structure but not to other glycolipids. The globoserias oligosaccharides inhibited binding to globo-receptor, Lacto-N-neotetraose inhibited binding to neolacto glycolipid and the sialylalctoses inhibited binding to the sialyl-receptor glycolipids as described above at 0.3 mM final concentration, but no cross-reactivity was observed. Lacto-N-tetraose was also able to inhibit the binding of diarrheagenic *E. coli* to lactotetraosylceramide when lowest densities of glycolipids were used in inhibition experiments as described above. As a control lactose was tested with final concentrations to 1-2 mg/ml (3-6 mM) with no inhibiting activity. Interestingly the disaccharide terminal ganglioside epitope Galβ3GalNAc was also not active as monovalent inhibitor at 0.3 mM concentration against the Ganlio-receptor glycolipid, indicating the the disaccharide sequence may be more useful when conjugated to a polyvalent carrier than a monovalent inhibitor.

The inhibition experiments show that the frequent binding specificities according to the invention are
1. separate
2. inhibitable by relatively low concentrations of monovalent oligosaccharides and
3. inhibitable simultaneously with no harmful effects due to presence of several oligosaccharides,

### Selective switch-off of binding specificities

During these studies we have observed that upon prolonged sub-culture of diarrheagenic *E. coli* one or several of the binding specificities may be selectively lost. This is exemplified in Fig. 8 demonstrating a loss of binding to isoglobotriaosylceramide (lane 4), while the binding to gangliotetrasoylceramide (lane 5) and globotetraosylceramide (lane 6) remain suggesting a down-regulation of the isoglobotetraosylceramide/lactosylceramide binding adhesin. The isoglobosylceramide contains activating a hydroxylfatty acid, giving it activity of the lactosylceramide family. However, no specific pattern in this switch-off could be discerned, *i.e.* different binding specificities were lost at different times. The inventors noticed quite random loss of any of the binding specificities. As the any of the binding specificities may be lost the use of at least two binding monovalent or polyvalent inhibitors according to the inventio is preferred.

### The frequent binding specificities

Numerous TCL-overalys were run with the large collection of different types of diarrhea causing *E. coli* strains. Four binding specificities were found out to be especially frequently occurring amont the bacteria: binding to Globo-receptor, Sialic acid-receptors, Neolacto-receptors, and Lacto-receptors. The Globo- receptor binding was especially stabile against the spontaneous "switch-off'.

*Analysis of protein glycosylation from human gastrointestinal system -* The occurrence of some terminal glycan epitopes in the samples is exemplified below. In all these analyses, the detection level is of the order of 5 % of the relative abundances of the most abundant components.

*Gal*β*1-4GlcNAc*β*-R.* Terminal type II N-acetyllactosaminyl groups, as evidenced by susceptibility to *Streptococcus pneumoniae* β1,4-galactosidase digestion, were detected in all the analysed tissues, namely larynx, stomach, and colon. For example, a peak at m/z 1809.73 in the positive ion reflector mode mass spectrum of the colon sample, corresponding to the ion structure [Hex₅HexNAc₄Fuc+Na]⁺, calc. m/z = 1809.64, was eliminated by β1,4-galactosidase treatment and transformed into m/z 1485.68, corresponding to the ion structure [Hex₃HexNAc₄Fuc+Na]⁺, calc. m/z = 1485.53.

*G*α*l*β*1, 3-R.* Terminal β1,3-galactosidase susceptible galactose residues were detected only in colon epithelium, but not in larynx or stomach epithelium. A clear increase in the intensity of a peak at m/z 933.37 in the positive ion reflector mode mass spectrum, corresponding to the ion structure [Hex₃HexNAc₂+Na]⁺, calc. m/z = 933.32, was generated in a β1,4-galactosidase pretreated sample by the action of β1,3-galactosidase. Also, the intensity of a peak at m/z 1996.84, corresponding to the ion structure [Hex₄HexNAc₅Fuc₂+Na]⁺, calc. m/z = 1996.72, was clearly increased in a β1,4-galactosidase pretreated sample by the action of β1,3-galactosidase. This indicates that there are β1,3-galactosylated derivatives of these structures.

*Fuc*α*1,2-R.* Possible terminal α1,2-fucosyl residues were detected in the stomach epithelium sample, but not in larynx or colon epithelium. Upon α1,2-fucosidase digestion of the stomach sample, in the positive ion reflector mode mass spectrum there was increases in the intensities of peaks at m/z 755.24, corresponding to the ion structure [HexHexNAc₂Fuc+Na]⁺ (calc. m/z 755.27), and m/z 917.29, corresponding to the ion structure [HeX₂HexNAC₂Fuc+Na]⁺ (calc. m/z 917.32), suggesting the presence of fucosylated derivatives of these structures.

*Fuc*α*1,3-R and Fuc*α*1,4-R.* Possible terminal Lewis^{a} or Lewis^{x} blood group determinants were detected in the larynx and colon epithelium, but not in the stomach sample. For example, a clear increase in the intensity of a peak at m/z 2012.81 in the positive ion reflector mode mass spectrum of the colon sample, corresponding to the ion structure [Hex₅HexNAc₅Fuc+Na]⁺, calc. m/z = 2012.72, was generated in a α1,2-fucosidase pretreated sample by the action of α1,3/4-fucosidase, showing the presence of fucosylated derivatives of this structure.

*M*α*n*α*-R.* Terminal α-mannosyl residues were detected in all samples, as α-mannosidase digestion affected a varying series of peaks in the positive ion reflector mode mass spectra, namely at calculated m/z 771.26 [Hex₂HexNAc₂+Na]⁺, m/z 933.32 [Hex₃HexNAc₂+Na]⁺, m/z 1095.37 [Hex₄HexNAc₂+Na]⁺, m/z 1257.42 [Hex₅HexNAc₂+Na]⁺, m/z 1419.48 [Hex₆HexNAc₂+Na]⁺, m/z 1581.53 [Hex₇HexNAC₂+Na]⁺, m/z 1743.58 [Hex₈HexNAc₂+Na]⁺, and m/z 1905.63 [Hex₉HexNAc₂+Na]⁺. After α-mannosidase digestion, these signals were converted to a single peak at calculated m/z 609.21 [Hex₁HexNAc₂+Na]⁺, indicating that the digested structures were high-mannose N-glycans.

*NeuAc*α*2,3-R.* Terminal sialic acids with α2,3-linkages to Gal (Toivonen et al., 2002) were detected in the samples. For example, upon α2,3-sialidase digestion of stomach glycans, a decrease was observed in the relative intensity of a peak at m/z 2369.4, corresponding to the ion structure [NeuAc₂Hex₅HexNAc₄Fuc-H]⁻, calc. m/z = 2369.1.

*NeuAc*α*2,6*/*8*/*9-R.* Terminal sialic acids with linkages other than α2,3 to Gal, or sialic acids α2,3-linked to GalNAc (Toivonen et al., 2002), were detected in the samples. For example, *Arthrobacter ureafaciens* sialidase digestion of α2,3-sialidase treated stomach glycans completely digested peaks at m/z 1931.6 [NeuAc₁Hex₅HexNAC₄-H]⁻ (calc. m/z = 1931.7), m/z 2077.9 [NeuAc₁Hex₅HexNAc₄Fuc-H]⁻(calc. m/z = 2077.9), m/z 2223.3 [NeuAc₂Hex₅HexNAc₄-H]⁻ (calc. m/z = 2223.0), m/z 2369.4 [NeuAc₂Hex₅HexNAc₄Fuc-H]⁻ (calc. m/z = 2369.1), m/z 2735.1 [NeuAc₂Hex₆HexNAc₅Fuc-H]⁻(calc. m/z = 2734.5), and m/z 3026.5 [NeuAc₃Hex₆HexNAc₅Fuc-H]⁻(calc. m/z = 3025.7). Due to their large size and typical monosaccharide composition, these glycans would most likely be N-glycans, but potentially also O-glycans. However, smaller glycans that were also affected by *A. ureafaciens* sialidase, namely at m/z 1038.7 [NeuAcHex₂HexNAc₂-H]⁻ (calc. m/z = 1038.9), and m/z 1185.4 [NeuAcHex₂HexNAc₂Fuc-H]⁻ (calc. m/z = 1185.1), would most likely be O-glycans. It must be noted that in all structures in this paragraph with a single sialic acid residue, the linkage must be α2,6 (or α2,3 to GalNAc).

### EXAMPLE II

Gastric species examined in the present study included, *Helicobacter mustelae* ferret isolates from the National Collection of Type Cultures (NCTC) and the Culture Collection of the University of Gothenberg (CCUG), NCTC 12198/CCUG 25175 (equivalent strains from different sources tested), CCUG 23950 and CCUG 23951, *Helicobacter felis* CCUG 28539 from a cat, in addition, *H. pylori* strains CCUG 17874, CCUG 17875 and a clinical isolate 119/95 were used. Enterohepatic helicobacters of animal origin were purchased from the CCUG including, *Helicobacter canis* CCUG 33835, *Helicobacter bilis* CCUG 38995, *Helicobacter hepaticus* CCUG 33637, and *Helicobacter fennelliae* (CCUG 18820).

### Glycolipid binding assays

### Binding of Helicobacter spp. to glycosphingolipids, both acid and non-acid fractions.

Glycosphingolipids were isolated by standard procedures (Karlsson, 1987). The identity of the purified glycosphingolipids was confirmed by mass spectrometry (Samuelsson *et al.,* 1990), proton NMR spectroscopy (Koerner *et al.,* 1983) and degradation studies (Stellner *et al.,* 1973; Yang and Hakomori, 1971).

Mixtures of glycosphingolipids (40 µg/lane) or pure compounds (2 µg/lane) were subsequently separated using thin-layer chromatography (TLC) on glass- or aluminum-backed silica gel 60 HPTLC plates (Merck, Darmstadt, Germany), with chloroform/methanol/water (60:35:8, by volume) as solvent system. Chemical detection was accomplished by anisaldehyde (Waldi, 1962). *Helicobacter* spp. were subjected to ³⁵S-labeling (Ångström *et al.,* 1998) and suspended in PBS (10⁸ CFU/ml) with a specific activity of approximately 1 cpm per 100 organisms. Binding of the labeled-bacteria to glycosphingolipids separated by TLC was achieved using a bacterial-overlay technique coupled with autoradiography detection using XAR-5 x-ray films (Eastman Kodak, Rochester, NY) as previously described (Hansson *et al.,* 1985).

### The carbohydrate binding specificities of zHelicobacter species

It has been established previously that both *H. pylori* and *H. mustelae* bind gangliotetraosylceramide binding was demonstrated for *H. felis, H. canis* and *H. hepaticus* and *H. bilis* (Table 3). Furthermore, in common with *H. pylori* we found that both gastric and enterohepatic *Helicobacter* spp. tested were capable of binding to lactotetraosylceramide, lactosylceramide with phytosphingosine and/or hydroxy fatty acids and isoglobotriaosylceramide. In contrast, binding to Le^{b} glycosphingolipid was only observed for *H. pylori* CCUG 17875 (Table 3).

The binding of certain *H. pylori* strains to slow-migrating gangliosides in the acid glycosphingolipid fraction of human granulocytes is sialic acid-dependent (Miller-Podraza *et al.,* 1999), and this fraction was therefore used as an indicator of sialic acid-recognition. Binding to this fraction was noted for *H. hepaticus* CCUG 33637 (exemplified in Fig. 4B. lane 1) and *H. pylori* CCUG 17874 and occasionally for *H. mustelae* CCUG 25715 (Table 3). Sialic acid binding capacity assayed by other substances is also present at least in species of *H. bilis.*

The ability of predominantly gastric and enterohepatic species of *Helicobacter* to glycosphingolipids is indicative of the use of host-carbohydrate binding by these species in their adhesion strategies.

The binding specificities of different helicobacteria may be indicative of the ability to colonize a specific niche with different receptors being expressed in the intestine and hepatobillary tree. In addition, different strategies may be useful at different times during infection due to changes in antigen expression by inflamed tissue (Mahdavi *et al.* 2002). From the present study it is apparent that strains of hepatobillary helicobacters namely *H*. *hepaticus* and *H. bilis* share common adhesion strategies with *H. pylori.* These types of hepatobiliary pathogens have ability to bind various glycoconjugates and even some sialylated structures.

### EXAMPLE III

Production of soluble polyvalent conjugates of the oligosaccharide sequences according to the invention

### Amidation of chitosan oligosaccharides

For the preparation of aminooxy functionalized chitosan, the 19-mer chitosan prepared as above was amidated with BOC-aminooxyacetic acid. A sample the chitosan was dissolved in 75% aqueous pyridine, and 5-fold molar excess (per chitosan amino groups) of BOC-aminooxyacetic acid, HBTU and diisopropylethylamine were added. The reaction was allowed to proceed for 42 h at room temperature in the dark, and then dried by rotary evaporation. Small molecular weight reagents were removed by dialysis, and the chitosan was subjected to proton NMR analysis. The analysis shows that on average 4.5 BOC-aminooxyacetyl groups were present on the chitosan chain.

### Conjugation of biorecognition carbohydrates with the aminooxy-chitomer

Removal of the protecting groups by incubation with trifluoroacetic acid (TFA). The Boc-O-hydroxylamine modified chitomers were solubilized in TFA and incubated at room temperature for about 10 minutes. The TFA was removed by evaporation in vacuum. Various aldehyde containing molecules were reacted with the O-hydroxylamine terminals in 0.2 M sodium acetate buffer, pH 4.0, for 42 h at 37 °C. The reaction products were purified by dialysing against water of by gel filtration chromatography.
The reaction product between O-hydroxylamine chitomer and lactose was characterized by NMR-spectroscopy. The NMR spectrum showed presence of both β-anomeric glycosidic structure, Glc H1 signal at 4.136 ppm, and an oxime form with double bond with Glc H1 and H2 signals at 7.690 ppm and 4.626 ppm, respectively (Fig 3, A and B, respectively). The signals at 4.560 ppm and 4.512 ppm were assigned to H1, and signal at 3.054 ppm to H2 protons of backbone GlcN. The signal at 4.479 ppm corresponds to H1 signal of Gal of lactose residue. The signal at 4.163 ppm and signal at 4.449 ppm correspond to CH₂-protons of the ring closed glycosidic form and the double bonded form, respectively, in the spacer formed from aminooxyacetic acid. Almost identical data is obtained when Lacto-N-neotetraose is coupled to the polymeric carrier, additionally signals for the terminal N-acetyllactosamine could be analysed.

**Table 1. Bacterial Strains of Diarrheagenic E. coli tested for binding to glycolipids separated on TLC plates.**

| |
|---|
| Two of the type strains, CCUG 38068 and CCUG 38077, were analysed in more detail against a long list of natural glycolipids, see separate Table. The various other strains tested carry similar binding specificities as for the two type strains of this Table but with a variation in binding patterns for individual strains, similar to the variation between the two type strains tested in detail. |
| CCUG 17649 ETEC |
| CCUG 17650 ETEC |
| CCUG 38068 EPEC |
| CCUG 38077 EAEC |
| CCUG 38083 EAEC |
| CCUG 38092 EIEC |
| CCUG 38094 EIEC |
| |
| 12 EAEC strains |
| 9 EHEC strains |
| |
| 14 diarrheagenic *E. coli* clinical isolates |

The abbreviations are from Nataro and Kaper, Clin. Microbiol. Rev.11(1998) 142, and mean: ETEC enterotoxigenic, EPEC enteropathogenic, EAEC enteroaggregative, EIEC enteroinvasive, EHEC enterohemorrhagic *E. coli*

**TABLE 2. Binding of diarrheagenic Escherichia coli to glycosphingolipids on thin-layer chromatograms**

| No. Trivial name | Structure | CCUG 38068 | CCUG 38077 | Source |
|---|---|---|---|---|
| *Simple compounds* | | | | |
| 1. Cerebroside d18:1-16:0-24:0^{a} | Galβ1Cer | -^{b} | - | Pig kidney |
| 2. Cerebroside d18:1-16:0-24:0 | Glcβ1Cer | - | - | Pig kidney |
| 3. Sulfatide | SO₃-Galβ1Cer | - | - | Human meconium |
| 4. LacCer d18:1-16:0 and 24:1 | Galβ4Glcβ1Cer | - | - | Human granulocytes |
| 5. LacCer t18:0-h16:0-h24:0 | Galβ4Glcβ1Cer | + | + | Rabbit small intestine |
| 6. Galabiosyl | Galα4GalCer | + | + | c |
| 7. Isoglobotri | Galα3Galβ4Glcβ1Cer | + | + | Dog intestine |
| 8. Globotri | Galα4Galβ4Glcβ1Cer | + | + | Human erythrocytes |
| 9. Lactotri | GlcNAcβ3Galβ4Glcβ1Cer | - | - | Human granulocytes^{d} |
| | | | | |

| *Ganglioseries* | | | | |
|---|---|---|---|---|
| 10. Gangliotri | GalNAcβ4Galβ4Glcβ1Cer | + | + | Guinea pig erythrocytes |
| 11: Gangliotetra | Galβ3GalNAcβ4Galβ4Glcβ1Cer | + | + | Mouse feces |
| | | | | |

| *Globoseries* | | | | |
|---|---|---|---|---|
| 12. Globotetra | GalNAcβ3Galα4Galβ4Glcβ1Cer | + | + | Human erythrocytes |
| 13. Isoglobotetra | GalNAcβ3Galα3Galβ4Glcβ1Cer | - | - | Rat colon carcinoma |
| 14. Forssman | GalNAcα3GalNAcβ3Galα4Galβ4Glcβ1Cer | + | + | Dog intestine |
| | | | | |

| *Neolactoseries* | | | | |
|---|---|---|---|---|
| 15. Neolactotetra | Galβ4GlcNAcβ3Galβ4Glcβ1Cer | + | + | Human granulocytes |
| 16. H5-2 | Fucα2Galβ4GlcNAcβ3Galβ4Glcβ1Cer | - | - | Human erythrocytes |
| 17. B5 | GalαGalβ4GlcNAcβ3Galβ4Glcβ1Cer | - | - | Rabbit erythrocytes |
| 18. B6-2 | Galα3(Fucα2)Galβ4GlcNAcβ3Galβ4Glcβ1Cer | - | - | Human erythrocytes |
| 19. A6-2 | GalNAcα3(Fucα2)Galβ4GlcNAcβ3Galβ4Glcβ1Cer | - | - | Human erythrocytes |
| 20. A7-2 | GalNAcα3(Fucα2)Galβ4(Fucα3)GlcNAcβ3Galβ4Glcβ1Cer | - | - | Human erythrocytes |
| 21. | Galβ4GlcNAcβ6(Galβ4GlcNAcβ3)Galβ4Glcβ1Cer | - | - | Bovine buttermilk |
| 22. | Galβ4GlcNAcβ3Galβ4GlcNAcβ3Galβ4Glcβ1Cer | + | + | Rabbit thymus^{e} |
| | | | | |

| *Lactoseries* | | | | |
|---|---|---|---|---|
| 23. Lactotetra | Galβ3GlcNAcβ3Galβ4Glcβ1Cer | + | + | Human meconium |
| 24. Le^{a}-5 | Galβ3(Fucα4)GlcNAcβ3Galβ4Glcβ1Cer | + | - | Human meconium |
| 25.Le^{b}-6 | Fucα2Galβ3(Fucα4)GlcNAcβ3Galβ4Glcβ1Cer | - | - | Human meconium |
| 26. B7-1 | Galα3(Fucα2)Galβ3(Fucα4)GlcNAcβ3Galβ4Glcβ1Cer | - | - | Monkey intestine |
| | | | | |

| *Gangliosides* | | | | |
|---|---|---|---|---|
| 27. NeuAc-GM3 | NeuAcα3Galβ4Glcβ1Cer | - | - | Human brain |
| 28. NeuGc-GM3 | NeuGcα3Galβ4Glcβ1Cer | - | + | Horse erythrocytes |
| 29. GM1 | Galβ3GalNAcβ4(NeuAcα3)Galβ4Glcβ1Cer | - | + | Human brain |
| 30. GD1a | NeuAcα3Galβ3GalNAcβ4(NeuAcα3)Galβ4Glcβ1Cer | - | - | Human brain |
| 31. NeuAcα3SPG | NeuAcα3Galβ4GlcNAcβ3Galβ4Glcβ1Cer | - | + | Human erythrocytes |
| 32. NeuAcα6SPG | NeuAcα6Galβ4GlcNAcβ3Galβ4Glcβ1Cer | - | + | Human meconium |
| 33. NeuGcα3SPG | NeuGcα3Galβ4GlcNAcβ3Galβ4Glcβ1Cer | - | + | Rabbit thymus |
| 34. NeuAcα3Le^{a} | NeuAα3Galβ3(Fucα4)GlcNAcβ3Galβ4Glcβ1Cer | - | + | Human bilebladder tumor |
| 35. NeuAcα3Le^{x} | NeuAcα3Galβ4(Fucα3)GlcNAcβ3Galβ4Glcβ1Cer | - | + | Synthetic |
| 36. | NeuGcα3Galβ4GlcNAcβ3Galβ4GlcNAcβ3Galβ4Glcβ1Cer | + | + | Rabbit thymus |
| 37. | Galβ4GlcNAcβ6(NeuAcα6Galβ4GlcNAcβ3)Galβ4Glcβ1Cer | - | - | Bovine buttermilk |
| 38. Ge-GD2 | GalNAcβ4(NeuGcα8NeuGcα3)Galβ4Glcβ1Cer | - | - | Bovine intestine |
| 39. Ac-GD3 | NeuAcα8NeuAcα3Galβ4Glcβ1Cer | - | - | Bovine buttermilk |

| | | | | |
|---|---|---|---|---|
| a) In the shorthand nomenclature for fatty acids and bases, the number before the colon refers to the carbon chain length and the number after the colon gives the total number of double bonds in the molecule. Fatty acids with a 2-hydroxy group are denoted by the prefix h before the abbreviation e.g. h16:0. For long chain bases, d denotes dihydroxy and t trihydroxy. Thus d18:1 designates sphingosine (1,3-dihydroxy-2-aminooctadecene) and t18:0 phytosphingosine (1,3,4-trihydroxy-2-aminooctadacene). b) Binding is defined as follows: An significant darkening on the autoradiogram when 2 µg of the glycosphingolipid was applied on the thin-layer plate is denoted by +, while - denotes no binding c)Glycosphingolipid No. 6 was a kind gift from Dr. K. Stenvall, Symbicom AB, Lund, Sweden. d) Glycosphingolipids No. 9 was prepared from No. 15 by treatment with β-galactosidase. e) Glycosphingolipids No. 22 was prepared from No. 36 by mild acid hydrolysis. | | | | |

**TABLE 3. Binding of ³⁵S-labeled Helicobacter species to glycosphingolipids on thin-layer chromatograms**

| Trivial name | Structure | Binding^{a} of glycosphingolipids to: | | | | | | | |
|---|---|---|---|---|---|---|---|---|---|
| | | *H.pylori* 17874 | *H.pylori* 17875 | *H. felis*28539 | *H.canis* 33835 | *H.hepaticus* 33637 | *H.mustelae* 25715 | *H.mustelae* 23950 & 23951 | *H. bilis* 38995 |
| LacCer | Gal/β4Glcβ1Cer^{b} | + | + | + | + | + | + | + | + |
| Isoglobotri | GalαβGalβ4Glcβ1Cer | + | + | + | + | + | + | + | + |
| GgO4 | Galβ3GalNAcβ4Galβ4Glcβ1Cer | + | + | + | + | + | + | + | + |
| Le^{a}-₅ | Galβ3(Fucα4)GlcNAcβ3Galβ4Glcβ1Cer | - | - | - | - | - | - | - | - |
| Le^{b}-6 | Fucα2Galβ3(Fucα4)GlcNAcβ3Galβ4Glcβ1Cer | - | + | - | - | - | - | - | - |
| Globotetra | GalNAcβ3Galα4Galβ4Glcβ1Cer | - | - | - | - | - | - | - | - |
| Lactotetra | Galβ3GlcNAcβ3Galβ4Glcβ1Cer | + | + | + | + | + | + | + | + |
| Acid glycosphingolipids of human granulocytes | | + | - | - | - | + | + | - | - |
| NeuGc-nLc₆NeuGcαβ(Galβ4GlcNAcβ3)₂Galβ4Glcβ1Cer | | + | + | + | + | + | + | + | + |

| | | | | | | | | | |
|---|---|---|---|---|---|---|---|---|---|
| ^{a}Binding is defined as follows: + denotes a significant darkening on the autoradiogram when 2 µg (or 40 µg in the case of the last sample) was applied to TLC plates whereas - denotes no binding. ^{b} Ceramide composition (t18:0-h16:0-h24:0) | | | | | | | | | |

**TABLE 4. Binding specificities detected in different E. coli type strains**

| **STRAIN** | | | | | | | |
|---|---|---|---|---|---|---|---|
| | ETEC | ETEC | EPEC | EAGG | EIEC | EIEC | EHEC |
| CCUG | 17649 | 17650 | 38068 | 38077 | 38092 | 38094 | wt |
| **SPECIFICITY** | | | | | | | |
| LacCer-OH | + | + | + | + | + | + | + |
| Ganglio | + | + | + | + | + | + | + |
| Lacto | + | + | + | + | + | + | + |
| NeoLacto | + | + | + | + | + | + | + |
| Globo | + | + | + | + | + | + | - |
| Lea | ND | ND | + | ND | + | ND | + |
| Sialic acid | - | - | - | + | - | - | - |

### REFERENCES

Angstrom, J., S. Teneberg, M. A. Milh, T. Larsson, 1. Leonardsson, B. M. Olsson, M. O. Halvarsson, D. Danielsson, N. a. I, A. Ljungh, T. Wadström, and K. A. Karlsson. 1998. Glycobiology 8:297-309.
Bartus, H., Actor, P., Snipes, E., Sedlock, D., and Zajac, I. J. Clin Invest (1985) 21, 951-954
Borén, T., Falk, P., Roth, K. A., Larson, G. and Normark, S. (1993) Science, 262, 1892-1895.
Cravioto, A, Tello, A., Villafan, H., Ruiz, J., del Vedovo, S., and Neeser, J-C. (1991) J. Infect. Dis. 1247-1255
De Ree, J.M., Schwillens, P., and van den Bosch, JF FEMS Microbiol. Lett., (1985) 29, 91-97
Dewhurst, F.E., Fox, F.G., and On, S.L.W. (2000) Int. J. Syst. Evol Microbiol. 50, 2231-37
Ernst, B., Hart, G.W., and Sinay, P. (eds) (2000) Carbohydrates in Chemistry and Biology, ISBN 3-527-29511-9, Wiley-VCH, Weinheim
Evans, D.G., Evans, D.J jr., Glegg, S., Pauley, J.A. Infect. Immun (1979) 25, 738-748
Evans, D. G., Evans Jr, D.J., Molds, J. J., and Graham, D. Y. (1988) Infect. Immun., 56, 2896-06
Firon, N., Ofek, I., and Sharon, N. BBRC (1982) 105, 1426-1432
Fox, J.G. (2002) Gut 50, 273-283.
Fox, J. G., N. S. Taylor, M. Ihrig, M. I. Esteves, R. T. Chung, and M. M. Kaplan. 2000. Gut 47:A67-A67
Gerhard, M., S. Hirmo, T. Wadstrom, H. Miller-Pedroza, S. Teneberg, K. A. Karlsson, B. J. Appelmelk, S. Odenbreit, R. Haas, A. Arnqvist, and T. Boren. 2001. p. 185-206. In M. Achtman and S. Suerbaum (ed.), Helicobacter pylori: Molecular and Cellular Biology. Horizon Scientific Press, Wymondham
Hansson, G. C., Karlsson, K.-A., Larson, G., Strömberg, N., and Thurin, J. (1985) Anal. Biochem. 146, 158-163
Harvey, D.J., et al. (1993) Rapid Commun. Mass Spectrom. 7(7):614-9
Hunt, R. H. 1996. Scand J Gastroenterol Suppl 220:3-9
Idota, T., and Kawakami, H. (1995) Biosci. Biotech. Biochem. 59 (1) 69-72.
Jagannatha, H.M., Sharma, U.K., Ramaseshan, T., Surolia, A., and Balganesh, T.S. (1991) Microbial pathogenesis (11) 259-268.
Karlsson, K.-A. (1987) Methods Enzymol. 138, 212-220
Koerner Jr, T. A. W., Prestegard, J. H., Demou, P. C., and Yu, R. K. (1983) Biochemistry 22, 2676-2687
Lingwood, C. A., Huesca, M. and Kuksis, A. (1992) Infect. Immun., 60, 2470-2474.
Mahdavi, J., B. Sonden, M. Hurtig, F. O. Olfat, L. Forsberg, N. Roche, J. Angstrom, T. Larsson, S. Teneberg, K. Karlsson, S. Altraja, T. Wadstrom, D. Kersulyte, D. E. Berg, A. Dubois, C. Petersson, K.-E. Magnusson, T. Norberg, F. Lindh, B. B. Lundskog, A. Arnqvist, L. Hammarström, T. Boren, and T. Boren. 2002. Proceedings from the 5th International Workshop on Pathogenesis and Host Response in Helicobacter Infections:P3
Miller-Podraza, H., J. Bergström, S. Teneberg, M. Abul Milh, M. Longard, B.-M. Olsson, L. Uggla, and K.-A. Karlsson. 1999. Infect Immun 67:6309-13.
Miller-Podraza, H., Abul Milh, M., Bergström, J. and Karlsson, K.-A. (1996) Glycoconj. J., 13, 453-460.
Miller-Podraza, H., Bergström, J., Abul Milh, M. and Karlsson, K.-A. (1997a) Glycoconj. J.,14, 467-471.
Mysore, J.V., Wiggington, T., Simon, P.M., Zopf, D., Heman-Ackah, L.M. and Dubois, A. (1999) Gastroenterology, 117, 1316-1325.
Nakamura, T., Kawase, H., Kimura, K., Watanabe, Y., Ohtani, M., Arai, I., and Urashima, T (2003) J. Dairy Sci. 86: 1315-1320.
Nakhla, T., Fu, D., Zopf, D., Brodsky, N., and Hurt, H. (1999) British J. Nutr, 82, 361-367.
Nascimento de Araújo, A., and Giugliano, L.G. (2001) BMC Microbiol. 1,25.
Nascimento de Araújo, A., and Giugliano, L.G. (2000) FEMS Mircrobiol Lett 184, 91-94
Neeser, J.-R., Koellreutter, B., and Wuersch, P. (1986) Infect. Immun 52, 428-436.
Neeser, J.-R., Chambaz, A., Golliard, M., Link-Amster, H., Fryder, V., and Kolodziejczyk (1989) Infect. Immun 57, 3727-3734
Nilsson, H. O., M. Castedal, R Olsson, and T. Wadstrom. 1999. J Physiol Pharmacol 50:875-82
Nyman, T.A., et al. (1998) Eur. J. Biochem. 253(2):485-93
On, S. L. 2001. J Appl Microbiol 90 Suppl:1S-15S.
Oroe, H.S., Kolstoe, A-B., Wennerås, C., and Svennerholm, A.-M. FEMS Mircrobiol Lett (1990), 289-292.
Papac, D.I., et al. (1996) Anal. Chem. 68(18):3215-23
Pieroni, P., Worobec, E.A., Paranchych, W., and Armstrong, G.D. (1988) Infect. Immun. 56, 1334-1340.
Saarinen, J., et al. (1999) Eur. J. Biochem. 259(3):829-40
Saitoh, T., Natomi, H., Zhao, W., Okuzumi, K., Sugano, K., Iwamori, M. and Nagai, Y. (1991) FEBS Lett., 282, 385-387.
Samuelsson, B. E., Pimlott, W., and Karlsson, K.-A. (1990) Methods Enzymol. 193, 623-646
Scatelsky, I.C.A., Milani, S.R., Trabulsi, L.R., and Travassos, L.R. Infect Immun (1988) 56, 2979-298
Sears, P. and Wong, C-H. (1996) Proc. Natl. Acad. Sci., 93, 12086-12093.
Simon, P. M., Goode, P. L., Mobasseri, A., and Zopf, D. (1997) Infect. Immun. 65, 750-757.
Stellner, K., Saito, H., and Hakomori, S.-i. (1973) Arch. Biochem. Biophys. 155, 464-472
Teneberg, S., I. Leonardsson, H. Karlsson, P.Å., Jovall, J., Ångstrom, D. Danielsson, I. Näslund, A. Ljungh, T. Wadström, and K. A. Karlsson. 2002. J Biol Chem 277:19709-19
Toivonen, S., et al. (2002) J. Biol. Chem. 276(40):37141-8
Vanmaele, R.P., Finlayson, M.C., and Armstrong, G.D. (1995) Infection and Immunity 63 (1) 191-198
Vanmaele, R.P., Heerze, L.D., and Armstrong, G.D. (1999) Infection and Immunity 67, 3302-7
Waldi, D. (1962) Dünnschicht-Chromatographie. (Stahl, E., ed.) pp. 496-515. Springer-Verlag, Berlin
Wennerås, C., Neeser, J-R., and Svennerholm A.-M. Infection and Immunity (1995) 640-646.
Wennerås, C., Holmgren, J., and Svennerholm A.-M. FEMS Mircrobiol Lett (1990) 66, 107-112
Yang, H. J., and S. I. Hakomori. 1971. J Biol Chem 246:1192-200.

## Claims

1. A therapeutical composition containing purified fraction(s) of at least two compounds being or containing a pathogen inhibiting oligosaccharide sequence selected from the pathogen receptors
as defined in the formula
[Sacch1]ₘ₁Galβx(Fucα4)ₘ₂Glc[NAc]ₘ₃[β3Gal{β4Glc(NAc)ₙ₁}ₙ₂]ₙ₃[βR₂]ₙ₄ (I)
wherein x is linkage position 3 or 4, Sacch1 is GlcNAcβ3, Galα3, GalNAcβ4, Gala4, or Neu5Xα3/6, in which X is independently either Ac or Gc;
n1, n2, n3, n4, m1, m2, and m3 are independently integers 0 or 1 with the provisions that m2 may be 1 only when x is 3, m1 is 0, and m3 is 1;
m3 may be 0 only when Sacch1 is Neu5Xα3, Neu5Xα6, Gala3, GalNAcβ4 or Galα4;
when n4 is 1, then m3 is 0 and n3 is 0, and
when n4 is 0, then m1 is 1, m2 is 1, or n3 is 1;
R₂ is a ceramide comprising a hydroxyl fatty acid or an analog of a ceramide comprising a hydroxyl fatty acid;
Sacch1 is Galα3 or GalNAcβ4 with the provision that when the composition contains at least two receptors according to formula (I), these have at least one different variable selected from the group consisting of Sacch1, x, m2, and n4 with the provision that two sialic acid receptors or two neolacto receptors cannot be selected;
with the provision that when Sacch1 is Galα4, Neu5Xα3, Neu5Xα6, or GalNAcβ4, the oligosaccharide sequence according to the formula I may be a partial oligosaccharide sequence Galα4Gal, Neu5Xα3Gal, Neu5Xα6Gal, or GalNAcβ4Gal; and
with the provision that when the composition contains only one receptor according to formula (I) then it is together with at least one alpha-hexose receptor as defined in the formula
Hexap[(Hexαr)]ₙHex (II)
wherein Hex is Gal or Man, n is independently 0 or 1, p and r are linkage position 3 or 6 between Man residues, with the provision that when Hex is Man, then p is 3 and then r is 6, and when p is 6, then r is 3, and when Hex is Gal, then p is 4 and n is 0, with the provision that when Hex is Gal, it is not with Galα4Gal-receptor according to the formula I.

2. The composition according to claim 1, wherein the terminal activating sequence is Galα4 and the composition comprises the partial epitope Galα4Gal and a Mannose receptor comprising the oligosaccharide sequence
Manα3[(Manα6)]ₙMan,
wherein n is 0 or 1.

3. The composition according to claim 1 containing purified fraction(s) of at least two compounds being or containing a pathogen inhibiting oligosaccharide sequence selected from the pathogen receptors as defined by the formula
[Al]ₘ₃Galβ4Glc[βA2]ₙ₄ (Ib)
wherein m3 and n4 are independently integers 0 or 1;
wherein the natural type non-reducing end activator sequence A1 is selected from the group consisting of GalNAcβ4, Galα4, Neu5Xα3, Neu5Xα6, GalNAcβGalα4, Galβ3GalNAcβ4, Galβ4GlcNAcβ3, GlcNAcβ3Galβ4GlcNAc, Galβ3GlcNAcβ3, Neu5Xα3Galβ4GlcNAcβ3, Neu5Xα6Galβ4GlcNAcβ3, and Galβ3(Fuca4)GlcNAcβ3;
and wherein X is independently either Ac or Gc, and A2 is a ceramide comprising a hydroxyl fatty acid or an analog of a ceramide comprising a hydroxyl fatty acid.

4. The composition according to claim 3, wherein A1 is selected from the group consisting of Galα4, Neu5Xα3, Neu5Xα6, Galβ4GlcNAcβ3 or Galβ3GlcNAcβ3.

5. The composition according to claim 1 containing purified fraction(s) of at least two compounds being or containing a pathogen inhibiting oligosaccharide sequence selected from the pathogen receptors as defined by the formula
[Sacch1]ₘ₁[Galβx(Fucα4)ₘ₂GlcNAcβ3]ₘ₃Galβ4Glc[βA2]ₙ₄ (Ic)
wherein x is linkage position 3 or 4, Sacch1 is GlcNAcβ3, Galα3, GalNAcβ4, Gala4, or Neu5Xα3/6, in which X is independently either Ac or Gc;
n4, m1, m2, and m3 are independently integers 0 or 1,
with the provisions that m2 is 1 only when x is 3,
when Sacch1 is GlcNAcβ3, then m3 is 1 and x is 4, and
m3 may be 0 only when m1 is 1 or n4 is 1,
when n4 is 0, then m1 is 1 or m3 is 1;
A2 is a ceramide comprising a hydroxyl fatty acid or an analog of a ceramide comprising a hydroxyl fatty acid, and
with the provision that at least two receptors are selected so that these have at least one different variable selected from the group Sacch1 x, m2, n4, with the provision that not two sialic acid receptors are selected.

6. The composition according to claim 1 containing purified fraction(s) of at least two compounds being or containing a pathogen inhibiting oligosaccharide sequence selected from the pathogen receptors as defined by the formula
[Sacch1]ₘ₁[GalβxGlcNAcβ3]ₘ₃Galβ4Glc (Id)
wherein x is linkage position 3 or 4, Sacch1 is Galα4, Neu5Xα3 or Neu5Xα6, wherein X is independently either Ac or Gc;
m1, and m3 are independently integers 0 or 1,
with the provision that either m1 is 1 or m3 is 1,
with the provision that at least two receptors are selected so that these have at least one different variable Sacch1 or x, with the provision that not two sialic acid receptors are selected.

7. The composition according to claim 6, wherein the oligosaccharide sequences are selected from the group consisting of Galα4Galβ4Glc, NeuNAcα3Galβ4Glc, NeuNAcα6Galβ4Glc, NeuNAcα3Galβ4GlcNAc, NeuNAcα6Galβ4GlcNAc, Galβ4GlcNAcβ3Galβ4Glc and Galβ3GlcNAcβ3Galβ4Glc.

8. The composition according to claim 1, wherein at least one sialylated oligosaccharide selected from the group consisting of NeuNAcα3Galβ4Glc, NeuNAcα6Galβ4Glc and NeuNAcα6Galβ4GlcNAc, is used together with at least one neutral oligosaccharide selected from the group consisting of Galα4Galβ4Glc, Galα4Gal, Galβ4GlcNAcβ3Galβ4Glc (LNnT) and Galβ3GlcNAcβ3Galβ4Glc (LNT).

9. The composition according to claim 6, wherein said pathogen inhibiting oligosaccharides comprise a mixture of two different types of oligosaccarides selected from the group consisting of globo-oligosaccharides, Neolacto-oligosaccarides, and sialyl-oligosaccharides, and/or sialyllactoses.

10. The composition according to claim 1 comprising a purified fraction(s) of at least two compounds being or containing a pathogen inhibiting oligosaccharide sequence selected from at least two of the following groups of pathogen receptors:
a) lactosylceramide receptors as defined in the formula
R₁xGalβ4GlcβR₂ (X)
wherein x is linkage position 3 or 4, R₂ is a ceramide comprising a hydroxyl fatty acid or an analog of a ceramide comprising a hydroxyl fatty acid, and R₁ is Galα, Galβ, GalNAcβ, GlcNAcβ or a longer oligosaccharide comprising Galα, Galβ, GalNAcβ or GlcNAcβ at the reducing end or Neu5Xα, wherein X is Ac or Gc, with the proviso that when R₁ is GlcNAcβ or Neu5Xα then x is 3;
b) ganglio-receptors as defined in the formula
[Galβ3]ₙ₁GalNAc[β4Gal{β4Glc}ₙ₂]ₙ₃ (XI)
wherein n1, n2 and n3 are independently integers 0 or 1, with the proviso that either n1 or n3 is 1, and with the proviso that no sialic acids are linked to the oligosaccharide sequence;
c) Galα4Gal-receptors as defined in the formula
[GalNAcβ3]ₙ₁Galα4Gal{β4Glc(NAc)ₙ₂}ₙ₃ (XII)
wherein n1, n2, and n3 are independently integers 0 or 1, and the GalNAc-residue can be further substituted by other monosaccharide residues;
d) lacto-receptors as defined in the formula
Galβ3GlcNAc[β3Gal{β4Glc(NAc)ₙ₁}ₙ₂]ₙ₃ (XIII)
wherein n1, n2, and n3 are independently integers 0 or 1;
e) neolacto-receptors as defined in the formula
[GlcNAcβ3]ₙ₁Galβ4GlcNAc[β3Gal{β4Glc(NAc)ₙ₂}ₙ₃]ₙ₄ (XIV)
wherein n1 n2, n3 and n4 are independently integers 0 or 1, when n1 is 1, the non-reducing terminal GlcNAc can be further substituted by a monosaccharide residue or an oligosaccharide;
f) fucosyl-receptors as defined in the formula
Galβ3(Fucα4)GlcNAc[β3Gal{β4Glc(NAc)ₙ₁}ₙ₂]ₙ₃ (XV)
wherein n1, n2, and n3 are independently integers 0 or 1;
g) sialic acid-receptors as defined in the formula
Neu5XαpGalβr[(Fucαs)]ₙ₁Glc(NAc)ₙ₂ (XVI)
wherein independently X is either Ac or Gc meaning that the sialic acic is either Neu5Ac or Neu5Gc, n1 and n2 are either 0 or 1, p is linkage position 3 or 6, r and s are linkage positions 3 or 4 with the proviso that when r is 3 then s is 4 and when r is 4 then s is 3;
h) mannose receptors as defined in the formula
Manαp[(Manαr)]ₙ₁Man (XVII)
wherein n is independently 0 or 1, p and r are linkage position 3 or 6 between the Man residues, with the proviso that when p is 3 then r is 6, and when p is 6 then r is 3.

11. The composition according to claim 10, wherein the pathogen receptor of group a) is selected from the group of receptor oligosaccharide sequences consisting of:
lactosylceramide, lactosylceramide comprising hydroxyl fatty acids, lactosylceramide with modified carbon 3 of a galactose residue and isoglobotriaocylceramide

12. The composition according to claim 10, wherein the pathogen receptor of group g) is selected from the group of receptor oligosaccharide sequences consisting of:
oligosaccharides with Neu5Xα3Galβ3(Fucα4)GlcNAc,
Neu5Xα3Galβ4(Fucα3)GlcNAc, Neu5Xα3Galβ4(Fucα3)Glc,
Neu5Xα3Galβ3GlcNAc, Neu5Xα3Galβ4GlcNAc, Neu5Xα3Galβ4Glc,
Neu5Xα6Galβ4GlcNAc or Neu5Xα6Galβ4Glc structures

13. The composition according to claim 1, wherein at least one of said compounds is in monovalent form being a glycosylamine or a glycosylamide or a methyl glycoside or a glycoside including other N-glycosides, C-glycosides or S-glycosides.

14. The composition according to claim 1, wherein at least one of said compounds is linked to a polyvalent carrier.

15. The composition according to claim 14, wherein said polyvalent carrier is a carbohydrate carrier or a particle carrier or a soluble carbohydrate carrier, or a particle carrier or a bacterial polysaccharide or part of bacterial polysaccharide also comprising the receptor oligosaccharide sequence, or a carbohydrate particle, a synthetic polymer particle or a cell, or an antigenic or immunostimulating carbohydrate conjugate.

16. The composition according to claim 1 further comprising one or several oligosaccharide sequences selected from the group of:
oligosaccharides comprising sequences Fuca2Gal, Fucα3GlcNAc, Fucα3Glc, NeuNAcα8NeuNAc, Fucα2Galβ3/4GlcNAc, Fucα2Galβ4Glc, Fucα2Galβ4(Fucα3)Glc, Galβ4(Fucα3)GlcNAc, Fucα2Galβ3/4(Fucα4/3)GlcNAc and ganglioseries ganglioside oligosaccharide sequences.

17. Use of a composition comprising a pharmaceutically or therapeutically or prophylactically effective amount of the composition of claim 1 for the manufacture of a medicament for profylaxis or treatment of a bacterial infection.

18. The use according to claim 17, wherein said bacterial infection is a gastrointestinal infection.

19. The use according to claim 18, wherein said gastrointestinal infection causes diarrhea or traveller's diarrhea, children's diarrheas, persistent diarrhea, watery diarrhea, hemorrhagic colitis or haemolytic uremic syndrome.

20. The useaccording to claim 18, wherein said infection is caused by EPEC (enteropathogenic *Escherichia coli*), ETEC (enterotoxigenic *Escherichia coli),* EHEC (enterohemorrhagic *Escherichia coli),* EIEC (enteroinvasive *Escherichia coli*) or EAEC (enteroaggregative *Escherichia coli*).

21. The use according to claim 18, wherein said infection is caused by *Vibrio* species including *Vibrio cholerae, Campylobacter* species including *Campylobacter jejuni,* intestinal eukariotic parasites including the *Entamobae* species, *Salmonella* including *Salmonella typhimurium, Shigella* species, *Aeromonas* species, zoonotic *Helicobacter* species, *Listeria* species or rotavirus or the cause of infection is not diagnosed.

22. The use according to claim 17, wherein said subject is a human patient or an animal patient.

23. A method of improving food safety comprising a step of coating a food product with a composition according to claim 1.

24. A nutritional composition or a nutritional additive or infant formula comprising a purified fraction(s) of at least of two compounds as defined in claim 1 for prophylaxis or treatment of gastrointestinal infection.

25. A nutritional composition or a nutritional additive or infant formula according to claim 24 further comprising a probiotic microorganism or a prebiotic substance.

26. A product for inhibition of pathogens, especially diarrhea causing *E. coli, ex vivo* comprising a purified fraction(s) of at least of two compounds as defined in claim 1, wherein said product is selected from the group consisting of: a mouth hygiene product, a food coating product, a food preservative, or a topical, washing, or cosmetic product.

27. A method of analysis or diagnostics comprising a step of contacting a putative pathogenic or probiotic microbe with at least three pathogen receptors as defined in claim 1.

28. A method of analysis or diagnostics comprising a step of contacting a putative pathogenic or probiotic microbe with a receptor selected from the group consisting of:
lacto-receptors, neolacto-receptors, fucosyl-receptors, mannose receptors or sialic acid receptors
for analysis or diagnosis of pathogen or probiotic binding,
wherein the said receptors are
i) protein linked receptors and
ii) comprising a terminal non-reducing end oligosaccharide sequence present in the epithelium of human intestine, human stomach or human larynx.

29. A method for a search or design of bacteria binding oligosaccharide substances comprising a step of modelling the binding properties of the oligosaccharide receptors as defined in claim 1.

30. A diarrheagenic *E. coli* inhibiting substance according to the formula
[OS-(y)ₚ - (S)_{q} - (z)ᵣ -]ₙPO (SP1)
wherein PO is an oligomeric or polymeric carrier structure, OS is an oligosaccharide sequence as defined in Formula I or II of claim 1, n is an integer ≥ 1 indicating the number of oligosaccharide groups covalently attached to the carrier PO, S is a spacer group, p, q and r are each 0 or 1, whereby at least one of p and r is different from 0, y and z are linking groups, at least one of y and z being an O-hydroxylamine residue -0-NH- or - O-N=, with the nitrogen atom being linked to the OS and/or PO structure, respectively, and the other y and z, if present, is a chemoselective ligation group.

## Patentansprüche

1. Therapeutische Zusammensetzung, enthaltend (eine) aufgereinigte Fraktion(en) von mindestens zwei Verbindungen, die eine pathogenhemmende Oligosaccharidsequenz ausgewählt aus den Pathogenrezeptoren wie definiert in der Formel
[Sacch1]ₘ₁Galβx(Fucα4)ₘ₂Glc[NAc]ₘ₃[β3Gal{β4Glc(NAc)ₙ₁}ₙ₂]ₙ₃[βR₂]ₙ₄ (I)
sind oder enthalten,
worin x Bindungsposition 3 oder 4 ist, Sacch1 GlcNAcβ3, Gala3, GalNAcβ4, Gala4 oder Neu5Xα3/6 ist, in dem X unabhängig entweder Ac oder Gc ist;
n1, n2, n3, n4, m1, m2 und m3 unabhängig die ganzen Zahlen 0 oder 1 sind, mit den Maßgaben, dass m2 1 sein kann nur, wenn x 3 ist, m1 0 ist und m3 1 ist;
m3 0 sein kann nur, wenn Sacch1 Neu5Xα3, Neu5Xα6, Gala3, GalNAcβ4 oder Gala4 ist;
wenn n4 1 ist, dann m3 0 und n3 0 ist, und
wenn n4 0 ist, dann m1 1, m2 1 oder n3 1 ist;
R₂ ein Ceramid ist, das eine Hydroxyfettsäure umfasst, oder ein Ceramidanalogon, das eine Hydroxyfettsäure umfasst;
Sacch1 Gala3 oder GalNAcβ4 ist, mit der Maßgabe, dass, wenn die Zusammensetzung mindestens zwei Rezeptoren gemäß Formel (I) enthält, diese mindestens eine unterschiedliche Variable aufweisen, die aus der Gruppe bestehend aus Sacch1, x, m2 und n4 ausgewählt ist, mit der Maßgabe, dass nicht zwei Sialinsäurerezeptoren oder zwei Neolacto-Rezeptoren ausgewählt werden können;
mit der Maßgabe, dass, wenn Sacch1 Gala4, Neu5Xα3, Neu5Xα6 oder GalNAcβ4 ist, die Oligosaccharidsequenz gemäß Formel (I) eine partielle Oligosaccharidsequenz Galα4Gal, Neu5Xα3Gal, Neu5Xα6Gal oder GalNAcβ4Gal sein kann; und
mit der Maßgabe, dass, wenn die Zusammensetzung nur einen Rezeptor gemäß Formel (I) enthält, er dann zusammen mit mindestens einem Alpha-Hexose-Rezeptor wie in der Formel
Hexαp[(Hexar)]ₙHex (II)
definiert vorliegt, wobei Hex Gal oder Man ist, n unabhängig 0 oder 1 ist, p und r die Bindungsposition 3 oder 6 zwischen den Man-Resten sind, mit der Maßgabe, dass, wenn Hex Man ist, p dann 3 ist, und r dann 6 ist, und wenn p 6 ist, r dann 3 ist, und wenn Hex Gal ist, dann p 4 und n 0 ist, mit der Maßgabe, dass, wenn Hex Gal ist, es nicht zusammen mit dem Galα4Gal-Rezeptor gemäß Formel I ist.

2. Zusammensetzung gemäß Anspruch 1, wobei die terminale aktivierende Sequenz Gala4 ist und die Zusammensetzung das partielle Galα4Gal-Epitop und einen Mannoserezeptor umfasst, der die Oligosaccharidsequenz
Manα3[(Manα6)]ₙMan,
umfasst, worin n 0 oder 1 ist.

3. Zusammensetzung gemäß Anspruch 1, enthaltend (eine) aufgereinigte Fraktion(en) von mindestens zwei Verbindungen, die eine pathogenhemmende Oligosaccharidsequenz ausgewählt aus den Pathogenrezeptoren wie definiert in der Formel
[Al]ₘ₃Galβ4Glc[βA2]ₙ₄ (Ib)
sind oder enthalten,
worin m3 und n4 unabhängig die ganzen Zahlen 0 oder 1 sind;
worin die Aktivatorsequenz A1 vom natürlichen Typ am nicht-reduzierenden Ende ausgewählt ist aus der Gruppe bestehend aus GalNAcβ4, Gala4, Neu5Xα3, Neu5Xα6, GalNAcβ3Galα4, Galβ3GalNAcβ4, Galβ4GlcNAcβ3, GlcNAcβ3Galβ4GlcNAc, Galβ3GlcNAcβ3, Neu5Xα3Galβ4GlcNAcβ3, Neu5Xα6Galβ4GlcNAcβ3 und Galβ3(Fucα4)GlcNAcβ3;
und worin X unabhängig entweder Ac oder Gc ist und A2 ein Ceramid ist, das eine Hydroxyfettsäure umfasst, oder ein Ceramidanalogon, das eine Hydroxyfettsäure umfasst.

4. Zusammensetzung gemäß Anspruch 3, worin A1 ausgewählt ist aus der Gruppe bestehend aus Gala4, Neu5Xα3, Neu5Xα6, Galβ4GlcNAcβ3 oder Galβ3GlcNAcβ3.

5. Zusammensetzung gemäß Anspruch 1, enthaltend (eine) aufgereinigte Fraktion(en) von mindestens zwei Verbindungen, die eine pathogenhemmende Oligosaccharidsequenz ausgewählt aus den Pathogenrezeptoren wie definiert in der Formel
[Sacch1]ₘ₁[Galβx(Fucα4)ₘ₂GlcNAcβ3]ₘ₃Galβ4Glc[βA2]ₙ₄ (Ic)
sind oder enthalten,
worin x die Bindungsposition 3 oder 4 ist, Sacch1 GlcNAcβ3, Gala3, GalNAcβ4, Gala4 oder Neu5Xα3/6 ist, in dem X unabhängig entweder Ac oder Gc ist;
n4, m1, m2 und m3 unabhängig die ganzen Zahlen 0 oder 1 sind, mit den Maßgaben, dass m2 1 ist nur, wenn x 3 ist,
wenn Sacch1 GlcNAcβ3 ist, dann m3 1 und x 4 ist, und
m3 0 sein kann nur, wenn m1 1 ist oder n4 1 ist,
wenn n4 0 ist, dann m1 1 oder m3 1 ist;
A2 ein Ceramid ist, das eine Hydroxyfettsäure umfasst, oder ein Ceramidanalogon, das eine Hydroxyfettsäure umfasst, und
mit der Maßgabe, dass mindestes zwei Rezeptoren so ausgewählt sind, dass diese mindestens eine unterschiedliche Variable ausgewählt aus der Gruppe Sacch1, x, m2, n4 enthalten, mit der Maßgabe, dass nicht zwei Sialinsäurerezeptoren ausgewählt werden.

6. Zusammensetzung gemäß Anspruch 1, enthaltend (eine) aufgereinigte Fraktion(en) von mindestens zwei Verbindungen, die eine pathogenhemmende Oligosaccharidsequenz ausgewählt aus den Pathogenrezeptoren wie definiert in der Formel
[Sacch1]ₘ₁[GalβxGlcNAcβ3]ₘ₃Galβ4Glc (Id)
sind oder enthalten,
worin x die Bindungsposition 3 oder 4 ist, Sacch1 Galα4, Neu5Xα3 oder Neu5Xα6 ist,
worin X unabhängig entweder Ac oder Gc ist;
m1 und m3 unabhängig die ganzen Zahlen 0 oder 1 sind,
mit der Maßgabe, dass entweder m1 1 ist oder m3 1 ist,
mit der Maßgabe, dass mindestes zwei Rezeptoren so ausgewählt sind, dass diese mindestens eine unterschiedliche Variable Sacch1 oder x enthalten, mit der Maßgabe, dass nicht zwei Sialinsäurerezeptoren ausgewählt werden.

7. Zusammensetzung gemäß Anspruch 6, worin die Oligosaccharidsequenzen ausgewählt sind aus der Gruppe bestehend aus Galα4Galβ4Glc, NeuNAcα3Galβ4Glc, NeuNAcα6Galβ4Glc, NeuNAcα3Galβ4GlcNAc, NeuNAcα6Galβ4GlcNAc, Galβ4GlcNAcβ3Galβ4Glc und Galβ3GlcNAcβ3Galβ4Glc.

8. Zusammensetzung gemäß Anspruch 1, worin mindestens ein sialyliertes Oligosaccharid ausgewählt aus der Gruppe bestehend aus NeuNAcα3Galβ4Glc, NeuNAcα6Galβ4Glc und NeuNAcα6Galβ4GlcNAc, zusammen mit mindestens einem neutralen Oligosaccharid ausgewählt aus der Gruppe bestehend aus Galα4Galβ4Glc, Galα4Gal, Galβ4GlcNAcβ3Galβ4Glc (LNnT) und Galβ3GlcNAcβ3Galβ4Glc (LNT) verwendet wird.

9. Zusammensetzung gemäß Anspruch 6, worin die pathogenhemmenden Oligosaccharide ein Gemisch aus zwei verschiedenen Oligosaccharidtypen ausgewählt aus der Gruppe bestehend aus Globo-Oligosacchariden, Neolacto-Oligosacchariden und Sialyl-Oligosacchariden und/oder Silalyllactosen umfassen.

10. Zusammensetzung gemäß Anspruch 1, umfassend (eine) aufgereinigte Fraktion(en) von mindestens zwei Verbindungen, die eine pathogenhemmende Oligosaccharidsequenz sind oder enthalten, ausgewählt aus mindestens zwei der folgenden Gruppen von Pathogenrezeptoren:
a) Lactosylceramid-Rezeptoren wie in der Formel
R₁xGalβ4GlcβR₂ (X)
definiert, worin x die Bindungsposition 3 oder 4 ist, R₂ ein Ceramid ist, das eine Hydroxyfettsäure umfasst, oder ein Ceramidanalogon ist, das eine Hydroxyfettsäure umfasst, und R₁ Galα, Galβ, GalNAcβ, GlcNAcβ oder ein längeres Oligosaccharid ist, das Galα, Galβ, GalNAcβ oder GlcNAcβ am reduzierenden Ende umfasst, oder Neu5Xα, worin X Ac oder Gc ist, mit der Maßgabe, dass, wenn R1 GlcNAcβ oder Neu5Xα ist, x dann 3 ist;
b) Ganglio-Rezeptoren wie in der Formel
[Galβ3]ₙ₁GalNAc[β4Gal{β4Glc}ₙ₂]ₙ₃ (XI)
definiert, worin n1, n2 und n3 unabhängig die ganzen Zahlen 0 oder 1 sind, mit der Maßgabe, dass entweder n1 oder n3 1 ist, und mit der Maßgabe, dass keine Sialinsäuren mit der Oligosaccharidsequenz verbunden sind;
c) Galα4Gal-Rezeptoren wie in der Formel
[GalNAcβ3]ₙ₁Galα4Gal{β4Glc(NAc)ₙ₂}ₙ₃ (XII)
definiert, worin n1, n2 und n3 unabhängig die ganzen Zahlen 0 oder 1 sind, und der GalNAc-Rest weiter mit anderen Monosaccharidresten substituiert sein kann;
d) Lacto-Rezeptoren wie in der Formel
Galβ3GlcNAc[β3Gal{β4Glc(NAc)ₙ₁}ₙ₂]ₙ₃ (XIII)
definiert, worin n1, n2 und n3 unabhängig die ganzen Zahlen 0 oder 1 sind;
e) Neolacto-Rezeptoren wie in der Formel
[GlcNAcβ3]ₙ₁Galβ4GlcNAc[β3Gal{β4Glc(NAc)ₙ₂}ₙ₃]ₙ₄ (XIV)
definiert, worin n1, n2, n3 und n4 unabhängig die ganzen Zahlen 0 oder 1 sind, wenn n1 1 ist, das nicht-reduzierende terminale GlcNAc weiter mit einem Monosaccharidrest oder einem Oligosaccharid substituiert sein kann;
f) Fucosyl-Rezeptoren wie in der Formel
Galβ3(Fucα4)GlcNAc[β3Gal{β4Glc(NAc)ₙ₁}ₙ₂]ₙ₃ (XV)
definiert, worin n1, n2 und n3 unabhängig die ganzen Zahlen 0 oder 1 sind;
g) Sialinsäure-Rezeptoren wie in der Formel
Neu5XαpGalβr[(Fucαs)ₙ₁Glc(NAc)ₙ₂ (XVI)
definiert, worin X unabhängig entweder Ac oder Gc ist, was bedeutet, dass die Sialinsäure entweder Neu5Ac oder Neu5Gc ist, n1 und n2 entweder 0 oder 1 sind, p die Bindungsposition 3 oder 6 ist, r und s die Bindungspositionen 3 oder 4 sind, mit der Maßgabe, dass wenn r 3 ist, s dann 4 ist, und wenn r 4 ist, s dann 3 ist;
h) Mannoserezeptoren wie in der Formel
Manαp[(Manαr)]ₙ₁Man (XVII)
definiert, worin n unabhängig 0 oder 1 ist, p und r die Bindungsposition 3 oder 6 zwischen den Man-Resten sind, mit der Maßgabe, dass wenn p 3 ist, r dann 6 ist, und wenn p 6 ist, r dann 3 ist.

11. Zusammensetzung gemäß Anspruch 10, worin der Pathogenrezeptor der Gruppe a) ausgewählt ist aus der Gruppe der Rezeptoroligosaccharidsequenzen bestehend aus:
Lactosylceramid, Lactosylceramid, das Hydroxyfettsäuren umfasst, Lactosylceramid mit modifiziertem Kohlenstoff 3 von einem Galactose-Rest und Isoglobotriaocylceramid.

12. Zusammensetzung gemäß Anspruch 10, worin der Pathogenrezeptor der Gruppe g) ausgewählt ist aus der Gruppe der Rezeptoroligosaccharidsequenzen bestehend aus:
Oligosacchariden mit Neu5Xα3Galβ3(Fucα4)GlcNAc-, Neu5Xα3Galβ4(Fucα3)GlcNAc-, Neu5Xα3Galβ4(Fucα3)Glc-, Neu5Xα3Galβ3GlcNAc-, Neu5Xα3Galβ4GlcNAc-, Neu5Xα3Galβ4Glc-, Neu5Xα6Galβ4GlcNAc- oder Neu5Xα6Galβ4Glc-Strukturen.

13. Zusammensetzung gemäß Anspruch 1, worin mindestens eine der Verbindungen in monovalenter Form ist und ein Glycosylamin oder ein Glycosylamid oder ein Methylglycosid oder ein Glycosid ist, das andere N-Glycoside, C-Glycoside oder S-Glycoside einschließt.

14. Zusammensetzung gemäß Anspruch 1, worin mindestens eine der Verbindungen mit einem polyvalenten Träger verbunden ist.

15. Zusammensetzung gemäß Anspruch 14, worin der polyvalente Träger ein Kohlenhydratträger oder ein Partikelträger oder ein löslicher Kohlenhydratträger ist, oder ein Partikelträger oder ein bakterielles Polysaccharid oder Teil eines bakteriellen Polysaccharids ist, das auch die Rezeptoroligosaccharidsequenz umfasst, oder ein Kohlenhydratpartikel, ein synthetisches Polymerpartikel oder eine Zelle oder ein antigenes oder immunstimulierendes Kohlenhydratkonjugat.

16. Zusammensetzung gemäß Anspruch 1, die weiter eine oder mehrere Oligosaccharidsequenzen umfasst, die ausgewählt sind aus der Gruppe der:
Oligosaccharide, umfassend die Sequenzen Fuca2Gal, Fucα3GlcNAc, Fuca3Glc, NeuNAcα8NeuNAc, Fucα2Galβ3/4GlcNAc, Fucα2Galβ4Glc, Fucα2Galβ4(Fucα3)Glc, Galβ4(Fucα3)GlcNAc, Fucα2Galβ3/4(Fucα4/3)GlcNAc und Gangliosidoligosaccharidsequenzen der Ganglioserie.

17. Verwendung einer Zusammensetzung, die eine pharmazeutisch oder therapeutisch oder prophylaktisch wirksame Menge der Zusammensetzung nach Anspruch 1 umfasst, für die Herstellung eines Medikaments für die Prophylaxe oder Behandlung einer bakteriellen Infektion.

18. Verwendung gemäß Anspruch 17, worin die bakterielle Infektion eine gastrointestinale Infektion ist.

19. Verwendung gemäß Anspruch 18, worin die gastrointestinale Infektion Diarrhöe, Reisediarrhöe, Kinderdiarrhöe, persistierende Diarrhöe, wässrige Diarrhöe, hämorrhagische Kolitis oder hämolytisches urämisches Syndrom verursacht.

20. Verwendung gemäß Anspruch 18, worin die Infektion durch EPEC (enteropathogenes Escherichia coli), ETEC (enterotoxigenes Escherichia coli), EHEC (enterohämorrhagisches Escherichia coli), EIEC (enteroinvasives Escherichia coli) oder EAEC (enteroaggregatives Escherichia coli) verursacht wird.

21. Verwendung gemäß Anspruch 18, worin die Infektion durch Vibrio-Arten einschließlich Vibrio cholerae, Campylobacter-Arten einschließlich Campylobacter jejuni, intestinale eukaryontische Parasiten einschließlich der Entamobae-Arten, Salmonella einschließlich Salmonella typhimurium, Shigella-Arten, Aeromonas-Arten, zoonotische Helicobacter-Arten, Listeria-Arten oder Rotavirus verursacht wird oder die Ursache der Infektion nicht diagnostiziert ist.

22. Verwendung gemäß Anspruch 17, worin das Individuum ein menschlicher Patient oder ein tierischer Patient ist.

23. Verfahren zur Verbesserung der Lebensmittelsicherheit, das einen Schritt der Beschichtung eines Lebensmittelprodukts mit einer Zusammensetzung gemäß Anspruch 1 umfasst.

24. Nährstoffzusammensetzung oder Nährstoffzusatz oder Säuglingsnahrung, umfassend (eine) aufgereinigte Fraktion(en) von mindestens zwei Verbindungen gemäß der Definition in Anspruch 1, für die Prophylaxe oder Behandlung einer gastrointestinalen Infektion.

25. Nährstoffzusammensetzung oder Nährstoffzusatz oder Säuglingsnahrung gemäß Anspruch 24, die/der weiter einen probiotischen Mikroorganismus oder einen präbiotischen Stoff enthält.

26. Produkt zur Hemmung von Pathogenen, vor allem Diarrhöe verursachenden E. coli, ex vivo, umfassend (eine) aufgereinigte Fraktion(en) von mindestens zwei Verbindungen gemäß der Definition in Anspruch 1, worin das Produkt ausgewählt ist aus der Gruppe bestehend aus: einem Mundhygieneprodukt, einem Lebensmittelbeschichtungsprodukt, einem Lebensmittelkonservierungsmittel oder einem topischen, Wäsche- oder Kosmetik-Produkt.

27. Verfahren zur Analyse oder Diagnose, umfassend einen Schritt, in dem eine putativ pathogene oder probiotische Mikrobe mit mindestens drei Pathogenrezeptoren, wie in Anspruch 1 definiert, in Kontakt gebracht wird.

28. Verfahren zur Analyse oder Diagnose, umfassend einen Schritt, in dem eine putativ pathogene oder probiotische Mikrobe mit einem Rezeptor in Kontakt gebracht wird, der ausgewählt ist aus der Gruppe bestehend aus:
Lacto-Rezeptoren, Neolacto-Rezeptoren, Fucosyl-Rezeptoren, Mannose-Rezeptoren oder Sialinsäure-Rezeptoren
für die Analyse oder Diagnose pathogener oder probiotischer Bindung,
worin die Rezeptoren
i) proteinverknüpfte Rezeptoren sind und
ii) eine terminale Oligosaccharidsequenz am nicht-reduzierenden Ende umfassen, die im Epithel des menschlichen Darms, des menschlichen Magens oder der menschlichen Larynx vorhanden ist.

29. Verfahren zum Suchen oder Gestalten von bakterienbindenden Oligosaccharidsubstanzen, umfassend einen Schritt der Modellierung der Bindeeigenschaften der Oligosaccharidrezeptoren wie in Anspruch 1 definiert.

30. Stoff, der Diarrhöe verursachendes E. coli hemmt, mit der Formel
[OS-(y)ₚ - (S)_{q} - (z)ᵣ ]ₙPO (SP1)
worin PO eine oligomere oder polymere Trägerstruktur ist, OS eine Oligosaccharidsequenz ist, wie in der Formel I oder II in Anspruch 1 definiert, n eine ganze Zahl ≥ 1 ist, die die Zahl der kovalent an den Träger PO gebundenen Oligosaccharidgruppen angibt, S eine Abstandsgruppe ist, p, q und r jeweils 0 oder 1 ist, wobei mindestens eine von p und r von 0 verschieden ist, y und z Bindungsgruppen sind, wobei mindestens eine von y und z ein O-Hydroxylaminrest -O-NH- oder -O-N= ist, wobei das Stickstoffatom mit der OS- und/oder PO-Struktur verbunden ist, und die andere y und z, wenn vorhanden, eine chemoselektive Ligationsgruppe ist.

## Revendications

1. Composition thérapeutique contenant une ou plusieurs fractions purifiées d'au moins deux composés étant ou contenant une séquence oligosaccharidique inhibant des agents pathogènes choisie parmi les récepteurs d'agents pathogènes tels que définis dans la formule
[Sacch1]ₘ₁Galβx(Fucα4)ₘ₂Glc[NAc]ₘ₃[β3Gal{β4Glc(NAc)ₙ₁}ₙ₂]ₙ₃[βR₂]ₙ₄ (I)
dans laquelle x représente une position de liaison en 3 ou 4, Sacch1 représente GlcNAcβ3, Gala3, GalNAcβ4, Gala4 ou Neu5Xα3/6, où X représente indépendamment soit Ac soit Gc ;
n1, n2, n3, n4, m1, m2 et m3 représentent indépendamment des nombres entiers de 0 ou 1
à condition que m2 puisse valoir 1 seulement lorsque x représente 3, m1 vaut 0 et m3 vaut 1 ;
m3 puisse valoir 0 seulement lorsque Sacch1 représente Neu5Xα3, Neu5Xα6, Gala3, GalNAcβ4 ou Gala4 ;
lorsque n4 vaut 1, alors m3 vaut 0 et n3 vaut 0, et
lorsque n4 vaut 0, alors m1 vaut 1, m2 vaut 1 ou n3 vaut 1 ;
R₂ représente un céramide comprenant un acide gras hydroxylé ou un analogue d'un céramide comprenant un acide gras hydroxylé ;
Sacch1 représente Gala3 ou GalNAcβ4 à condition que lorsque la composition contient au moins deux récepteurs selon la formule (I), ceux-ci aient au moins une variable différente choisie dans le groupe constitué de Sacch1, x, m2 et n4 à condition que deux récepteurs d'acide sialique ou deux néolacto-récepteurs ne puissent pas être choisis ;
à condition que lorsque Sacch1 représente Gala4, Neu5Xα3, Neu5Xα6 ou GalNAcβ4, la séquence oligosaccharidique selon la formule 1 puisse être une séquence oligosaccharidique partielle Gala4Gal, Neu5Xα3Gal, Neu5Xα6Gal ou GalNAcβ4Gal ; et
à condition que lorsque la composition contient seulement un récepteur selon la formule (I) alors il est conjointement avec au moins un récepteur d'hexose alpha tel que défini dans la formule
Hexαp[(Hexαr)]ₙHex (II)
dans laquelle Hex représente Gal ou Man, n vaut indépendamment 0 ou 1, p et r représentent une position de liaison en 3 ou 6 entre les résidus Man, à condition que lorsque Hex représente Man, alors p représente 3 et alors r représente 6, et lorsque p représente 6, alors r représente 3, et lorsque Hex représente Gal, alors p représente 4 et n vaut 0, à condition que lorsque Hex représente Gal, il ne soit pas avec le récepteur Galα4Gal selon la formule I.

2. Composition selon la revendication 1, dans laquelle la séquence d'activation terminale est Gala4 et la composition comprend l'épitope partiel Galα4Gal et un récepteur de Mannose comprenant la séquence oligosaccharidique
Manα3 [(Manα6)]ₙMan,
dans laquelle n vaut 0 ou 1.

3. Composition selon la revendication 1, contenant une ou plusieurs fractions purifiées d'au moins deux composés étant ou contenant une séquence oligosaccharidique inhibant des agents pathogènes choisie parmi les récepteurs d'agents pathogènes tels que définis par la formule
[Al]ₙ₃Galβ4Glc[βA2]ₙ₄ (Ib)
dans laquelle m3 et n4 représentent indépendamment des nombres entiers de 0 ou 1 ;
où la séquence activatrice terminale de type non réducteur naturelle A1 est choisie dans le groupe constitué de GalNAcβ4, Gala4, Neu5Xα3, Neu5Xα6, GalNAcβ3Galα4, Galβ3GalNAcβ4, Galβ4GlcNAcβ3, GlcNAcβ3Galβ4GlcNAc, Galβ3GlcNAcβ3, Neu5Xα3Galβ4GlcNAcβ3, Neu5Xα6Galβ4GlcNAcβ3 et Galβ3(Fucα4)GlcNAcβ3 ; et où X représente indépendamment soit Ac soit Gc, et A2 représente un céramide comprenant un acide gras hydroxylé ou un analogue d'un céramide comprenant un acide gras hydroxylé.

4. Composition selon la revendication 3, dans laquelle A1 est choisi dans le groupe constitué de Gala4, Neu5Xα3, Neu5Xα6, Galβ4GlcNAcβ3 ou Galβ3GlcNAcβ3.

5. Composition selon la revendication 1, contenant une ou plusieurs fractions purifiées d'au moins deux composés étant ou contenant une séquence oligosaccharidique inhibant des agents pathogènes choisie parmi les récepteurs d'agents pathogènes tels que définis par la formule
[Sacch1]ₘ₁[Galβx(Fucα4)ₘ₂GlcNAcβ3]ₘ₃Galβ4Glc[βA2]ₙ₄ (Ic)
dans laquelle x représente une position de liaison en 3 ou 4, Sacch1 représente GlcNAcβ3, Gala3, GalNAcβ4, Gala4 ou Neu5Xα3/6, où X représente indépendamment soit Ac soit Gc ;
n4, m1, m2 et m3 représentent indépendamment des nombres entiers de 0 ou 1 ,
à condition que m2 vaille 1 seulement lorsque x représente 3,
lorsque Sacch1 représente GlcNAcβ3, alors m3 vaut 1 et x représente 4, et
m3 puisse valoir 0 uniquement lorsque m1 vaut 1 ou n4 vaut 1,
lorsque n4 vaut 0, alors m1 vaut 1 ou m3 vaut 1 ;
A2 représente un céramide comprenant un acide gras hydroxylé ou un analogue d'un céramide comprenant un acide gras hydroxylé, et
à condition qu'au moins deux récepteurs soient choisis de manière à ce qu'ils aient au moins une variable différente choisie dans le groupe de Sacch1, x, m2, n4, à condition que deux récepteurs d'acide sialique ne soient pas choisis.

6. Composition selon la revendication 1, contenant une ou plusieurs fractions purifiées d'au moins deux composés étant ou contenant une séquence oligosaccharidique inhibant des agents pathogènes choisie parmi les récepteurs d'agents pathogènes tels que définis par la formule
[Sacch1]ₘ₁[GalβxGlcNAcβ3]ₘ₃Galβ4Glc (Id)
dans laquelle x représente une position de liaison en 3 ou 4, Sacch1 représente Gala4, Neu5Xα3 ou Neu5Xα6, où X représente indépendamment soit Ac soit Gc ;
m1 et m3 représentent indépendamment des nombres entiers de 0 ou 1,
à condition que soit m1 vaille 1 soit m3 vaille 1,
à condition qu'au moins deux récepteurs soient choisis de manière à ce qu'ils aient au moins une variable différente Sacch1 ou x, à condition que deux récepteurs d'acide sialique ne soient pas choisis.

7. Composition selon la revendication 6, dans laquelle les séquences oligosaccharidiques sont choisies dans le groupe constitué de Galα4Galβ4Glc, NeuNAcα3Galβ4Glc, NeuNAcα6Galβ4Glc, NeuNAcα3Galβ4GlcNAc, NeuNAcα6Galβ4GlcNAc, Galβ4GlcNAcβ3Galβ4Glc et Galβ3GlcNAcβ3Galβ4Glc.

8. Composition selon la revendication 1, dans laquelle au moins un oligosaccharide sialylé choisi dans le groupe constitué de NeuNAcα3Galβ4Glc, NeuNAcα6Galβ4Glc et NeuNAcα6Galβ4GlcNAc, est utilisé conjointement avec au moins un oligosaccharide neutre choisi dans le groupe constitué de Galα4Galβ4Glc, Gala4Gal, Galβ4GlcNAcβ3Galβ4Glc (LNnT) et Galβ3GlcNAcβ3Galβ4Glc (LNT).

9. Composition selon la revendication 6, dans laquelle lesdits oligosaccharides inhibant des agents pathogènes comprennent un mélange de deux types différents d'oligosaccharides choisis dans le groupe constitué de globo-oligosaccharides, néolacto-oligosaccharides et sialyl-oligosaccharides et/ou de sialyl-lactoses.

10. Composition selon la revendication 1, comprenant une ou plusieurs fractions purifiées d'au moins deux composés étant ou contenant une séquence oligosaccharidique inhibant des agents pathogènes choisie parmi au moins deux des groupes suivants de récepteurs d'agents pathogènes :
a) récepteurs de lactosylcéramide tels que définis dans la formule
R₁xGalβ4GlcβR₂ (X)
dans laquelle x représente une position de liaison en 3 ou 4, R₂ représente un céramide comprenant un acide gras hydroxylé ou un analogue d'un céramide comprenant un acide gras hydroxylé, et R₁ représente Galα, Galβ, GalNAcβ, GlcNAcβ ou un oligosaccharide plus long comprenant Galα, Galβ, GalNAcβ ou GlcNAcβ à l'extrémité réductrice ou Neu5Xα, où X représente Ac ou Gc, à condition que lorsque R₁ représente GlcNAcβ ou Neu5Xα alors x représente 3 ;
b) ganglio-récepteurs tels que définis dans la formule
[Galβ3]ₙ₁GalNAc[β4Gal{β4Glc}ₙ₂]ₙ₃ (XI)
dans laquelle n1, n2 et n3 représentent indépendamment des nombres entiers de 0 ou 1, à condition que soit n1 soit n3 vaille 1, et à condition qu'aucun acide sialique ne soit lié à la séquence oligosaccharidique ;
c) récepteurs de Galα4Gal tels que définis dans la formule
[GalNAcβ3]ₙ₁Galα4Gal{β4Glc(NAc)ₙ₂}ₙ₃ (XII)
dans laquelle n1, n2 et n3 représentent indépendamment des nombres entiers de 0 ou 1, et le résidu GalNAc peut être en outre substitué par d'autres résidus monosaccharidiques ;
d) lacto-récepteurs tels que définis dans la formule
Galβ3GlcNAc[β3Gal{β4Glc(NAc)ₙ₁}ₙ₂]ₙ₃ (XIII)
dans laquelle n1, n2 et n3 représentent indépendamment des nombres entiers de 0 on 1 ;
e) néolacto-récepteurs tels que définis dans la formule
[GlcNAcβ3]ₙ₁Galβ4GlcNAc[β3Gal{β4Glc(NAc)ₙ₂}ₙ₃]ₙ₄ (XIV)
dans laquelle n1, n2, n3 et n4 représentent indépendamment des nombres entiers de 0 ou 1, lorsque n1 vaut 1, le GlcNAc terminal réducteur peut être en outre substitué par un résidu monosaccharidique ou un oligosaccharide ;
f) fucosyl-récepteurs tels que définis dans la formule
Galβ3(Fucα4)GlcNAc[β3Gal{β4Glc(NAc)ₙ₁}ₙ₂]ₙ₃ (XV)
dans laquelle n1, n2 et n3 représentent indépendamment des nombres entiers de 0 on 1 ;
g) récepteurs d'acide sialique tels que définis dans la formule
Neu5XαpGalβr[(Fucαs)]ₙ₁Glc(NAc)ₙ₂ (XVI)
dans laquelle X représente indépendamment soit Ac soit Gc, ce qui signifie que l'acide sialique est soit Neu5Ac soit Neu5Gc, n1 et n2 valent soit 0 soit 1, p représente une position de liaison en 3 ou 6, r et s représentent des positions de liaison en 3 ou 4, à condition que lorsque r représente 3 alors s représente 4 et lorsque r représente 4 alors s représente 3 ;
h) récepteurs de mannose tels que définis dans la formule
Manαp[(Manαr)]ₙ₁Man (XVII)
dans laquelle n vaut indépendamment 0 ou 1, p et r représentent une position de liaison en 3 ou 6 entre les résidus Man, à condition que lorsque p représente 3 alors r représente 6, et lorsque p représente 6 alors r représente 3.

11. Composition selon la revendication 10, dans laquelle le récepteur d'agent pathogène du groupe a) est choisi dans le groupe des séquences oligosaccharidiques de récepteurs constitué de :
lactosylcéramide, lactosylcéramide comprenant des acides gras hydroxylés, lactosylcéramide avec le carbone 3 modifié d'un résidu de galactose et isoglobotriaocylcéramide.

12. Composition selon la revendication 10, dans laquelle le récepteur d'agent pathogène du groupe g) est choisi dans le groupe des séquences oligosaccharidiques de récepteurs constitué de :
oligosaccharides avec les structures Neu5Xα3Galβ3(Fucα4)GlcNAc, Neu5Xα3Galβ4(Fucα3)GlcNAc, Neu5Xα3Galβ4(Fucα3)Glc, Neu5Xα3Galβ3GlcNAc, Neu5Xα3Galβ4GlcNAc, Neu5Xα3Galβ4Glc, Neu5Xα6Galβ4GlcNAc ou Neu5Xα6Galβ4Glc.

13. Composition selon la revendication 1, dans laquelle au moins l'un desdits composés est une forme monovalente étant une glycosylamine ou un glycosylamide ou un méthylglycoside ou un glycoside y compris d'autres N-glycosides, C-glycosides ou S-glycosides.

14. Composition selon la revendication 1, dans laquelle au moins l'un desdits composés est lié à un support polyvalent.

15. Composition selon la revendication 14, dans laquelle ledit support polyvalent est un support glucidique ou un support particulaire ou un support glucidique soluble, ou un support particulaire ou un polysaccharide bactérien ou une partie de polysaccharide bactérien comprenant également la séquence oligosaccharidique du récepteur, ou une particule glucidique, une particule polymère synthétique ou une cellule, ou un conjugué glucidique antigénique ou immunostimulant.

16. Composition selon la revendication 1, comprenant en outre une ou plusieurs séquences oligosaccharidiques choisies dans le groupe de :
oligosaccharides comprenant les séquences Fuca2Gal, Fucα3GlcNAc, Fuca3Glc, NeuNAcα8NeuNAc, Fucα2Galβ3/4GlcNAc, Fucα2Galβ4Glc, Fucα2Galβ4(Fucα3)Glc, Galβ4(Fucα3)GlcNAc, Fucα2Galβ3/4(Fucα4/3)GlcNAc et des séquences oligosaccharidiques de gangliosides de la gangliosérie.

17. Utilisation d'une composition comprenant une quantité efficace sur le plan pharmaceutique ou thérapeutique ou prophylactique de la composition selon la revendication 1 pour la fabrication d'un médicament destiné à la prophylaxie ou au traitement d'une infection bactérienne.

18. Utilisation selon la revendication 17, où ladite infection bactérienne est une infection gastro-intestinale.

19. Utilisation selon la revendication 18, où ladite infection gastro-intestinale provoque une diarrhée ou la diarrhée du voyageur, les diarrhées des enfants, une diarrhée persistante, une diarrhée hydrique, une rectocolite hémorragique ou un syndrome urémique hémolytique.

20. Utilisation selon la revendication 18, où ladite infection est provoquée par EPEC (*Escherichia coli* entéropathogénique), ETEC (*Escherichia coli* entérotoxigénique), EHEC (*Escherichia coli* entéro-hémorragique), EIEC (*Escherichia coli* entéro-invasif) ou EAEC (*Escherichia coli* entéro-agrégant).

21. Utilisation selon la revendication 18, où ladite infection est provoquée par une espèce de *Vibrio* y compris *Vibrio cholerae,* une espèce de *Campylobacter* y compris *Campylobacter jejuni,* des parasites intestinaux eucaryotes y compris l'espèce *Entamobae, Salmonella* y compris *Salmonella typhimurium,* une espèce de *Shigella,* une espèce de *Aeromonas,* une espèce de *Helicobacter* zoonotique, une espèce de *Listeria* ou un rotavirus ou la cause de l'infection n'est pas diagnostiquée.

22. Utilisation selon la revendication 17, où ledit sujet est un patient humain ou un patient animal.

23. Procédé d'amélioration de la sécurité alimentaire comprenant une étape consistant à enrober un produit alimentaire avec une composition selon la revendication 1.

24. Composition nutritionnelle ou additif nutritionnel ou lait pour nourrissons comprenant une ou plusieurs fractions purifiées d'au moins deux composés tels que définis dans la revendication 1 pour la prophylaxie ou le traitement d'une infection gastro-intestinale.

25. Composition nutritionnelle ou additif nutritionnel ou lait pour nourrissons selon la revendication 24 comprenant en outre un micro-organisme probiotique ou une substance prébiotique.

26. Produit pour l'inhibition d'agents pathogènes, spécialement de *E. coli* provoquant une diarrhée, comprenant *ex vivo* une ou plusieurs fractions purifiées d'au moins deux composés tels que définis dans la revendication 1, où ledit produit est choisi dans le groupe constitué : d'un produit d'hygiène buccale, d'un produit d'enrobage alimentaire, d'un conservateur alimentaire, d'un produit topique, de lavage ou cosmétique.

27. Procédé d'analyse ou de diagnostic comprenant une étape consistant à mettre en contact un microbe pathogène ou probiotique potentiel avec au moins trois récepteurs d'agents pathogènes tels que définis dans la revendication 1.

28. Procédé d'analyse ou de diagnostic comprenant une étape consistant à mettre en contact un microbe pathogène ou probiotique potentiel avec un récepteur choisi dans le groupe constitué de : lacto-récepteurs, néolacto-récepteurs, fucosyl-récepteurs, récepteurs de mannose ou récepteurs d'acide sialique
pour l'analyse ou le diagnostic de la liaison d'un agent pathogène ou d'un probiotique,
où lesdits récepteurs
i) sont des récepteurs liés à des protéines et
ii) comprennent une séquence oligosaccharidique terminale non réductrice présente dans l'épithélium de l'intestin humain, de l'estomac humain ou du larynx humain.

29. Procédé de recherche ou de conception de substances oligosaccharidiques fixant les bactéries comprenant une étape consistant à modéliser les propriétés de liaison des récepteurs oligosaccharidiques tels que définis dans la revendication 1.

30. Substance inhibant *E. coli* diarrhéigénique selon la formule
[OS-(Y)ₚ - (S)_{q} - (z)ᵣ -]ₙPO (SP1)
dans laquelle PO représente une structure de support oligomère ou polymère, OS représente une séquence oligosaccharidique telle que définie dans la formule 1 ou II de la revendication 1, n représente un nombre entier ≥ 1 indiquant le nombre de groupes oligosaccharidiques liés de manière covalente au support PO, S représente un groupe espaceur, p, q et r valent chacun 0 ou 1, moyennant quoi l'un de p et r est différent de 0, y et z représentent des groupes de liaison, au moins l'un de y et z représentant un résidu d'O-hydroxylamine -O-NH- ou -O-N=, avec l'atome d'azote étant lié à la structure OS et/ou PO, respectivement, et l'autre de y et z, s'il est présent, représente un groupe de ligature chimio-sélective.
